# EUROPEAN PATENT SPECIFICATION

(11) **EP 2 411 513 B1**
(45) Date of publication and mention of the grant of the patent: **09.11.2016**
(21) Application number: 10713874.5
(22) Date of filing: 23.03.2010
(51) Int. Cl.: C12N 9/88

(54) **MUTANT CyaA POLYPEPTIDES AND POLYPEPTIDE DERIVATIVES SUITABLE FOR THE DELIVERY OF IMMUNOGENIC MOLECULES INTO A CELL**
MUTIERTE CYAA-POLYPEPTIDE UND -POLYPEPTIDDERIVATE MIT EIGNUNG ZUR ZUFÜHRUNG IMMUNOGENER MOLEKÜLE IN EINE ZELLE
POLYPEPTIDES CYAA MUTANTS ET DÉRIVÉS DE POLYPEPTIDES APPROPRIÉS POUR DISTRIBUER DES MOLÉCULES IMMUNOGÈNES DANS UNE CELLULE

(30) Priority: 23.03.2009 EP 09155929; 23.03.2009 US 409324
(43) Date of publication of application: 01.02.2012
(73) Proprietor: Institut Pasteur, 75724 Paris Cédex 15 (FR); Institute of Physiology of the ASCR, v.v.i., Prague 4 - Krc (CZ); Institute of Microbiology of the ASCR, v.v.i., Prague 4 - Krc (CZ)
(72) Inventor: SEBO, Peter, 142 00 Prague 4 (CZ); OSICKOVA, Adriana, 142 00 Prague 4 (CZ); MASIN, Jiri, 25082 Uvaly (CZ); FAYOLLE, Catherine, F-91360 Epinay sur Orge (FR); KRUSEK, Jan, 182 20 Prague 8 (CZ); BASLER, Marek, 143 00 Prague 4 (CZ); LECLERC, Claude, F-75015 Paris (FR); OSICKA, Radim, 142 00 Prague 4 (CZ)
(74) Representative: Desaix, Anne
(86) International application number: PCT/EP2010/053795
(87) International publication number: WO 2010/136231

(56) References cited:
- WO-A-2005/035557
- DATABASE EMBL 21 July 2008 (2008-07-21), "bifunctional hemolysin-adenylate cyclase precursor [Bordetella parapertussis 12822]" XP002525392 retrieved from NCBI Database accession no. NP_882677
- DATABASE EMBL 18 April 2005 (2005-04-18), "cyaA [Bordetella pertussis]" XP002525393 retrieved from NCBI Database accession no. CAA32411
- DATABASE EMBL 29 July 2008 (2008-07-29), "bifunctional hemolysin-adenylate cyclase precursor [Bordetella bronchiseptica RB50]" XP002525394 retrieved from NCBI Database accession no. CAE30822
- DATABASE EMBL 4 November 2005 (2005-11-04), "adenylate cyclase toxin [Bordetella hinzii]" XP002525395 retrieved from NCBI Database accession no. AAZ57194
- BASLER MAREK ET AL: "Segments crucial for membrane translocation and pore-forming activity of Bordetella adenylate cyclase toxin" JOURNAL OF BIOLOGICAL CHEMISTRY, vol. 282, no. 17, April 2007 (2007-04), pages 12419-12429, XP002525387 ISSN: 0021-9258
- BASLER MAREK ET AL: "Pore-forming and enzymatic activities of Bordetella pertussis adenylate cyclase toxin synergize in promoting lysis of monocytes" INFECTION AND IMMUNITY, vol. 74, no. 4, April 2006 (2006-04), pages 2207-2214, XP002525388 ISSN: 0019-9567
- BASAR TUMAY ET AL: "The conserved lysine 860 in the additional fatty-acylation site of Bordetella pertussis adenylate cyclase is crucial for toxin function independently of its acylation status" JOURNAL OF BIOLOGICAL CHEMISTRY, vol. 274, no. 16, 16 April 1999 (1999-04-16), pages 10777-10783, XP002525389 ISSN: 0021-9258
- FISER RADOVAN ET AL: "Third activity of Bordetella adenylate cyclase (AC) toxin-hemolysin - Membrane translocation of AC domain polypeptide promotes calcium influx into CD11b(+) monocytes independently of the catalytic and hemolytic activities" JOURNAL OF BIOLOGICAL CHEMISTRY, vol. 282, no. 5, February 2007 (2007-02), pages 2808-2820, XP002525390 ISSN: 0021-9258
- MASIN JIRI ET AL: "Acylation of lysine 860 allows tight binding and cytotoxicity of Bordetella adenylate cyclase on CD11b-expressing cells" BIOCHEMISTRY, vol. 44, no. 38, September 2005 (2005-09), pages 12759-12766, XP002525391 ISSN: 0006-2960
- GUERMONPREZ P ET AL: "BORDETELLA PERTUSSIS ADENYLATE CYCLASE TOXIN: A VEHICLE TO DELIVER CD8-POSITIVE T-CELL EPITOPES INTO ANTIGEN-PRESENTING CELLS" METHODS IN ENZYMOLOGY, ACADEMIC PRESS INC, SAN DIEGO, CA, US, vol. 326, 1 January 2000 (2000-01-01), pages 527-542, XP009002613 ISSN: 0076-6879

### SEQUENCE LISTING

<110> INSTITUT PASTEUR
<120> Mutant CyaA polypeptides and polypeptide derivatives suitable for the delivery of immunogenic molecules into a cell
<130> B08142B
<150> EP 09155929.4
   <151> 2009-03-23
<150> US 12/409324
   <151> 2009-03-23
<160> 9
<170> PatentIn version 3.3
<210> 1
   <211> 1706
   <212> PRT
   <213> Bordetella pertussis
<400> 1
   Met Gln Gln Ser His Gln Ala Gly Tyr Ala Asn Ala Ala Asp Arg Glu
   1               5                   10                  15
   Ser Gly Ile Pro Ala Ala Val Leu Asp Gly Ile Lys Ala Val Ala Lys
               20                  25                  30
   Glu Lys Asn Ala Thr Leu Met Phe Arg Leu Val Asn Pro His Ser Thr
           35                  40                  45
   Ser Leu Ile Ala Glu Gly Val Ala Thr Lys Gly Leu Gly Val His Ala
       50                  55                  60
   Lys Ser Ser Asp Trp Gly Leu Gln Ala Gly Tyr Ile Pro Val Asn Pro
   65                  70                  75                  80
   Asn Leu Ser Lys Leu Phe Gly Arg Ala Pro Glu Val Ile Ala Arg Ala
                   85                  90                  95
   Asp Asn Asp Val Asn Ser Ser Leu Ala His Gly His Thr Ala Val Asp
               100                 105                 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
                   165                 170                 175
   Ile Gly Asn Ala Ala Gly Ile Pro Leu Thr Ala Asp Ile Asp Met Phe
               180                 185                 190
   Ala Ile Met Pro His Leu Ser Asn Phe Arg Asp Ser Ala Arg Ser Ser
           195                 200                 205
   Val Thr Ser Gly Asp Ser Val Thr Asp Tyr Leu Ala Arg Thr Arg Arg
       210                 215                 220
   Ala Ala Ser Glu Ala Thr Gly Gly Leu Asp Arg Glu Arg Ile Asp Leu
   225                 230                 235                 240
   Leu Trp Lys Ile Ala Arg Ala Gly Ala Arg Ser Ala Val Gly Thr Glu
                   245                 250                 255
   Ala Arg Arg Gln Phe Arg Tyr Asp Gly Asp Met Asn Ile Gly Val Ile
               260                 265                 270
   Thr Asp Phe Glu Leu Glu Val Arg Asn Ala Leu Asn Arg Arg Ala His
           275                 280                 285
   Ala Val Gly Ala Gln Asp Val Val Gln His Gly Thr Glu Gln Asn Asn
       290                 295                 300
   Pro Phe Pro Glu Ala Asp Glu Lys Ile Phe Val Val Ser Ala Thr Gly
   305                 310                 315                 320
   Glu Ser Gln Met Leu Thr Arg Gly Gln Leu Lys Glu Tyr Ile Gly Gln
                   325                 330                 335
   Gln Arg Gly Glu Gly Tyr Val Phe Tyr Glu Asn Arg Ala Tyr Gly Val
               340                 345                 350
   Ala Gly Lys Ser Leu Phe Asp Asp Gly Leu Gly Ala Ala Pro Gly Val
           355                 360                 365
   Pro Ser Gly Arg Ser Lys Phe Ser Pro Asp Val Leu Glu Thr Val Pro
       370                 375                 380
   Ala Ser Pro Gly Leu Arg Arg Pro Ser Leu Gly Ala Val Glu Arg Gln
   385                 390                 395                 400
   Asp Ser Gly Tyr Asp Ser Leu Asp Gly Val Gly Ser Arg Ser Phe Ser
                   405                 410                 415
   Leu Gly Glu Val Ser Asp Met Ala Ala Val Glu Ala Ala Glu Leu Glu
               420                 425                 430
   Met Thr Arg Gln Val Leu His Ala Gly Ala Arg Gln Asp Asp Ala Glu
           435                 440                 445
   Pro Gly Val Ser Gly Ala Ser Ala His Trp Gly Gln Arg Ala Leu Gln
       450                 455                 460
   Gly Ala Gln Ala Val Ala Ala Ala Gln Arg Leu Val His Ala Ile Ala
   465                 470                 475                 480
   Leu Met Thr Gln Phe Gly Arg Ala Gly Ser Thr Asn Thr Pro Gln Glu
                   485                 490                 495
   Ala Ala Ser Leu Ser Ala Ala Val Phe Gly Leu Gly Glu Ala Ser Ser
               500                 505                 510
   Ala Val Ala Glu Thr Val Ser Gly Phe Phe Arg Gly Ser Ser Arg Trp
           515                 520                 525
   Ala Gly Gly Phe Gly Val Ala Gly Gly Ala Met Ala Leu Gly Gly Gly
       530                 535                 540
   Ile Ala Ala Ala Val Gly Ala Gly Met Ser Leu Thr Asp Asp Ala Pro
   545                 550                 555                 560
   Ala Gly Gln Lys Ala Ala Ala Gly Ala Glu Ile Ala Leu Gln Leu Thr
                   565                 570                 575
   Gly Gly Thr Val Glu Leu Ala Ser Ser Ile Ala Leu Ala Leu Ala Ala
               580                 585                 590
   Ala Arg Gly Val Thr Ser Gly Leu Gln Val Ala Gly Ala Ser Ala Gly
           595                 600                 605
   Ala Ala Ala Gly Ala Leu Ala Ala Ala Leu Ser Pro Met Glu Ile Tyr
       610                 615                 620
   Gly Leu Val Gln Gln Ser His Tyr Ala Asp Gln Leu Asp Lys Leu Ala
   625                 630                 635                 640
   Gln Glu Ser Ser Ala Tyr Gly Tyr Glu Gly Asp Ala Leu Leu Ala Gln
                   645                 650                 655
   Leu Tyr Arg Asp Lys Thr Ala Ala Glu Gly Ala Val Ala Gly Val Ser
               660                 665                 670
   Ala Val Leu Ser Thr Val Gly Ala Ala Val Ser Ile Ala Ala Ala Ala
           675                 680                 685
   Ser Val Val Gly Ala Pro Val Ala Val Val Thr Ser Leu Leu Thr Gly
       690                 695                 700
   Ala Leu Asn Gly Ile Leu Arg Gly Val Gln Gln Pro Ile Ile Glu Lys
   705                 710                 715                 720
   Leu Ala Asn Asp Tyr Ala Arg Lys Ile Asp Glu Leu Gly Gly Pro Gln
                   725                 730                 735
   Ala Tyr Phe Glu Lys Asn Leu Gln Ala Arg His Glu Gln Leu Ala Asn
               740                 745                 750
   Ser Asp Gly Leu Arg Lys Met Leu Ala Asp Leu Gln Ala Gly Trp Asn
           755                 760                 765
   Ala Ser Ser Val Ile Gly Val Gln Thr Thr Glu Ile Ser Lys Ser Ala
       770                 775                 780
   Leu Glu Leu Ala Ala Ile Thr Gly Asn Ala Asp Asn Leu Lys Ser Val
   785                 790                 795                 800
   Asp Val Phe Val Asp Arg Phe Val Gln Gly Glu Arg Val Ala Gly Gln
                   805                 810                 815
   Pro Val Val Leu Asp Val Ala Ala Gly Gly Ile Asp Ile Ala Ser Arg
               820                 825                 830
   Lys Gly Glu Arg Pro Ala Leu Thr Phe Ile Thr Pro Leu Ala Ala Pro
           835                 840                 845
   Gly Glu Glu Gln Arg Arg Arg Thr Lys Thr Gly Lys Ser Glu Phe Thr
       850                 855                 860
   Thr Phe Val Glu Ile Val Gly Lys Gln Asp Arg Trp Arg Ile Arg Asp
   865                 870                 875                 880
   Gly Ala Ala Asp Thr Thr Ile Asp Leu Ala Lys Val Val Ser Gln Leu
                   885                 890                 895
   Val Asp Ala Asn Gly Val Leu Lys His Ser Ile Lys Leu Asp Val Ile
               900                 905                 910
   Gly Gly Asp Gly Asp Asp Val Val Leu Ala Asn Ala Ser Arg Ile His
           915                 920                 925
   Tyr Asp Gly Gly Ala Gly Thr Asn Thr Val Ser Tyr Ala Ala Leu Gly
       930                 935                 940
   Arg Gln Asp Ser Ile Thr Val Ser Ala Asp Gly Glu Arg Phe Asn Val
   945                 950                 955                 960
   Arg Lys Gln Leu Asn Asn Ala Asn Val Tyr Arg Glu Gly Val Ala Thr
                   965                 970                 975
   Gln Thr Thr Ala Tyr Gly Lys Arg Thr Glu Asn Val Gln Tyr Arg His
               980                 985                 990
   Val Glu Leu Ala Arg Val Gly Gln  Val Val Glu Val Asp  Thr Leu Glu
           995                 1000                1005
   His Val  Gln His Ile Ile Gly  Gly Ala Gly Asn Asp  Ser Ile Thr
       1010                 1015                 1020
   Gly Asn  Ala His Asp Asn Phe  Leu Ala Gly Gly Ser  Gly Asp Asp
       1025                 1030                 1035
   Arg Leu  Asp Gly Gly Ala Gly  Asn Asp Thr Leu Val  Gly Gly Glu
       1040                 1045                 1050
   Gly Gln  Asn Thr Val Ile Gly  Gly Ala Gly Asp Asp  Val Phe Leu
       1055                 1060                 1065
   Gln Asp  Leu Gly Val Trp Ser  Asn Gln Leu Asp Gly  Gly Ala Gly
       1070                 1075                 1080
   Val Asp  Thr Val Lys Tyr Asn  Val His Gln Pro Ser  Glu Glu Arg
       1085                 1090                 1095
   Leu Glu  Arg Met Gly Asp Thr  Gly Ile His Ala Asp  Leu Gln Lys
       1100                 1105                 1110
   Gly Thr  Val Glu Lys Trp Pro  Ala Leu Asn Leu Phe  Ser Val Asp
       1115                 1120                 1125
   His Val  Lys Asn Ile Glu Asn  Leu His Gly Ser Arg  Leu Asn Asp
       1130                 1135                 1140
   Arg Ile  Ala Gly Asp Asp Gln  Asp Asn Glu Leu Trp  Gly His Asp
   1145 1150 1155
   Gly Asn Asp Thr Ile Arg Gly Arg Gly Gly Asp Asp Ile Leu Arg
   1160 1165 1170
   Gly Gly Leu Gly Leu Asp Thr Leu Tyr Gly Glu Asp Gly Asn Asp
   1175 1180 1185
   Ile Phe Leu Gln Asp Asp Glu Thr Val Ser Asp Asp Ile Asp Gly
   1190 1195 1200
   Gly Ala Gly Leu Asp Thr Val Asp Tyr Ser Ala Met Ile His Pro
   1205 1210 1215
   Gly Arg Ile Val Ala Pro His Glu Tyr Gly Phe Gly Ile Glu Ala
   1220 1225 1230
   Asp Leu Ser Arg Glu Trp Val Arg Lys Ala Ser Ala Leu Gly Val
   1235 1240 1245
   Asp Tyr Tyr Asp Asn Val Arg Asn Val Glu Asn Val Ile Gly Thr
   1250 1255 1260
   Ser Met Lys Asp Val Leu Ile Gly Asp Ala Gln Ala Asn Thr Leu
   1265 1270 1275
   Met Gly Gln Gly Gly Asp Asp Thr Val Arg Gly Gly Asp Gly Asp
   1280 1285 1290
   Asp Leu Leu Phe Gly Gly Asp Gly Asn Asp Met Leu Tyr Gly Asp
   1295 1300 1305
   Ala Gly Asn Asp Thr Leu Tyr Gly Gly Leu Gly Asp Asp Thr Leu
   1310 1315 1320
   Glu Gly Gly Ala Gly Asn Asp Trp Phe Gly Gln Thr Gln Ala Arg
   1325 1330 1335
   Glu His Asp Val Leu Arg Gly Gly Asp Gly Val Asp Thr Val Asp
   1340 1345 1350
   Tyr Ser Gln Thr Gly Ala His Ala Gly Ile Ala Ala Gly Arg Ile
   1355 1360 1365
   Gly Leu Gly Ile Leu Ala Asp Leu Gly Ala Gly Arg Val Asp Lys
   1370 1375 1380
   Leu Gly Glu Ala Gly Ser Ser Ala Tyr Asp Thr Val Ser Gly Ile
   1385 1390 1395
   Glu Asn Val Val Gly Thr Glu Leu Ala Asp Arg Ile Thr Gly Asp
   1400 1405 1410
   Ala Gln Ala Asn Val Leu Arg Gly Ala Gly Gly Ala Asp Val Leu
   1415 1420 1425
   Ala Gly Gly Glu Gly Asp Asp Val Leu Leu Gly Gly Asp Gly Asp
   1430 1435 1440
   Asp Gln Leu Ser Gly Asp Ala Gly Arg Asp Arg Leu Tyr Gly Glu
   1445 1450 1455
   Ala Gly Asp Asp Trp Phe Phe Gln Asp Ala Ala Asn Ala Gly Asn
   1460 1465 1470
   Leu Leu Asp Gly Gly Asp Gly Arg Asp Thr Val Asp Phe Ser Gly
   1475 1480 1485
   Pro Gly Arg Gly Leu Asp Ala Gly Ala Lys Gly Val Phe Leu Ser
   1490 1495 1500
   Leu Gly Lys Gly Phe Ala Ser Leu Met Asp Glu Pro Glu Thr Ser
   1505 1510 1515
   Asn Val Leu Arg Asn Ile Glu Asn Ala Val Gly Ser Ala Arg Asp
   1520 1525 1530
   Asp Val Leu Ile Gly Asp Ala Gly Ala Asn Val Leu Asn Gly Leu
   1535 1540 1545
   Ala Gly Asn Asp Val Leu Ser Gly Gly Ala Gly Asp Asp Val Leu
   1550 1555 1560
   Leu Gly Asp Glu Gly Ser Asp Leu Leu Ser Gly Asp Ala Gly Asn
   1565 1570 1575
   Asp Asp Leu Phe Gly Gly Gln Gly Asp Asp Thr Tyr Leu Phe Gly
   1580 1585 1590
   Val Gly Tyr Gly His Asp Thr Ile Tyr Glu Ser Gly Gly Gly His
   1595 1600 1605
   Asp Thr Ile Arg Ile Asn Ala Gly Ala Asp Gln Leu Trp Phe Ala
   1610 1615 1620
   Arg Gln Gly Asn Asp Leu Glu Ile Arg Ile Leu Gly Thr Asp Asp
   1625 1630 1635
   Ala Leu Thr Val His Asp Trp Tyr Arg Asp Ala Asp His Arg Val
   1640 1645 1650
   Glu Ile Ile His Ala Ala Asn Gln Ala Val Asp Gln Ala Gly Ile
   1655 1660 1665
   Glu Lys Leu Val Glu Ala Met Ala Gln Tyr Pro Asp Pro Gly Ala
   1670 1675 1680
   Ala Ala Ala Ala Pro Pro Ala Ala Arg Val Pro Asp Thr Leu Met
   1685 1690 1695
   Gln Ser Leu Ala Val Asn Trp Arg
   1700 1705
<210> 2
   <211> 1706
   <212> PRT
   <213> Artificial
<220>
   <223> CyaA/E570Q+K860R
<400> 2
   Met Gln Gln Ser His Gln Ala Gly Tyr Ala Asn Ala Ala Asp Arg Glu
   1 5 10 15
   Ser Gly Ile Pro Ala Ala Val Leu Asp Gly Ile Lys Ala Val Ala Lys
   20 25 30
   Glu Lys Asn Ala Thr Leu Met Phe Arg Leu Val Asn Pro His Ser Thr
   35 40 45
   Ser Leu Ile Ala Glu Gly Val Ala Thr Lys Gly Leu Gly Val His Ala
   50 55 60
   Lys Ser Ser Asp Trp Gly Leu Gln Ala Gly Tyr Ile Pro Val Asn Pro
   65 70 75 80
   Asn Leu Ser Lys Leu Phe Gly Arg Ala Pro Glu Val Ile Ala Arg Ala
   85 90 95
   Asp Asn Asp Val Asn Ser Ser Leu Ala His Gly His Thr Ala Val Asp
   100 105 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
                   165                 170                 175
   Ile Gly Asn Ala Ala Gly Ile Pro Leu Thr Ala Asp Ile Asp Met Phe
               180                 185                 190
   Ala Ile Met Pro His Leu Ser Asn Phe Arg Asp Ser Ala Arg Ser Ser
           195                 200                 205
   Val Thr Ser Gly Asp Ser Val Thr Asp Tyr Leu Ala Arg Thr Arg Arg
       210                 215                 220
   Ala Ala Ser Glu Ala Thr Gly Gly Leu Asp Arg Glu Arg Ile Asp Leu
   225                 230                 235                 240
   Leu Trp Lys Ile Ala Arg Ala Gly Ala Arg Ser Ala Val Gly Thr Glu
                   245                 250                 255
   Ala Arg Arg Gln Phe Arg Tyr Asp Gly Asp Met Asn Ile Gly Val Ile
               260                 265                 270
   Thr Asp Phe Glu Leu Glu Val Arg Asn Ala Leu Asn Arg Arg Ala His
           275                 280                 285
   Ala Val Gly Ala Gln Asp Val Val Gln His Gly Thr Glu Gln Asn Asn
       290                 295                 300
   Pro Phe Pro Glu Ala Asp Glu Lys Ile Phe Val Val Ser Ala Thr Gly
   305                 310                 315                 320
   Glu Ser Gln Met Leu Thr Arg Gly Gln Leu Lys Glu Tyr Ile Gly Gln
                   325                 330                 335
   Gln Arg Gly Glu Gly Tyr Val Phe Tyr Glu Asn Arg Ala Tyr Gly Val
               340                 345                 350
   Ala Gly Lys Ser Leu Phe Asp Asp Gly Leu Gly Ala Ala Pro Gly Val
           355                 360                 365
   Pro Ser Gly Arg Ser Lys Phe Ser Pro Asp Val Leu Glu Thr Val Pro
       370                 375                 380
   Ala Ser Pro Gly Leu Arg Arg Pro Ser Leu Gly Ala Val Glu Arg Gln
   385                 390                 395                 400
   Asp Ser Gly Tyr Asp Ser Leu Asp Gly Val Gly Ser Arg Ser Phe Ser
                   405                 410                 415
   Leu Gly Glu Val Ser Asp Met Ala Ala Val Glu Ala Ala Glu Leu Glu
               420                 425                 430
   Met Thr Arg Gln Val Leu His Ala Gly Ala Arg Gln Asp Asp Ala Glu
           435                 440                 445
   Pro Gly Val Ser Gly Ala Ser Ala His Trp Gly Gln Arg Ala Leu Gln
       450                 455                 460
   Gly Ala Gln Ala Val Ala Ala Ala Gln Arg Leu Val His Ala Ile Ala
   465                 470                 475                 480
   Leu Met Thr Gln Phe Gly Arg Ala Gly Ser Thr Asn Thr Pro Gln Glu
                   485                 490                 495
   Ala Ala Ser Leu Ser Ala Ala Val Phe Gly Leu Gly Glu Ala Ser Ser
               500                 505                 510
   Ala Val Ala Glu Thr Val Ser Gly Phe Phe Arg Gly Ser Ser Arg Trp
           515                 520                 525
   Ala Gly Gly Phe Gly Val Ala Gly Gly Ala Met Ala Leu Gly Gly Gly
       530                 535                 540
   Ile Ala Ala Ala Val Gly Ala Gly Met Ser Leu Thr Asp Asp Ala Pro
   545                 550                 555                 560
   Ala Gly Gln Lys Ala Ala Ala Gly Ala Gln Ile Ala Leu Gln Leu Thr
                   565                 570                 575
   Gly Gly Thr Val Glu Leu Ala Ser Ser Ile Ala Leu Ala Leu Ala Ala
               580                 585                 590
   Ala Arg Gly Val Thr Ser Gly Leu Gln Val Ala Gly Ala Ser Ala Gly
           595                 600                 605
   Ala Ala Ala Gly Ala Leu Ala Ala Ala Leu Ser Pro Met Glu Ile Tyr
       610                 615                 620
   Gly Leu Val Gln Gln Ser His Tyr Ala Asp Gln Leu Asp Lys Leu Ala
   625 630 635 640
   Gln Glu Ser Ser Ala Tyr Gly Tyr Glu Gly Asp Ala Leu Leu Ala Gln
   645 650 655
   Leu Tyr Arg Asp Lys Thr Ala Ala Glu Gly Ala Val Ala Gly Val Ser
   660 665 670
   Ala Val Leu Ser Thr Val Gly Ala Ala Val Ser Ile Ala Ala Ala Ala
   675 680 685
   Ser Val Val Gly Ala Pro Val Ala Val Val Thr Ser Leu Leu Thr Gly
   690 695 700
   Ala Leu Asn Gly Ile Leu Arg Gly Val Gln Gln Pro Ile Ile Glu Lys
   705 710 715 720
   Leu Ala Asn Asp Tyr Ala Arg Lys Ile Asp Glu Leu Gly Gly Pro Gln
   725 730 735
   Ala Tyr Phe Glu Lys Asn Leu Gln Ala Arg His Glu Gln Leu Ala Asn
   740 745 750
   Ser Asp Gly Leu Arg Lys Met Leu Ala Asp Leu Gln Ala Gly Trp Asn
   755 760 765
   Ala Ser Ser Val Ile Gly Val Gln Thr Thr Glu Ile Ser Lys Ser Ala
   770 775 780
   Leu Glu Leu Ala Ala Ile Thr Gly Asn Ala Asp Asn Leu Lys Ser Val
   785 790 795 800
   Asp Val Phe Val Asp Arg Phe Val Gln Gly Glu Arg Val Ala Gly Gln
   805 810 815
   Pro Val Val Leu Asp Val Ala Ala Gly Gly Ile Asp Ile Ala Ser Arg
   820 825 830
   Lys Gly Glu Arg Pro Ala Leu Thr Phe Ile Thr Pro Leu Ala Ala Pro
   835 840 845
   Gly Glu Glu Gln Arg Arg Arg Thr Lys Thr Gly Arg Ser Glu Phe Thr
   850 855 860
   Thr Phe Val Glu Ile Val Gly Lys Gln Asp Arg Trp Arg Ile Arg Asp
   865 870 875 880
   Gly Ala Ala Asp Thr Thr Ile Asp Leu Ala Lys Val Val Ser Gln Leu
   885 890 895
   Val Asp Ala Asn Gly Val Leu Lys His Ser Ile Lys Leu Asp Val Ile
   900 905 910
   Gly Gly Asp Gly Asp Asp Val Val Leu Ala Asn Ala Ser Arg Ile His
   915 920 925
   Tyr Asp Gly Gly Ala Gly Thr Asn Thr Val Ser Tyr Ala Ala Leu Gly
   930 935 940
   Arg Gln Asp Ser Ile Thr Val Ser Ala Asp Gly Glu Arg Phe Asn Val
   945 950 955 960
   Arg Lys Gln Leu Asn Asn Ala Asn Val Tyr Arg Glu Gly Val Ala Thr
   965 970 975
   Gln Thr Thr Ala Tyr Gly Lys Arg Thr Glu Asn Val Gln Tyr Arg His
   980 985 990
   Val Glu Leu Ala Arg Val Gly Gln Val Val Glu Val Asp Thr Leu Glu
   995 1000 1005
   His Val Gln His Ile Ile Gly Gly Ala Gly Asn Asp Ser Ile Thr
   1010 1015 1020
   Gly Asn Ala His Asp Asn Phe Leu Ala Gly Gly Ser Gly Asp Asp
   1025 1030 1035
   Arg Leu Asp Gly Gly Ala Gly Asn Asp Thr Leu Val Gly Gly Glu
   1040 1045 1050
   Gly Gln Asn Thr Val Ile Gly Gly Ala Gly Asp Asp Val Phe Leu
   1055 1060 1065
   Gln Asp Leu Gly Val Trp Ser Asn Gln Leu Asp Gly Gly Ala Gly
   1070 1075 1080
   Val Asp Thr Val Lys Tyr Asn Val His Gln Pro Ser Glu Glu Arg
   1085 1090 1095
   Leu Glu Arg Met Gly Asp Thr Gly Ile His Ala Asp Leu Gln Lys
   1100 1105 1110
   Gly Thr Val Glu Lys Trp Pro Ala Leu Asn Leu Phe Ser Val Asp
   1115 1120 1125
   His Val Lys Asn Ile Glu Asn Leu His Gly Ser Arg Leu Asn Asp
   1130 1135 1140
   Arg Ile Ala Gly Asp Asp Gln Asp Asn Glu Leu Trp Gly His Asp
   1145 1150 1155
   Gly Asn Asp Thr Ile Arg Gly Arg Gly Gly Asp Asp Ile Leu Arg
   1160 1165 1170
   Gly Gly Leu Gly Leu Asp Thr Leu Tyr Gly Glu Asp Gly Asn Asp
   1175 1180 1185
   Ile Phe Leu Gln Asp Asp Glu Thr Val Ser Asp Asp Ile Asp Gly
   1190 1195 1200
   Gly Ala Gly Leu Asp Thr Val Asp Tyr Ser Ala Met Ile His Pro
   1205 1210 1215
   Gly Arg Ile Val Ala Pro His Glu Tyr Gly Phe Gly Ile Glu Ala
   1220 1225 1230
   Asp Leu Ser Arg Glu Trp Val Arg Lys Ala Ser Ala Leu Gly Val
   1235 1240 1245
   Asp Tyr Tyr Asp Asn Val Arg Asn Val Glu Asn Val Ile Gly Thr
   1250 1255 1260
   Ser Met Lys Asp Val Leu Ile Gly Asp Ala Gln Ala Asn Thr Leu
   1265 1270 1275
   Met Gly Gln Gly Gly Asp Asp Thr Val Arg Gly Gly Asp Gly Asp
   1280 1285 1290
   Asp Leu Leu Phe Gly Gly Asp Gly Asn Asp Met Leu Tyr Gly Asp
   1295 1300 1305
   Ala Gly Asn Asp Thr Leu Tyr Gly Gly Leu Gly Asp Asp Thr Leu
   1310 1315 1320
   Glu Gly Gly Ala Gly Asn Asp Trp Phe Gly Gln Thr Gln Ala Arg
   1325 1330 1335
   Glu His Asp Val Leu Arg Gly Gly Asp Gly Val Asp Thr Val Asp
   1340 1345 1350
   Tyr Ser  Gln Thr Gly Ala His  Ala Gly Ile Ala Ala  Gly Arg Ile
       1355                 1360                 1365
   Gly Leu  Gly Ile Leu Ala Asp  Leu Gly Ala Gly Arg  Val Asp Lys
       1370                 1375                 1380
   Leu Gly  Glu Ala Gly Ser Ser  Ala Tyr Asp Thr Val  Ser Gly Ile
       1385                 1390                 1395
   Glu Asn  Val Val Gly Thr Glu  Leu Ala Asp Arg Ile  Thr Gly Asp
       1400                 1405                 1410
   Ala Gln  Ala Asn Val Leu Arg  Gly Ala Gly Gly Ala  Asp Val Leu
       1415                 1420                 1425
   Ala Gly  Gly Glu Gly Asp Asp  Val Leu Leu Gly Gly  Asp Gly Asp
       1430                 1435                 1440
   Asp Gln  Leu Ser Gly Asp Ala  Gly Arg Asp Arg Leu  Tyr Gly Glu
       1445                 1450                 1455
   Ala Gly  Asp Asp Trp Phe Phe  Gln Asp Ala Ala Asn  Ala Gly Asn
       1460                 1465                 1470
   Leu Leu  Asp Gly Gly Asp Gly  Arg Asp Thr Val Asp  Phe Ser Gly
       1475                 1480                 1485
   Pro Gly  Arg Gly Leu Asp Ala  Gly Ala Lys Gly Val  Phe Leu Ser
       1490                 1495                 1500
   Leu Gly  Lys Gly Phe Ala Ser  Leu Met Asp Glu Pro  Glu Thr Ser
       1505                 1510                 1515
   Asn Val  Leu Arg Asn Ile Glu  Asn Ala Val Gly Ser  Ala Arg Asp
       1520                 1525                 1530
   Asp Val  Leu Ile Gly Asp Ala  Gly Ala Asn Val Leu  Asn Gly Leu
       1535                 1540                 1545
   Ala Gly  Asn Asp Val Leu Ser  Gly Gly Ala Gly Asp  Asp Val Leu
       1550                 1555                 1560
   Leu Gly  Asp Glu Gly Ser Asp  Leu Leu Ser Gly Asp  Ala Gly Asn
       1565                 1570                 1575
   Asp Asp  Leu Phe Gly Gly Gln  Gly Asp Asp Thr Tyr  Leu Phe Gly
       1580                 1585                 1590
   Val Gly Tyr Gly His Asp Thr Ile Tyr Glu Ser Gly Gly Gly His
   1595 1600 1605
   Asp Thr Ile Arg Ile Asn Ala Gly Ala Asp Gln Leu Trp Phe Ala
   1610 1615 1620
   Arg Gln Gly Asn Asp Leu Glu Ile Arg Ile Leu Gly Thr Asp Asp
   1625 1630 1635
   Ala Leu Thr Val His Asp Trp Tyr Arg Asp Ala Asp His Arg Val
   1640 1645 1650
   Glu Ile Ile His Ala Ala Asn Gln Ala Val Asp Gln Ala Gly Ile
   1655 1660 1665
   Glu Lys Leu Val Glu Ala Met Ala Gln Tyr Pro Asp Pro Gly Ala
   1670 1675 1680
   Ala Ala Ala Ala Pro Pro Ala Ala Arg Val Pro Asp Thr Leu Met
   1685 1690 1695
   Gln Ser Leu Ala Val Asn Trp Arg
   1700 1705
<210> 3
   <211> 1708
   <212> PRT
   <213> Artificial
<220>
   <223> CyaA/E570Q+K860R/AC-
<400> 3
   Met Gln Gln Ser His Gln Ala Gly Tyr Ala Asn Ala Ala Asp Arg Glu
   1 5 10 15
   Ser Gly Ile Pro Ala Ala Val Leu Asp Gly Ile Lys Ala Val Ala Lys
   20 25 30
   Glu Lys Asn Ala Thr Leu Met Phe Arg Leu Val Asn Pro His Ser Thr
   35 40 45
   Ser Leu Ile Ala Glu Gly Val Ala Thr Lys Gly Leu Gly Val His Ala
   50 55 60
   Lys Ser Ser Asp Trp Gly Leu Gln Ala Gly Tyr Ile Pro Val Asn Pro
   65 70 75 80
   Asn Leu Ser Lys Leu Phe Gly Arg Ala Pro Glu Val Ile Ala Arg Ala
                   85                  90                  95
   Asp Asn Asp Val Asn Ser Ser Leu Ala His Gly His Thr Ala Val Asp
               100                 105                 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
                   165                 170                 175
   Ile Gly Asn Ala Ala Gly Ile Pro Leu Thr Ala Asp Gly Ser Ile Asp
               180                 185                 190
   Met Phe Ala Ile Met Pro His Leu Ser Asn Phe Arg Asp Ser Ala Arg
           195                 200                 205
   Ser Ser Val Thr Ser Gly Asp Ser Val Thr Asp Tyr Leu Ala Arg Thr
       210                 215                 220
   Arg Arg Ala Ala Ser Glu Ala Thr Gly Gly Leu Asp Arg Glu Arg Ile
   225                 230                 235                 240
   Asp Leu Leu Trp Lys Ile Ala Arg Ala Gly Ala Arg Ser Ala Val Gly
                   245                 250                 255
   Thr Glu Ala Arg Arg Gln Phe Arg Tyr Asp Gly Asp Met Asn Ile Gly
               260                 265                 270
   Val Ile Thr Asp Phe Glu Leu Glu Val Arg Asn Ala Leu Asn Arg Arg
           275                 280                 285
   Ala His Ala Val Gly Ala Gln Asp Val Val Gln His Gly Thr Glu Gln
       290                 295                 300
   Asn Asn Pro Phe Pro Glu Ala Asp Glu Lys Ile Phe Val Val Ser Ala
   305                 310                 315                 320
   Thr Gly Glu Ser Gln Met Leu Thr Arg Gly Gln Leu Lys Glu Tyr Ile
                   325                 330                 335
   Gly Gln Gln Arg Gly Glu Gly Tyr Val Phe Tyr Glu Asn Arg Ala Tyr
   340 345 350
   Gly Val Ala Gly Lys Ser Leu Phe Asp Asp Gly Leu Gly Ala Ala Pro
   355 360 365
   Gly Val Pro Ser Gly Arg Ser Lys Phe Ser Pro Asp Val Leu Glu Thr
   370 375 380
   Val Pro Ala Ser Pro Gly Leu Arg Arg Pro Ser Leu Gly Ala Val Glu
   385 390 395 400
   Arg Gln Asp Ser Gly Tyr Asp Ser Leu Asp Gly Val Gly Ser Arg Ser
   405 410 415
   Phe Ser Leu Gly Glu Val Ser Asp Met Ala Ala Val Glu Ala Ala Glu
   420 425 430
   Leu Glu Met Thr Arg Gln Val Leu His Ala Gly Ala Arg Gln Asp Asp
   435 440 445
   Ala Glu Pro Gly Val Ser Gly Ala Ser Ala His Trp Gly Gln Arg Ala
   450 455 460
   Leu Gln Gly Ala Gln Ala Val Ala Ala Ala Gln Arg Leu Val His Ala
   465 470 475 480
   Ile Ala Leu Met Thr Gln Phe Gly Arg Ala Gly Ser Thr Asn Thr Pro
   485 490 495
   Gln Glu Ala Ala Ser Leu Ser Ala Ala Val Phe Gly Leu Gly Glu Ala
   500 505 510
   Ser Ser Ala Val Ala Glu Thr Val Ser Gly Phe Phe Arg Gly Ser Ser
   515 520 525
   Arg Trp Ala Gly Gly Phe Gly Val Ala Gly Gly Ala Met Ala Leu Gly
   530 535 540
   Gly Gly Ile Ala Ala Ala Val Gly Ala Gly Met Ser Leu Thr Asp Asp
   545 550 555 560
   Ala Pro Ala Gly Gln Lys Ala Ala Ala Gly Ala Gln Ile Ala Leu Gln
   565 570 575
   Leu Thr Gly Gly Thr Val Glu Leu Ala Ser Ser Ile Ala Leu Ala Leu
   580 585 590
   Ala Ala Ala Arg Gly Val Thr Ser Gly Leu Gln Val Ala Gly Ala Ser
   595 600 605
   Ala Gly Ala Ala Ala Gly Ala Leu Ala Ala Ala Leu Ser Pro Met Glu
   610 615 620
   Ile Tyr Gly Leu Val Gln Gln Ser His Tyr Ala Asp Gln Leu Asp Lys
   625 630 635 640
   Leu Ala Gln Glu Ser Ser Ala Tyr Gly Tyr Glu Gly Asp Ala Leu Leu
   645 650 655
   Ala Gln Leu Tyr Arg Asp Lys Thr Ala Ala Glu Gly Ala Val Ala Gly
   660 665 670
   Val Ser Ala Val Leu Ser Thr Val Gly Ala Ala Val Ser Ile Ala Ala
   675 680 685
   Ala Ala Ser Val Val Gly Ala Pro Val Ala Val Val Thr Ser Leu Leu
   690 695 700
   Thr Gly Ala Leu Asn Gly Ile Leu Arg Gly Val Gln Gln Pro Ile Ile
   705 710 715 720
   Glu Lys Leu Ala Asn Asp Tyr Ala Arg Lys Ile Asp Glu Leu Gly Gly
   725 730 735
   Pro Gln Ala Tyr Phe Glu Lys Asn Leu Gln Ala Arg His Glu Gln Leu
   740 745 750
   Ala Asn Ser Asp Gly Leu Arg Lys Met Leu Ala Asp Leu Gln Ala Gly
   755 760 765
   Trp Asn Ala Ser Ser Val Ile Gly Val Gln Thr Thr Glu Ile Ser Lys
   770 775 780
   Ser Ala Leu Glu Leu Ala Ala Ile Thr Gly Asn Ala Asp Asn Leu Lys
   785 790 795 800
   Ser Val Asp Val Phe Val Asp Arg Phe Val Gln Gly Glu Arg Val Ala
   805 810 815
   Gly Gln Pro Val Val Leu Asp Val Ala Ala Gly Gly Ile Asp Ile Ala
   820 825 830
   Ser Arg Lys Gly Glu Arg Pro Ala Leu Thr Phe Ile Thr Pro Leu Ala
           835                 840                 845
   Ala Pro Gly Glu Glu Gln Arg Arg Arg Thr Lys Thr Gly Arg Ser Glu
       850                 855                 860
   Phe Thr Thr Phe Val Glu Ile Val Gly Lys Gln Asp Arg Trp Arg Ile
   865                 870                 875                 880
   Arg Asp Gly Ala Ala Asp Thr Thr Ile Asp Leu Ala Lys Val Val Ser
                   885                 890                 895
   Gln Leu Val Asp Ala Asn Gly Val Leu Lys His Ser Ile Lys Leu Asp
               900                 905                 910
   Val Ile Gly Gly Asp Gly Asp Asp Val Val Leu Ala Asn Ala Ser Arg
           915                 920                 925
   Ile His Tyr Asp Gly Gly Ala Gly Thr Asn Thr Val Ser Tyr Ala Ala
       930                 935                 940
   Leu Gly Arg Gln Asp Ser Ile Thr Val Ser Ala Asp Gly Glu Arg Phe
   945                 950                 955                 960
   Asn Val Arg Lys Gln Leu Asn Asn Ala Asn Val Tyr Arg Glu Gly Val
                   965                 970                 975
   Ala Thr Gln Thr Thr Ala Tyr Gly Lys Arg Thr Glu Asn Val Gln Tyr
               980                 985                 990
   Arg His Val Glu Leu Ala Arg Val  Gly Gln Val Val Glu  Val Asp Thr
           995                 1000                1005
   Leu Glu  His Val Gln His Ile  Ile Gly Gly Ala Gly  Asn Asp Ser
       1010                1015                1020
   Ile Thr  Gly Asn Ala His Asp  Asn Phe Leu Ala Gly  Gly Ser Gly
       1025                1030                1035
   Asp Asp  Arg Leu Asp Gly Gly  Ala Gly Asn Asp Thr  Leu Val Gly
       1040                1045                1050
   Gly Glu  Gly Gln Asn Thr Val  Ile Gly Gly Ala Gly  Asp Asp Val
       1055                1060                1065
   Phe Leu  Gln Asp Leu Gly Val  Trp Ser Asn Gln Leu  Asp Gly Gly
       1070                1075                1080
   Ala Gly Val Asp Thr Val Lys Tyr Asn Val His Gln Pro Ser Glu
   1085 1090 1095
   Glu Arg Leu Glu Arg Met Gly Asp Thr Gly Ile His Ala Asp Leu
   1100 1105 1110
   Gln Lys Gly Thr Val Glu Lys Trp Pro Ala Leu Asn Leu Phe Ser
   1115 1120 1125
   Val Asp His Val Lys Asn Ile Glu Asn Leu His Gly Ser Arg Leu
   1130 1135 1140
   Asn Asp Arg Ile Ala Gly Asp Asp Gln Asp Asn Glu Leu Trp Gly
   1145 1150 1155
   His Asp Gly Asn Asp Thr Ile Arg Gly Arg Gly Gly Asp Asp Ile
   1160 1165 1170
   Leu Arg Gly Gly Leu Gly Leu Asp Thr Leu Tyr Gly Glu Asp Gly
   1175 1180 1185
   Asn Asp Ile Phe Leu Gln Asp Asp Glu Thr Val Ser Asp Asp Ile
   1190 1195 1200
   Asp Gly Gly Ala Gly Leu Asp Thr Val Asp Tyr Ser Ala Met Ile
   1205 1210 1215
   His Pro Gly Arg Ile Val Ala Pro His Glu Tyr Gly Phe Gly Ile
   1220 1225 1230
   Glu Ala Asp Leu Ser Arg Glu Trp Val Arg Lys Ala Ser Ala Leu
   1235 1240 1245
   Gly Val Asp Tyr Tyr Asp Asn Val Arg Asn Val Glu Asn Val Ile
   1250 1255 1260
   Gly Thr Ser Met Lys Asp Val Leu Ile Gly Asp Ala Gln Ala Asn
   1265 1270 1275
   Thr Leu Met Gly Gln Gly Gly Asp Asp Thr Val Arg Gly Gly Asp
   1280 1285 1290
   Gly Asp Asp Leu Leu Phe Gly Gly Asp Gly Asn Asp Met Leu Tyr
   1295 1300 1305
   Gly Asp Ala Gly Asn Asp Thr Leu Tyr Gly Gly Leu Gly Asp Asp
   1310 1315 1320
   Thr Leu  Glu Gly Gly Ala Gly  Asn Asp Trp Phe Gly  Gln Thr Gln
       1325                 1330                 1335
   Ala Arg  Glu His Asp Val Leu  Arg Gly Gly Asp Gly  Val Asp Thr
       1340                 1345                 1350
   Val Asp  Tyr Ser Gln Thr Gly  Ala His Ala Gly Ile  Ala Ala Gly
       1355                 1360                 1365
   Arg Ile  Gly Leu Gly Ile Leu  Ala Asp Leu Gly Ala  Gly Arg Val
       1370                 1375                 1380
   Asp Lys  Leu Gly Glu Ala Gly  Ser Ser Ala Tyr Asp  Thr Val Ser
       1385                 1390                 1395
   Gly Ile  Glu Asn Val Val Gly  Thr Glu Leu Ala Asp  Arg Ile Thr
       1400                 1405                 1410
   Gly Asp  Ala Gln Ala Asn Val  Leu Arg Gly Ala Gly  Gly Ala Asp
       1415                 1420                 1425
   Val Leu  Ala Gly Gly Glu Gly  Asp Asp Val Leu Leu  Gly Gly Asp
       1430                 1435                 1440
   Gly Asp  Asp Gln Leu Ser Gly  Asp Ala Gly Arg Asp  Arg Leu Tyr
       1445                 1450                 1455
   Gly Glu  Ala Gly Asp Asp Trp  Phe Phe Gln Asp Ala  Ala Asn Ala
       1460                 1465                 1470
   Gly Asn  Leu Leu Asp Gly Gly  Asp Gly Arg Asp Thr  Val Asp Phe
       1475                 1480                 1485
   Ser Gly  Pro Gly Arg Gly Leu  Asp Ala Gly Ala Lys  Gly Val Phe
       1490                 1495                 1500
   Leu Ser  Leu Gly Lys Gly Phe  Ala Ser Leu Met Asp  Glu Pro Glu
       1505                 1510                 1515
   Thr Ser  Asn Val Leu Arg Asn  Ile Glu Asn Ala Val  Gly Ser Ala
       1520                 1525                 1530
   Arg Asp  Asp Val Leu Ile Gly  Asp Ala Gly Ala Asn  Val Leu Asn
       1535                 1540                 1545
   Gly Leu  Ala Gly Asn Asp Val  Leu Ser Gly Gly Ala  Gly Asp Asp
   1550 1555 1560
   Val Leu Leu Gly Asp Glu Gly Ser Asp Leu Leu Ser Gly Asp Ala
   1565 1570 1575
   Gly Asn Asp Asp Leu Phe Gly Gly Gln Gly Asp Asp Thr Tyr Leu
   1580 1585 1590
   Phe Gly Val Gly Tyr Gly His Asp Thr Ile Tyr Glu Ser Gly Gly
   1595 1600 1605
   Gly His Asp Thr Ile Arg Ile Asn Ala Gly Ala Asp Gln Leu Trp
   1610 1615 1620
   Phe Ala Arg Gln Gly Asn Asp Leu Glu Ile Arg Ile Leu Gly Thr
   1625 1630 1635
   Asp Asp Ala Leu Thr Val His Asp Trp Tyr Arg Asp Ala Asp His
   1640 1645 1650
   Arg Val Glu Ile Ile His Ala Ala Asn Gln Ala Val Asp Gln Ala
   1655 1660 1665
   Gly Ile Glu Lys Leu Val Glu Ala Met Ala Gln Tyr Pro Asp Pro
   1670 1675 1680
   Gly Ala Ala Ala Ala Ala Pro Pro Ala Ala Arg Val Pro Asp Thr
   1685 1690 1695
   Leu Met Gln Ser Leu Ala Val Asn Trp Arg
   1700 1705
<210> 4
   <211> 1720
   <212> PRT
   <213> ARTIFICIAL
<220>
   <223> CyaA/2330VA/E570Q+K860R/AC-
<400> 4
   Met Gln Gln Ser His Gln Ala Gly Tyr Ala Asn Ala Ala Asp Arg Glu
   1               5                   10                  15
   Ser Gly Ile Pro Ala Ala Val Leu Asp Gly Ile Lys Ala Val Ala Lys
               20                  25                  30
   Glu Lys Asn Ala Thr Leu Met Phe Arg Leu Val Asn Pro His Ser Thr
           35                  40                  45
   Ser Leu Ile Ala Glu Gly Val Ala Thr Lys Gly Leu Gly Val His Ala
       50                  55                  60
   Lys Ser Ser Asp Trp Gly Leu Gln Ala Gly Tyr Ile Pro Val Asn Pro
   65                  70                  75                  80
   Asn Leu Ser Lys Leu Phe Gly Arg Ala Pro Glu Val Ile Ala Arg Ala
                   85                  90                  95
   Asp Asn Asp Val Asn Ser Ser Leu Ala His Gly His Thr Ala Val Asp
               100                 105                 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
                   165                 170                 175
   Ile Gly Asn Ala Ala Gly Ile Pro Leu Thr Ala Asp Gly Ser Ile Asp
               180                 185                 190
   Met Phe Ala Ile Met Pro His Leu Ser Asn Phe Arg Asp Ser Ala Arg
           195                 200                 205
   Ser Ser Val Thr Ser Gly Asp Ser Val Thr Asp Tyr Leu Ala Arg Thr
       210                 215                 220
   Arg Arg Ala Ala Ser Glu Ala Thr Gly Gly Val Leu Ser Ile Ile Asn
   225                 230                 235                 240
   Phe Glu Lys Leu Val His Leu Asp Arg Glu Arg Ile Asp Leu Leu Trp
                   245                 250                 255
   Lys Ile Ala Arg Ala Gly Ala Arg Ser Ala Val Gly Thr Glu Ala Arg
               260                 265                 270
   Arg Gln Phe Arg Tyr Asp Gly Asp Met Asn Ile Gly Val Ile Thr Asp
           275                 280                 285
   Phe Glu Leu Glu Val Arg Asn Ala Leu Asn Arg Arg Ala His Ala Val
   290 295 300
   Gly Ala Gln Asp Val Val Gln His Gly Thr Glu Gln Asn Asn Pro Phe
   305 310 315 320
   Pro Glu Ala Asp Glu Lys Ile Phe Val Val Ser Ala Thr Gly Glu Ser
   325 330 335
   Gln Met Leu Thr Arg Gly Gln Leu Lys Glu Tyr Ile Gly Gln Gln Arg
   340 345 350
   Gly Glu Gly Tyr Val Phe Tyr Glu Asn Arg Ala Tyr Gly Val Ala Gly
   355 360 365
   Lys Ser Leu Phe Asp Asp Gly Leu Gly Ala Ala Pro Gly Val Pro Ser
   370 375 380
   Gly Arg Ser Lys Phe Ser Pro Asp Val Leu Glu Thr Val Pro Ala Ser
   385 390 395 400
   Pro Gly Leu Arg Arg Pro Ser Leu Gly Ala Val Glu Arg Gln Asp Ser
   405 410 415
   Gly Tyr Asp Ser Leu Asp Gly Val Gly Ser Arg Ser Phe Ser Leu Gly
   420 425 430
   Glu Val Ser Asp Met Ala Ala Val Glu Ala Ala Glu Leu Glu Met Thr
   435 440 445
   Arg Gln Val Leu His Ala Gly Ala Arg Gln Asp Asp Ala Glu Pro Gly
   450 455 460
   Val Ser Gly Ala Ser Ala His Trp Gly Gln Arg Ala Leu Gln Gly Ala
   465 470 475 480
   Gln Ala Val Ala Ala Ala Gln Arg Leu Val His Ala Ile Ala Leu Met
   485 490 495
   Thr Gln Phe Gly Arg Ala Gly Ser Thr Asn Thr Pro Gln Glu Ala Ala
   500 505 510
   Ser Leu Ser Ala Ala Val Phe Gly Leu Gly Glu Ala Ser Ser Ala Val
   515 520 525
   Ala Glu Thr Val Ser Gly Phe Phe Arg Gly Ser Ser Arg Trp Ala Gly
   530 535 540
   Gly Phe Gly Val Ala Gly Gly Ala Met Ala Leu Gly Gly Gly Ile Ala
   545                 550                 555                 560
   Ala Ala Val Gly Ala Gly Met Ser Leu Thr Asp Asp Ala Pro Ala Gly
                   565                 570                 575
   Gln Lys Ala Ala Ala Gly Ala Gln Ile Ala Leu Gln Leu Thr Gly Gly
               580                 585                 590
   Thr Val Glu Leu Ala Ser Ser Ile Ala Leu Ala Leu Ala Ala Ala Arg
           595                 600                 605
   Gly Val Thr Ser Gly Leu Gln Val Ala Gly Ala Ser Ala Gly Ala Ala
       610                 615                 620
   Ala Gly Ala Leu Ala Ala Ala Leu Ser Pro Met Glu Ile Tyr Gly Leu
   625                 630                 635                 640
   Val Gln Gln Ser His Tyr Ala Asp Gln Leu Asp Lys Leu Ala Gln Glu
                   645                 650                 655
   Ser Ser Ala Tyr Gly Tyr Glu Gly Asp Ala Leu Leu Ala Gln Leu Tyr
               660                 665                 670
   Arg Asp Lys Thr Ala Ala Glu Gly Ala Val Ala Gly Val Ser Ala Val
           675                 680                 685
   Leu Ser Thr Val Gly Ala Ala Val Ser Ile Ala Ala Ala Ala Ser Val
       690                 695                 700
   Val Gly Ala Pro Val Ala Val Val Thr Ser Leu Leu Thr Gly Ala Leu
   705                 710                 715                 720
   Asn Gly Ile Leu Arg Gly Val Gln Gln Pro Ile Ile Glu Lys Leu Ala
                   725                 730                 735
   Asn Asp Tyr Ala Arg Lys Ile Asp Glu Leu Gly Gly Pro Gln Ala Tyr
               740                 745                 750
   Phe Glu Lys Asn Leu Gln Ala Arg His Glu Gln Leu Ala Asn Ser Asp
           755                 760                 765
   Gly Leu Arg Lys Met Leu Ala Asp Leu Gln Ala Gly Trp Asn Ala Ser
       770                 775                 780
   Ser Val Ile Gly Val Gln Thr Thr Glu Ile Ser Lys Ser Ala Leu Glu
   785                 790                 795                 800
   Leu Ala Ala Ile Thr Gly Asn Ala Asp Asn Leu Lys Ser Val Asp Val
   805 810 815
   Phe Val Asp Arg Phe Val Gln Gly Glu Arg Val Ala Gly Gln Pro Val
   820 825 830
   Val Leu Asp Val Ala Ala Gly Gly Ile Asp Ile Ala Ser Arg Lys Gly
   835 840 845
   Glu Arg Pro Ala Leu Thr Phe Ile Thr Pro Leu Ala Ala Pro Gly Glu
   850 855 860
   Glu Gln Arg Arg Arg Thr Lys Thr Gly Arg Ser Glu Phe Thr Thr Phe
   865 870 875 880
   Val Glu Ile Val Gly Lys Gln Asp Arg Trp Arg Ile Arg Asp Gly Ala
   885 890 895
   Ala Asp Thr Thr Ile Asp Leu Ala Lys Val Val Ser Gln Leu Val Asp
   900 905 910
   Ala Asn Gly Val Leu Lys His Ser Ile Lys Leu Asp Val Ile Gly Gly
   915 920 925
   Asp Gly Asp Asp Val Val Leu Ala Asn Ala Ser Arg Ile His Tyr Asp
   930 935 940
   Gly Gly Ala Gly Thr Asn Thr Val Ser Tyr Ala Ala Leu Gly Arg Gln
   945 950 955 960
   Asp Ser Ile Thr Val Ser Ala Asp Gly Glu Arg Phe Asn Val Arg Lys
   965 970 975
   Gln Leu Asn Asn Ala Asn Val Tyr Arg Glu Gly Val Ala Thr Gln Thr
   980 985 990
   Thr Ala Tyr Gly Lys Arg Thr Glu Asn Val Gln Tyr Arg His Val Glu
   995 1000 1005
   Leu Ala Arg Val Gly Gln Val Val Glu Val Asp Thr Leu Glu His
   1010 1015 1020
   Val Gln His Ile Ile Gly Gly Ala Gly Asn Asp Ser Ile Thr Gly
   1025 1030 1035
   Asn Ala His Asp Asn Phe Leu Ala Gly Gly Ser Gly Asp Asp Arg
   1040 1045 1050
   Leu Asp Gly Gly Ala Gly Asn Asp Thr Leu Val Gly Gly Glu Gly
   1055 1060 1065
   Gln Asn Thr Val Ile Gly Gly Ala Gly Asp Asp Val Phe Leu Gln
   1070 1075 1080
   Asp Leu Gly Val Trp Ser Asn Gln Leu Asp Gly Gly Ala Gly Val
   1085 1090 1095
   Asp Thr Val Lys Tyr Asn Val His Gln Pro Ser Glu Glu Arg Leu
   1100 1105 1110
   Glu Arg Met Gly Asp Thr Gly Ile His Ala Asp Leu Gln Lys Gly
   1115 1120 1125
   Thr Val Glu Lys Trp Pro Ala Leu Asn Leu Phe Ser Val Asp His
   1130 1135 1140
   Val Lys Asn Ile Glu Asn Leu His Gly Ser Arg Leu Asn Asp Arg
   1145 1150 1155
   Ile Ala Gly Asp Asp Gln Asp Asn Glu Leu Trp Gly His Asp Gly
   1160 1165 1170
   Asn Asp Thr Ile Arg Gly Arg Gly Gly Asp Asp Ile Leu Arg Gly
   1175 1180 1185
   Gly Leu Gly Leu Asp Thr Leu Tyr Gly Glu Asp Gly Asn Asp Ile
   1190 1195 1200
   Phe Leu Gln Asp Asp Glu Thr Val Ser Asp Asp Ile Asp Gly Gly
   1205 1210 1215
   Ala Gly Leu Asp Thr Val Asp Tyr Ser Ala Met Ile His Pro Gly
   1220 1225 1230
   Arg Ile Val Ala Pro His Glu Tyr Gly Phe Gly Ile Glu Ala Asp
   1235 1240 1245
   Leu Ser Arg Glu Trp Val Arg Lys Ala Ser Ala Leu Gly Val Asp
   1250 1255 1260
   Tyr Tyr Asp Asn Val Arg Asn Val Glu Asn Val Ile Gly Thr Ser
   1265 1270 1275
   Met Lys Asp Val Leu Ile Gly Asp Ala Gln Ala Asn Thr Leu Met
   1280 1285 1290
   Gly Gln Gly Gly Asp Asp Thr Val Arg Gly Gly Asp Gly Asp Asp
   1295 1300 1305
   Leu Leu Phe Gly Gly Asp Gly Asn Asp Met Leu Tyr Gly Asp Ala
   1310 1315 1320
   Gly Asn Asp Thr Leu Tyr Gly Gly Leu Gly Asp Asp Thr Leu Glu
   1325 1330 1335
   Gly Gly Ala Gly Asn Asp Trp Phe Gly Gln Thr Gln Ala Arg Glu
   1340 1345 1350
   His Asp Val Leu Arg Gly Gly Asp Gly Val Asp Thr Val Asp Tyr
   1355 1360 1365
   Ser Gln Thr Gly Ala His Ala Gly Ile Ala Ala Gly Arg Ile Gly
   1370 1375 1380
   Leu Gly Ile Leu Ala Asp Leu Gly Ala Gly Arg Val Asp Lys Leu
   1385 1390 1395
   Gly Glu Ala Gly Ser Ser Ala Tyr Asp Thr Val Ser Gly Ile Glu
   1400 1405 1410
   Asn Val Val Gly Thr Glu Leu Ala Asp Arg Ile Thr Gly Asp Ala
   1415 1420 1425
   Gln Ala Asn Val Leu Arg Gly Ala Gly Gly Ala Asp Val Leu Ala
   1430 1435 1440
   Gly Gly Glu Gly Asp Asp Val Leu Leu Gly Gly Asp Gly Asp Asp
   1445 1450 1455
   Gln Leu Ser Gly Asp Ala Gly Arg Asp Arg Leu Tyr Gly Glu Ala
   1460 1465 1470
   Gly Asp Asp Trp Phe Phe Gln Asp Ala Ala Asn Ala Gly Asn Leu
   1475 1480 1485
   Leu Asp Gly Gly Asp Gly Arg Asp Thr Val Asp Phe Ser Gly Pro
   1490 1495 1500
   Gly Arg Gly Leu Asp Ala Gly Ala Lys Gly Val Phe Leu Ser Leu
   1505 1510 1515
   Gly Lys Gly Phe Ala Ser Leu Met Asp Glu Pro Glu Thr Ser Asn
   1520 1525 1530
   Val Leu Arg Asn Ile Glu Asn Ala Val Gly Ser Ala Arg Asp Asp
   1535 1540 1545
   Val Leu Ile Gly Asp Ala Gly Ala Asn Val Leu Asn Gly Leu Ala
   1550 1555 1560
   Gly Asn Asp Val Leu Ser Gly Gly Ala Gly Asp Asp Val Leu Leu
   1565 1570 1575
   Gly Asp Glu Gly Ser Asp Leu Leu Ser Gly Asp Ala Gly Asn Asp
   1580 1585 1590
   Asp Leu Phe Gly Gly Gln Gly Asp Asp Thr Tyr Leu Phe Gly Val
   1595 1600 1605
   Gly Tyr Gly His Asp Thr Ile Tyr Glu Ser Gly Gly Gly His Asp
   1610 1615 1620
   Thr Ile Arg Ile Asn Ala Gly Ala Asp Gln Leu Trp Phe Ala Arg
   1625 1630 1635
   Gln Gly Asn Asp Leu Glu Ile Arg Ile Leu Gly Thr Asp Asp Ala
   1640 1645 1650
   Leu Thr Val His Asp Trp Tyr Arg Asp Ala Asp His Arg Val Glu
   1655 1660 1665
   Ile Ile His Ala Ala Asn Gln Ala Val Asp Gln Ala Gly Ile Glu
   1670 1675 1680
   Lys Leu Val Glu Ala Met Ala Gln Tyr Pro Asp Pro Gly Ala Ala
   1685 1690 1695
   Ala Ala Ala Pro Pro Ala Ala Arg Val Pro Asp Thr Leu Met Gln
   1700 1705 1710
   Ser Leu Ala Val Asn Trp Arg
   1715 1720
<210> 5
   <211> 8825
   <212> DNA
   <213> Artificial
<220>
   <223> QR AC-
<400> 5
   cggaagagcg cccaatacgc aaaccgcctc tccccgcgcg ttggccgatt cattaatgca      60
   gctggcacga caggtttccc gactggaaag cgggcagtga gcgcaacgca attaatgtga     120
   gttagctcac tcattaggca ccccaggctt tacactttat gcttccggct cgtatgttgt     180
   gtggaattgt gagcggataa caatttcaca caggaaacag ctatgaccat gattacgaat     240
   ttaatacgac tcactatagg gaaagctcta gaaataattt tgtttaactt taagaaggag     300
   atatacatat gcttccgtcc gcccaagcgc cctccctcct caatcccacc gacgacttcg     360
   cggcactggg caatattgcc tggctgtgga tgaactctcc catgcaccgc gactggccgg     420
   tgcatctgct cgcacgcaac acgctcgcgc cgattcaact gggccaatac attctgctgc     480
   gatgcaatga cgtgccggtt gcatactgca gctgggccct aatggacgcc gacaccgaac     540
   tctcctatgt catggcgccc tcgtcgctgg gcgggaatgc ctggaactgc ggcgaccgac     600
   tgtggatcat cgactggatc gcgccattct cgcgcgacga caatcgtgcg ctgcgccgcg     660
   cgctggccga acggcacccc gacagcgtgg gccgttcgct gcgcgttcgg cgcggcggcg     720
   acaccgcgcg cgtcaaggag taccgaggcc gcgcgctgga cgcggccgcc actcgcgcgc     780
   agctggaccg ctaccatgcc gaactgatcg caggactgcg cgcgagcaac ggcggatacg     840
   cgccgcgagg ccggggcacc gcctaaggat cctctagagc ttgcatgccc tggcacgaca     900
   ggtttcccga ctggaaagcg ggcagtgagc gcaacgcaat taatgtgagt tagctcactc     960
   attaggcacc ccaggcttta cactttatgc ttccggctcg tatgttgtgt ggaattgtga    1020
   gcggataaca atttcacaca ggaaacagct atgaccatgc agcaatcgca tcaggctggt    1080
   tacgcaaacg ccgccgaccg ggagtctggc atccccgcag ccgtactcga tggcatcaag    1140
   gccgtggcga aggaaaaaaa cgccacattg atgttccgcc tggtcaaccc ccattccacc    1200
   agcctgattg ccgaaggggt ggccaccaaa ggattgggcg tgcacgccaa gtcgtccgat    1260
   tgggggttgc aggcgggcta cattcccgtc aacccgaatc tttccaaact gttcggccgt    1320
   gcgcccgagg tgatcgcgcg ggccgacaac gacgtcaaca gcagcctggc gcatggccat    1380
   accgcggtcg acctgacgct gtcgaaagag cggcttgact atctgcggca agcgggcctg    1440
   gtcaccggca tggccgatgg cgtggtcgcg agcaaccacg caggctacga gcagttcgag    1500
   tttcgcgtga aggaaacctc ggacgggcgc tatgccgtgc agtatcgccg caagggcggc    1560
   gacgatttcg aggcggtcaa ggtgatcggc aatgccgccg gtattccact gacggcggat    1620
   ggatccatcg acatgttcgc cattatgccg catctgtcca acttccgcga ctcggcgcgc    1680
   agttcggtga ccagcggcga ttcggtgacc gattacctgg cgcgcacgcg gcgggccgcc    1740
   agcgaggcca cgggcggtgt acacctggat cgcgaacgca tcgacttgtt gtggaaaatc    1800
   gctcgcgccg gcgcccgttc cgcagtgggc accgaggcgc gtcgccagtt ccgctacgac    1860
   ggcgacatga atatcggcgt gatcaccgat ttcgagctgg aagtgcgcaa tgcgctgaac 1920
   aggcgggcgc acgccgtcgg cgcgcaggac gtggtccagc atggcactga gcagaacaat 1980
   cctttcccgg aggcagatga gaagattttc gtcgtatcgg ccaccggtga aagccagatg 2040
   ctcacgcgcg ggcaactgaa ggaatacatt ggccagcagc gcggcgaggg ctatgtcttc 2100
   tacgagaacc gtgcatacgg cgtggcgggg aaaagcctgt tcgacgatgg gctgggagcc 2160
   gcgcccggcg tgccgagcgg acgttcgaag ttctcgccgg atgtactgga aacggtgccg 2220
   gcgtcacccg gattgcggcg gccgtcgctg ggcgcagtgg aacgccagga ttccggctat 2280
   gacagccttg atggggtggg atcgcgatcg ttctcgttgg gcgaggtgtc cgacatggcc 2340
   gccgtggaag cggcggaact ggaaatgacc cggcaagtct tgcacgccgg ggcgcggcag 2400
   gacgatgccg agccgggcgt gagcggtgcg tcggcgcact gggggcagcg ggcgctgcag 2460
   ggcgcccagg cggtggcggc ggcgcagcgg ctggttcatg ccattgccct gatgacgcaa 2520
   ttcggccggg ccggttccac caacacgccg caggaagcgg cctcgttgtc ggcggccgtg 2580
   ttcggcttgg gcgaggccag cagcgccgtg gccgaaaccg tgagcggttt tttccgcggg 2640
   tcttcgcgct gggccggcgg tttcggcgtg gctggcggcg cgatggcgct gggaggcggc 2700
   atcgccgcgg ccgttggcgc cgggatgtcg ttgaccgatg acgcgccggc cggacagaag 2760
   gccgccgccg gagctccgat cgcgctgcag ttaacgggtg gaacggtcga gctggcttct 2820
   tccatcgcgt tggcgctggc cgcggcgcgc ggcgtgacca gcggcttgca ggtggccggg 2880
   gcgtcggccg gggcggctgc cggcgcattg gccgcggcgc tcagtcccat ggagatctac 2940
   ggcctggtgc agcaatcgca ctatgcggat cagctggaca agctggcgca ggaatcgagc 3000
   gcatacggtt acgagggcga cgccttgctg gcccagctgt atcgcgacaa gacggccgcc 3060
   gagggcgccg tcgccggcgt ctccgccgtc ctgagcacgg tgggggcggc ggtgtcgatc 3120
   gccgcggcgg ccagcgtggt aggggccccg gtggcggtgg tcacttcctt gctgaccggg 3180
   gctctcaacg gcatcctgcg cggcgtgcag cagcccatca tcgaaaagct ggccaacgat 3240
   tacgctcgca agatcgacga gctgggcggg ccgcaagcgt acttcgagaa aaacctgcag 3300
   gcgcgtcacg aacaactggc caattcggac ggcctacgga aaatgctggc cgacctgcag 3360
   gccggttgga acgccagcag cgtgatcggg gtgcagacga cagagatctc caagtcggcg 3420
   ctcgaactgg ccgccattac cggcaacgcg gacaacctga aatccgtcga cgtgttcgtg 3480
   gaccgcttcg tccagggcga gcgggtggcc ggccagccgg tggtcctcga cgtcgccgcc 3540
   ggcggcatcg atatcgccag ccgcaagggc gagcggccgg cgctgacgtt catcacgccg 3600
   ctggccgcgc caggagaaga gcagcgccgg cgcacgaaaa cgggcagatc tgaattcacc 3660
   acattcgtcg agatcgtggg caagcaggac cgctggcgca tccgggacgg cgcggccgac 3720
   accaccatcg atctggccaa ggtggtgtcg caactggtcg acgccaatgg cgtgctcaag 3780
   cacagcatca aactggatgt gatcggcgga gatggcgatg acgtcgtgct tgccaatgct 3840
   tcgcgcatcc attatgacgg cggcgcgggc accaacacgg tcagctatgc cgccctgggt 3900
   cgacaggatt ccattaccgt gtccgccgac ggggaacgtt tcaacgtgcg caagcagttg 3960
   aacaacgcca acgtgtatcg cgaaggcgtg gctacccaga caaccgccta cggcaagcgc 4020
   acggagaatg tccaataccg ccatgtcgag ctggcccgtg tcgggcaagt ggtggaggtc 4080
   gacacgctcg agcatgtgca gcacatcatc ggcggggccg gcaacgattc gatcaccggc 4140
   aatgcgcacg acaacttcct agccggcggg tcgggcgacg acaggctgga tggcggcgcc 4200
   ggcaacgaca ccctggttgg cggcgagggc caaaacacgg tcatcggcgg cgccggcgac 4260
   gacgtattcc tgcaggacct gggggtatgg agcaaccagc tcgatggcgg cgcgggcgtc 4320
   gataccgtga agtacaacgt gcaccagcct tccgaggagc gcctcgaacg catgggcgac 4380
   acgggcatcc atgccgatct tcaaaagggc acggtcgaga agtggccggc cctgaacctg 4440
   ttcagcgtcg accatgtcaa gaatatcgag aatctgcacg gctcccgcct aaacgaccgc 4500
   atcgccggcg acgaccagga caacgagctc tggggccacg atggcaacga cacgatacgc 4560
   ggccggggcg gcgacgacat cctgcgcggc ggcctgggcc tggacacgct gtatggcgag 4620
   gacggcaacg acatcttcct gcaggacgac gagaccgtca gcgatgacat cgacggcggc 4680
   gcggggctgg acaccgtcga ctactccgcc atgatccatc caggcaggat cgttgcgccg 4740
   catgaatacg gcttcgggat cgaggcggac ctgtccaggg aatgggtgcg caaggcgtcc 4800
   gcgctgggcg tggactatta cgataatgtc cgcaatgtcg aaaacgtcat cggtacgagc 4860
   atgaaggatg tgctcatcgg cgacgcgcaa gccaataccc tgatgggcca gggcggcgac 4920
   gataccgtgc gcggcggcga cggcgatgat ctgctgttcg gcggcgacgg caacgacatg 4980
   ctgtatggcg acgccggcaa cgacaccctc tacggggggc tgggcgacga tacccttgaa 5040
   ggcggcgcgg gcaacgattg gttcggccag acgcaggcgc gcgagcatga cgtgctgcgc 5100
   ggcggagatg gggtggatac cgtcgattac agccagaccg gcgcgcatgc cggcattgcc 5160
   gcgggtcgca tcgggctggg catcctggct gacctgggcg ccggccgcgt cgacaagctg 5220
   ggcgaggccg gcagcagcgc ctacgatacg gtttccggta tcgagaacgt ggtgggcacg 5280
   gaactggccg accgcatcac gggcgatgcg caggccaacg tgctgcgcgg cgcgggtggc 5340
   gccgacgtgc ttgcgggcgg cgagggcgac gatgtgctgc tgggcggcga cggcgacgac 5400
   cagctgtcgg gcgacgccgg acgcgatcgc ttgtacggcg aagccggtga cgactggttc 5460
   ttccaggatg ccgccaatgc cggcaatctg ctcgacggcg gcgacggccg cgataccgtg 5520
   gatttcagcg gcccgggccg gggcctcgac gccggcgcaa agggcgtatt cctgagcttg 5580
   ggcaaggggt tcgccagcct gatggacgaa cccgaaacca gcaacgtgtt gcgcaatatc 5640
   gagaacgccg tgggcagcgc gcgtgatgac gtgctgatcg gcgacgcagg cgccaacgtc 5700
   ctcaatggcc tggcgggcaa cgacgtgctg tccggcggcg ctggcgacga tgtgctgctg 5760
   ggcgacgagg gctcggacct gctcagcggc gatgcgggca acgacgatct gttcggcggg 5820
   cagggcgatg atacttatct gttcggggtc gggtacgggc acgacacgat ctacgaatcg 5880
   ggcggcggcc atgacaccat ccgcatcaac gcgggggcgg accagctgtg gttcgcgcgc 5940
   cagggcaacg acctggagat ccgcattctc ggcaccgacg atgcacttac cgtgcacgac 6000
   tggtatcgcg acgccgatca ccgggtggaa atcatccatg ccgccaacca ggcggtagac 6060
   caggcaggca tcgaaaagct ggtcgaggca atggcgcagt atccggaccc cggcgcggcg 6120
   gcggctgccc cgccggcggc gcgcgtgccg gacacgctga tgcagtccct ggctgtcaac 6180
   tggcgctgaa gcgccgtgaa tcacggcccg cctgcctcgc gcggcggcgc cgtctctttg 6240
   cgttcttctc cgaggtattt cccatcatga attcactggc cgtcgtttta caacgtcgtg 6300
   actgggaaaa ccctggcgtt acccaactta atcgccttgc agcacatccc cctttcgcca 6360
   gctggcgtaa tagcgaagag gcccgcaccg atcgcccttc ccaacagttg cgcagcctga 6420
   atggcgaatg ggaaattgta aacgttaata ttttgttaat attttgttaa aattcgcgtt 6480
   aaatttttgt taaatcagct cattttttaa ccaataggcc gaaatcggca aaatccctta 6540
   taaatcaaaa gaatagaccg agatagggtt gagtgttgtt ccagtttgga acaagagtcc 6600
   actattaaag aacgtggact ccaacgtcaa agggcgaaaa accgtctatc agggcgatgg 6660
   cccactacgt gaaccatcac cctaatcaag ttttttgggg tcgaggtgcc gtaaagcact 6720
   aaatcggaac cctaaaggga tgccccgatt tagagcttga cggggaaagc cggcgaacgt 6780
   ggcgagaaag gaagggaaga aagcgaaagg agcgggcgct agggcgctgg caagtgtagc 6840
   ggtcacgctg cgcgtaacca ccacacccgc cgcgcttaat gcgccgctac agggcgcgtc 6900
   aggtggcact tttcggggaa atgtgcgcgg aacccctatt tgtttatttt tctaaataca 6960
   ttcaaatatg tatccgctca tgagacaata accctgataa atgcttcaat aatattgaaa 7020
   aaggaagagt atgagtattc aacatttccg tgtcgccctt attccctttt ttgcggcatt 7080
   ttgccttcct gtttttgctc acccagaaac gctggtgaaa gtaaaagatg ctgaagatca 7140
   gttgggtgca cgagtgggtt acatcgaact ggatctcaac agcggtaaga tccttgagag 7200
   ttttcgcccc gaagaacgtt ttccaatgat gagcactttt aaagttctgc tatgtggcgc 7260
   ggtattatcc cgtattgacg ccgggcaaga gcaactcggt cgccgcatac actattctca 7320
   gaatgacttg gttgagtact caccagtcac agaaaagcat cttacggatg gcatgacagt 7380
   aagagaatta tgcagtgctg ccataaccat gagtgataac actgcggcca acttacttct 7440
   gacaacgatc ggaggaccga aggagctaac cgcttttttg cacaacatgg gggatcatgt 7500
   aactcgcctt gatcgttggg aaccggagct gaatgaagcc ataccaaacg acgagcgtga 7560
   caccacgatg cctgtagcaa tggcaacaac gttgcgcaaa ctattaactg gcgaactact 7620
   tactctagct tcccggcaac aattaataga ctggatggag gcggataaag ttgcaggacc 7680
   acttctgcgc tcggcccttc cggctggctg gtttattgct gataaatctg gagccggtga 7740
   gcgtgggtct cgcggtatca ttgcagcact ggggccagat ggtaagccct cccgtatcgt 7800
   agttatctac acgacgggga gtcaggcaac tatggatgaa cgaaatagac agatcgctga 7860
   gataggtgcc tcactgatta agcattggta actgtcagac caagtttact catatatact 7920
   ttagattgat ttaaaacttc atttttaatt taaaaggatc taggtgaaga tcctttttga 7980
   taatctcatg accaaaatcc cttaacgtga gttttcgttc cactgagcgt cagaccccgt 8040
   agaaaagatc aaaggatctt cttgagatcc tttttttctg cgcgtaatct gctgcttgca 8100
   aacaaaaaaa ccaccgctac cagcggtggt ttgtttgccg gatcaagagc taccaactct 8160
   ttttccgaag gtaactggct tcagcagagc gcagatacca aatactgtcc ttctagtgta 8220
   gccgtagtta ggccaccact tcaagaactc tgtagcaccg cctacatacc tcgctctgct 8280
   aatcctgtta ccagtggctg ctgccagtgg cgataagtcg tgtcttaccg ggttggactc 8340
   aagacgatag ttaccggata aggcgcagcg gtcgggctga acggggggtt cgtgcacaca 8400
   gcccagcttg gagcgaacga cctacaccga actgagatac ctacagcgtg agctatgaga 8460
   aagcgccacg cttcccgaag ggagaaaggc ggacaggtat ccggtaagcg gcagggtcgg 8520
   aacaggagag cgcacgaggg agcttccagg gggaaacgcc tggtatcttt atagtcctgt 8580
   cgggtttcgc cacctctgac ttgagcgtcg atttttgtga tgctcgtcag gggggcggag 8640
   cctatggaaa aacgccagca acgcggcctt tttacggttc ctggcctttt gctggccttt 8700
   tgctcacatg ttctttcctg cgttatcccc tgattctgtg gataaccgta ttaccgcctt 8760
   tgagtgagct gataccgctc gccgcagccg aacgaccgag cgcagcgagt cagtgagcga 8820
   ggaag 8825
<210> 6
   <211> 8855
   <212> DNA
   <213> Artificial
<220>
   <223> OVA QR AC-
<400> 6
   cggaagagcg cccaatacgc aaaccgcctc tccccgcgcg ttggccgatt cattaatgca    60
   gctggcacga caggtttccc gactggaaag cgggcagtga gcgcaacgca attaatgtga   120
   gttagctcac tcattaggca ccccaggctt tacactttat gcttccggct cgtatgttgt   180
   gtggaattgt gagcggataa caatttcaca caggaaacag ctatgaccat gattacgaat   240
   ttaatacgac tcactatagg gaaagctcta gaaataattt tgtttaactt taagaaggag 300
   atatacatat gcttccgtcc gcccaagcgc cctccctcct caatcccacc gacgacttcg 360
   cggcactggg caatattgcc tggctgtgga tgaactctcc catgcaccgc gactggccgg 420
   tgcatctgct cgcacgcaac acgctcgcgc cgattcaact gggccaatac attctgctgc 480
   gatgcaatga cgtgccggtt gcatactgca gctgggccct aatggacgcc gacaccgaac 540
   tctcctatgt catggcgccc tcgtcgctgg gcgggaatgc ctggaactgc ggcgaccgac 600
   tgtggatcat cgactggatc gcgccattct cgcgcgacga caatcgtgcg ctgcgccgcg 660
   cgctggccga acggcacccc gacagcgtgg gccgttcgct gcgcgttcgg cgcggcggcg 720
   acaccgcgcg cgtcaaggag taccgaggcc gcgcgctgga cgcggccgcc actcgcgcgc 780
   agctggaccg ctaccatgcc gaactgatcg caggactgcg cgcgagcaac ggcggatacg 840
   cgccgcgagg ccggggcacc gcctaaggat cctctagagc ttgcatgccc tggcacgaca 900
   ggtttcccga ctggaaagcg ggcagtgagc gcaacgcaat taatgtgagt tagctcactc 960
   attaggcacc ccaggcttta cactttatgc ttccggctcg tatgttgtgt ggaattgtga 1020
   gcggataaca atttcacaca ggaaacagct atgaccatgc agcaatcgca tcaggctggt 1080
   tacgcaaacg ccgccgaccg ggagtctggc atccccgcag ccgtactcga tggcatcaag 1140
   gccgtggcga aggaaaaaaa cgccacattg atgttccgcc tggtcaaccc ccattccacc 1200
   agcctgattg ccgaaggggt ggccaccaaa ggattgggcg tgcacgccaa gtcgtccgat 1260
   tgggggttgc aggcgggcta cattcccgtc aacccgaatc tttccaaact gttcggccgt 1320
   gcgcccgagg tgatcgcgcg ggccgacaac gacgtcaaca gcagcctggc gcatggccat 1380
   accgcggtcg acctgacgct gtcgaaagag cggcttgact atctgcggca agcgggcctg 1440
   gtcaccggca tggccgatgg cgtggtcgcg agcaaccacg caggctacga gcagttcgag 1500
   tttcgcgtga aggaaacctc ggacgggcgc tatgccgtgc agtatcgccg caagggcggc 1560
   gacgatttcg aggcggtcaa ggtgatcggc aatgccgccg gtattccact gacggcggat 1620
   ggatccatcg acatgttcgc cattatgccg catctgtcca acttccgcga ctcggcgcgc 1680
   agttcggtga ccagcggcga ttcggtgacc gattacctgg cgcgcacgcg gcgggccgcc 1740
   agcgaggcca cgggcggtgt actctcaata attaatttcg aaaagcttgt acacctggat 1800
   cgcgaacgca tcgacttgtt gtggaaaatc gctcgcgccg gcgcccgttc cgcagtgggc 1860
   accgaggcgc gtcgccagtt ccgctacgac ggcgacatga atatcggcgt gatcaccgat 1920
   ttcgagctgg aagtgcgcaa tgcgctgaac aggcgggcgc acgccgtcgg cgcgcaggac 1980
   gtggtccagc atggcactga gcagaacaat cctttcccgg aggcagatga gaagattttc 2040
   gtcgtatcgg ccaccggtga aagccagatg ctcacgcgcg ggcaactgaa ggaatacatt 2100
   ggccagcagc gcggcgaggg ctatgtcttc tacgagaacc gtgcatacgg cgtggcgggg 2160
   aaaagcctgt tcgacgatgg gctgggagcc gcgcccggcg tgccgagcgg acgttcgaag 2220
   ttctcgccgg atgtactgga aacggtgccg gcgtcacccg gattgcggcg gccgtcgctg 2280
   ggcgcagtgg aacgccagga ttccggctat gacagccttg atggggtggg atcgcgatcg 2340
   ttctcgttgg gcgaggtgtc cgacatggcc gccgtggaag cggcggaact ggaaatgacc 2400
   cggcaagtct tgcacgccgg ggcgcggcag gacgatgccg agccgggcgt gagcggtgcg 2460
   tcggcgcact gggggcagcg ggcgctgcag ggcgcccagg cggtggcggc ggcgcagcgg 2520
   ctggttcatg ccattgccct gatgacgcaa ttcggccggg ccggttccac caacacgccg 2580
   caggaagcgg cctcgttgtc ggcggccgtg ttcggcttgg gcgaggccag cagcgccgtg 2640
   gccgaaaccg tgagcggttt tttccgcggg tcttcgcgct gggccggcgg tttcggcgtg 2700
   gctggcggcg cgatggcgct gggaggcggc atcgccgcgg ccgttggcgc cgggatgtcg 2760
   ttgaccgatg acgcgccggc cggacagaag gccgccgccg gagctccgat cgcgctgcag 2820
   ttaacgggtg gaacggtcga gctggcttct tccatcgcgt tggcgctggc cgcggcgcgc 2880
   ggcgtgacca gcggcttgca ggtggccggg gcgtcggccg gggcggctgc cggcgcattg 2940
   gccgcggcgc tcagtcccat ggagatctac ggcctggtgc agcaatcgca ctatgcggat 3000
   cagctggaca agctggcgca ggaatcgagc gcatacggtt acgagggcga cgccttgctg 3060
   gcccagctgt atcgcgacaa gacggccgcc gagggcgccg tcgccggcgt ctccgccgtc 3120
   ctgagcacgg tgggggcggc ggtgtcgatc gccgcggcgg ccagcgtggt aggggccccg 3180
   gtggcggtgg tcacttcctt gctgaccggg gctctcaacg gcatcctgcg cggcgtgcag 3240
   cagcccatca tcgaaaagct ggccaacgat tacgctcgca agatcgacga gctgggcggg 3300
   ccgcaagcgt acttcgagaa aaacctgcag gcgcgtcacg aacaactggc caattcggac 3360
   ggcctacgga aaatgctggc cgacctgcag gccggttgga acgccagcag cgtgatcggg 3420
   gtgcagacga cagagatctc caagtcggcg ctcgaactgg ccgccattac cggcaacgcg 3480
   gacaacctga aatccgtcga cgtgttcgtg gaccgcttcg tccagggcga gcgggtggcc 3540
   ggccagccgg tggtcctcga cgtcgccgcc ggcggcatcg atatcgccag ccgcaagggc 3600
   gagcggccgg cgctgacgtt catcacgccg ctggccgcgc caggagaaga gcagcgccgg 3660
   cgcacgaaaa cgggcagatc tgaattcacc acattcgtcg agatcgtggg caagcaggac 3720
   cgctggcgca tccgggacgg cgcggccgac accaccatcg atctggccaa ggtggtgtcg 3780
   caactggtcg acgccaatgg cgtgctcaag cacagcatca aactggatgt gatcggcgga 3840
   gatggcgatg acgtcgtgct tgccaatgct tcgcgcatcc attatgacgg cggcgcgggc 3900
   accaacacgg tcagctatgc cgccctgggt cgacaggatt ccattaccgt gtccgccgac 3960
   ggggaacgtt tcaacgtgcg caagcagttg aacaacgcca acgtgtatcg cgaaggcgtg 4020
   gctacccaga caaccgccta cggcaagcgc acggagaatg tccaataccg ccatgtcgag 4080
   ctggcccgtg tcgggcaagt ggtggaggtc gacacgctcg agcatgtgca gcacatcatc 4140
   ggcggggccg gcaacgattc gatcaccggc aatgcgcacg acaacttcct agccggcggg 4200
   tcgggcgacg acaggctgga tggcggcgcc ggcaacgaca ccctggttgg cggcgagggc 4260
   caaaacacgg tcatcggcgg cgccggcgac gacgtattcc tgcaggacct gggggtatgg 4320
   agcaaccagc tcgatggcgg cgcgggcgtc gataccgtga agtacaacgt gcaccagcct 4380
   tccgaggagc gcctcgaacg catgggcgac acgggcatcc atgccgatct tcaaaagggc 4440
   acggtcgaga agtggccggc cctgaacctg ttcagcgtcg accatgtcaa gaatatcgag 4500
   aatctgcacg gctcccgcct aaacgaccgc atcgccggcg acgaccagga caacgagctc 4560
   tggggccacg atggcaacga cacgatacgc ggccggggcg gcgacgacat cctgcgcggc 4620
   ggcctgggcc tggacacgct gtatggcgag gacggcaacg acatcttcct gcaggacgac 4680
   gagaccgtca gcgatgacat cgacggcggc gcggggctgg acaccgtcga ctactccgcc 4740
   atgatccatc caggcaggat cgttgcgccg catgaatacg gcttcgggat cgaggcggac 4800
   ctgtccaggg aatgggtgcg caaggcgtcc gcgctgggcg tggactatta cgataatgtc 4860
   cgcaatgtcg aaaacgtcat cggtacgagc atgaaggatg tgctcatcgg cgacgcgcaa 4920
   gccaataccc tgatgggcca gggcggcgac gataccgtgc gcggcggcga cggcgatgat 4980
   ctgctgttcg gcggcgacgg caacgacatg ctgtatggcg acgccggcaa cgacaccctc 5040
   tacggggggc tgggcgacga tacccttgaa ggcggcgcgg gcaacgattg gttcggccag 5100
   acgcaggcgc gcgagcatga cgtgctgcgc ggcggagatg gggtggatac cgtcgattac 5160
   agccagaccg gcgcgcatgc cggcattgcc gcgggtcgca tcgggctggg catcctggct 5220
   gacctgggcg ccggccgcgt cgacaagctg ggcgaggccg gcagcagcgc ctacgatacg 5280
   gtttccggta tcgagaacgt ggtgggcacg gaactggccg accgcatcac gggcgatgcg 5340
   caggccaacg tgctgcgcgg cgcgggtggc gccgacgtgc ttgcgggcgg cgagggcgac 5400
   gatgtgctgc tgggcggcga cggcgacgac cagctgtcgg gcgacgccgg acgcgatcgc 5460
   ttgtacggcg aagccggtga cgactggttc ttccaggatg ccgccaatgc cggcaatctg 5520
   ctcgacggcg gcgacggccg cgataccgtg gatttcagcg gcccgggccg gggcctcgac 5580
   gccggcgcaa agggcgtatt cctgagcttg ggcaaggggt tcgccagcct gatggacgaa 5640
   cccgaaacca gcaacgtgtt gcgcaatatc gagaacgccg tgggcagcgc gcgtgatgac 5700
   gtgctgatcg gcgacgcagg cgccaacgtc ctcaatggcc tggcgggcaa cgacgtgctg 5760
   tccggcggcg ctggcgacga tgtgctgctg ggcgacgagg gctcggacct gctcagcggc 5820
   gatgcgggca acgacgatct gttcggcggg cagggcgatg atacttatct gttcggggtc 5880
   gggtacgggc acgacacgat ctacgaatcg ggcggcggcc atgacaccat ccgcatcaac 5940
   gcgggggcgg accagctgtg gttcgcgcgc cagggcaacg acctggagat ccgcattctc 6000
   ggcaccgacg atgcacttac cgtgcacgac tggtatcgcg acgccgatca ccgggtggaa 6060
   atcatccatg ccgccaacca ggcggtagac caggcaggca tcgaaaagct ggtcgaggca 6120
   atggcgcagt atccggaccc cggcgcggcg gcggctgccc cgccggcggc gcgcgtgccg 6180
   gacacgctga tgcagtccct ggctgtcaac tggcgctgaa gcgccgtgaa tcacggcccg 6240
   cctgcctcgc gcggcggcgc cgtctctttg cgttcttctc cgaggtattt cccatcatga 6300
   attcactggc cgtcgtttta caacgtcgtg actgggaaaa ccctggcgtt acccaactta 6360
   atcgccttgc agcacatccc cctttcgcca gctggcgtaa tagcgaagag gcccgcaccg 6420
   atcgcccttc ccaacagttg cgcagcctga atggcgaatg ggaaattgta aacgttaata 6480
   ttttgttaat attttgttaa aattcgcgtt aaatttttgt taaatcagct cattttttaa 6540
   ccaataggcc gaaatcggca aaatccctta taaatcaaaa gaatagaccg agatagggtt 6600
   gagtgttgtt ccagtttgga acaagagtcc actattaaag aacgtggact ccaacgtcaa 6660
   agggcgaaaa accgtctatc agggcgatgg cccactacgt gaaccatcac cctaatcaag 6720
   ttttttgggg tcgaggtgcc gtaaagcact aaatcggaac cctaaaggga tgccccgatt 6780
   tagagcttga cggggaaagc cggcgaacgt ggcgagaaag gaagggaaga aagcgaaagg 6840
   agcgggcgct agggcgctgg caagtgtagc ggtcacgctg cgcgtaacca ccacacccgc 6900
   cgcgcttaat gcgccgctac agggcgcgtc aggtggcact tttcggggaa atgtgcgcgg 6960
   aacccctatt tgtttatttt tctaaataca ttcaaatatg tatccgctca tgagacaata 7020
   accctgataa atgcttcaat aatattgaaa aaggaagagt atgagtattc aacatttccg 7080
   tgtcgccctt attccctttt ttgcggcatt ttgccttcct gtttttgctc acccagaaac 7140
   gctggtgaaa gtaaaagatg ctgaagatca gttgggtgca cgagtgggtt acatcgaact 7200
   ggatctcaac agcggtaaga tccttgagag ttttcgcccc gaagaacgtt ttccaatgat 7260
   gagcactttt aaagttctgc tatgtggcgc ggtattatcc cgtattgacg ccgggcaaga 7320
   gcaactcggt cgccgcatac actattctca gaatgacttg gttgagtact caccagtcac 7380
   agaaaagcat cttacggatg gcatgacagt aagagaatta tgcagtgctg ccataaccat 7440
   gagtgataac actgcggcca acttacttct gacaacgatc ggaggaccga aggagctaac 7500
   cgcttttttg cacaacatgg gggatcatgt aactcgcctt gatcgttggg aaccggagct 7560
   gaatgaagcc ataccaaacg acgagcgtga caccacgatg cctgtagcaa tggcaacaac 7620
   gttgcgcaaa ctattaactg gcgaactact tactctagct tcccggcaac aattaataga 7680
   ctggatggag gcggataaag ttgcaggacc acttctgcgc tcggcccttc cggctggctg 7740
   gtttattgct gataaatctg gagccggtga gcgtgggtct cgcggtatca ttgcagcact 7800
   ggggccagat ggtaagccct cccgtatcgt agttatctac acgacgggga gtcaggcaac   7860
   tatggatgaa cgaaatagac agatcgctga gataggtgcc tcactgatta agcattggta   7920
   actgtcagac caagtttact catatatact ttagattgat ttaaaacttc atttttaatt   7980
   taaaaggatc taggtgaaga tcctttttga taatctcatg accaaaatcc cttaacgtga   8040
   gttttcgttc cactgagcgt cagaccccgt agaaaagatc aaaggatctt cttgagatcc   8100
   tttttttctg cgcgtaatct gctgcttgca aacaaaaaaa ccaccgctac cagcggtggt   8160
   ttgtttgccg gatcaagagc taccaactct ttttccgaag gtaactggct tcagcagagc   8220
   gcagatacca aatactgtcc ttctagtgta gccgtagtta ggccaccact tcaagaactc   8280
   tgtagcaccg cctacatacc tcgctctgct aatcctgtta ccagtggctg ctgccagtgg   8340
   cgataagtcg tgtcttaccg ggttggactc aagacgatag ttaccggata aggcgcagcg   8400
   gtcgggctga acggggggtt cgtgcacaca gcccagcttg gagcgaacga cctacaccga   8460
   actgagatac ctacagcgtg agctatgaga aagcgccacg cttcccgaag ggagaaaggc   8520
   ggacaggtat ccggtaagcg gcagggtcgg aacaggagag cgcacgaggg agcttccagg   8580
   gggaaacgcc tggtatcttt atagtcctgt cgggtttcgc cacctctgac ttgagcgtcg   8640
   atttttgtga tgctcgtcag gggggcggag cctatggaaa aacgccagca acgcggcctt   8700
   tttacggttc ctggcctttt gctggccttt tgctcacatg ttctttcctg cgttatcccc   8760
   tgattctgtg gataaccgta ttaccgcctt tgagtgagct gataccgctc gccgcagccg   8820
   aacgaccgag cgcagcgagt cagtgagcga ggaag                               8855
<210> 7
   <211> 1706
   <212> PRT
   <213> Bordetella parapertussis
<400> 7
   Met Gln Gln Ser His Gln Ala Gly Tyr Ala Asn Ala Ala Asp Arg Glu
   1               5                   10                  15
   Ser Gly Ile Pro Ala Ala Val Leu Asp Gly Ile Lys Ala Val Ala Lys
               20                  25                  30
   Glu Lys Asn Ala Thr Leu Met Phe Arg Leu Val Asn Pro His Ser Thr
           35                  40                  45
   Ser Leu Ile Ala Glu Gly Val Ala Thr Lys Gly Leu Gly Val His Ala
       50                  55                  60
   Lys Ser Ser Asp Trp Gly Leu Gln Ala Gly Tyr Ile Pro Val Asn Pro
   65                  70                  75                  80
   Asn Leu Ser Lys Leu Phe Gly Arg Ala Pro Glu Val Ile Ala Arg Ala
                   85                  90                  95
   Asp Asn Asp Val Asn Ser Ser Leu Ala His Gly His Thr Ala Val Asp
               100                 105                 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
                   165                 170                 175
   Ile Gly Asn Ala Ala Gly Ile Pro Leu Thr Ala Asp Ile Asp Met Phe
               180                 185                 190
   Ala Ile Met Pro His Leu Ser Asn Phe Arg Asp Ser Ala Arg Ser Ser
           195                 200                 205
   Val Thr Ser Gly Asp Ser Val Thr Asp Tyr Leu Ala Arg Thr Arg Arg
       210                 215                 220
   Ala Ala Ser Glu Ala Thr Gly Gly Leu Asp Arg Glu Arg Ile Asp Leu
   225                 230                 235                 240
   Leu Trp Lys Ile Ala Arg Ala Gly Ala Arg Ser Ala Val Gly Thr Glu
                   245                 250                 255
   Ala Arg Arg Gln Phe Arg Tyr Asp Gly Asp Met Asn Ile Gly Val Ile
               260                 265                 270
   Thr Asp Phe Glu Leu Glu Val Arg Asn Ala Leu Asn Arg Arg Ala His
           275                 280                 285
   Ala Val Gly Ala Gln Asp Val Val Gln His Gly Thr Glu Gln Asn Asn
       290                 295                 300
   Pro Phe Pro Glu Ala Asp Glu Lys Ile Phe Val Val Ser Ala Thr Gly
   305                 310                 315                 320
   Glu Ser Gln Met Leu Thr Arg Gly Gln Leu Lys Glu Tyr Ile Gly Gln
                   325                 330                 335
   Gln Arg Gly Glu Gly Tyr Val Phe Tyr Glu Asn Arg Ala Tyr Gly Val
               340                 345                 350
   Ala Gly Lys Ser Leu Phe Asp Asp Gly Leu Gly Ala Ala Pro Gly Val
           355                 360                 365
   Pro Gly Gly Arg Ser Lys Ser Ser Pro Asp Val Leu Glu Thr Val Pro
       370                 375                 380
   Ala Ser Pro Gly Leu Arg Arg Pro Ser Leu Gly Ala Val Glu Arg Gln
   385                 390                 395                 400
   Asp Ser Gly Tyr Asp Ser Leu Asp Gly Val Gly Ser Arg Ser Phe Ser
                   405                 410                 415
   Leu Gly Glu Val Ser Asp Met Ala Ala Val Glu Ala Ala Glu Leu Glu
               420                 425                 430
   Met Thr Arg Gln Val Leu His Ala Gly Ala Arg Gln Asp Asp Ala Glu
           435                 440                 445
   Pro Gly Val Ser Gly Ala Ser Ala His Trp Gly Gln Arg Ala Leu Gln
       450                 455                 460
   Gly Ala Gln Ala Val Ala Ala Ala Gln Arg Leu Val His Ala Ile Ala
   465                 470                 475                 480
   Leu Met Thr Gln Phe Gly Arg Ala Gly Ser Thr Asn Thr Pro Gln Glu
                   485                 490                 495
   Ala Ala Ser Leu Ser Ala Ala Val Phe Gly Leu Gly Glu Ala Ser Ser
               500                 505                 510
   Ala Val Ala Glu Thr Val Ser Gly Phe Phe Arg Gly Ser Ser Arg Trp
           515                 520                 525
   Ala Gly Gly Phe Gly Val Ala Gly Gly Ala Met Ala Leu Gly Gly Gly
       530                 535                 540
   Ile Ala Ala Ala Val Gly Ala Gly Met Ser Leu Thr Asp Asp Ala Pro
   545                 550                 555                 560
   Ala Gly Gln Lys Ala Ala Val Gly Ala Glu Ile Ala Leu Gln Leu Thr
                   565                 570                 575
   Gly Gly Thr Val Glu Leu Ala Ser Ser Ile Ala Leu Ala Leu Ala Ala
               580                 585                 590
   Ala Arg Gly Val Thr Ser Gly Leu Gln Val Ala Gly Ala Ser Ala Gly
           595                 600                 605
   Ala Ala Ala Gly Ala Leu Ala Ala Ala Leu Ser Pro Met Glu Ile Tyr
       610                 615                 620
   Gly Leu Val Gln Gln Ser His Tyr Ala Asp Gln Leu Asp Lys Leu Ala
   625                 630                 635                 640
   Gln Glu Ser Ser Ala Tyr Gly Tyr Glu Gly Asp Ala Leu Leu Ala Gln
                   645                 650                 655
   Leu Tyr Arg Asp Lys Thr Ala Ala Glu Gly Ala Val Ala Gly Val Ser
               660                 665                 670
   Ala Val Leu Ser Thr Val Gly Ala Ala Val Ser Ile Ala Ala Ala Ala
           675                 680                 685
   Ser Val Val Gly Ala Pro Val Ala Val Val Thr Ser Leu Leu Thr Gly
       690                 695                 700
   Ala Leu Asn Gly Ile Leu Arg Gly Val Gln Gln Pro Ile Ile Glu Lys
   705                 710                 715                 720
   Leu Ala Asn Asp Tyr Ala Arg Lys Ile Asp Glu Leu Gly Gly Pro Gln
                   725                 730                 735
   Ala Tyr Phe Glu Lys Asn Leu Gln Ala Arg His Glu Gln Leu Ala Asn
               740                 745                 750
   Ser Asp Gly Leu Arg Lys Met Leu Ala Asp Leu Gln Ala Gly Trp Asn
           755                 760                 765
   Ala Ser Ser Val Ile Gly Val Gln Thr Thr Glu Ile Ser Lys Ser Ala
       770                 775                 780
   Leu Glu Leu Ala Ala Ile Thr Gly Asn Ala Asp Asn Leu Lys Ser Ala
   785                 790                 795                 800
   Asp Val Phe Val Asp Arg Phe Ile Gln Gly Glu Arg Val Ala Gly Gln
                   805                 810                 815
   Pro Val Val Leu Asp Val Ala Ala Gly Gly Ile Asp Ile Ala Ser Arg
               820                 825                 830
   Lys Gly Glu Arg Pro Ala Leu Thr Phe Ile Thr Pro Leu Ala Ala Pro
           835                 840                 845
   Gly Glu Glu Gln Arg Arg Arg Thr Lys Thr Gly Lys Ser Glu Phe Thr
       850                 855                 860
   Thr Phe Val Glu Ile Val Gly Lys Gln Asp Arg Trp Arg Ile Arg Asp
   865                 870                 875                 880
   Gly Ala Ala Asp Thr Thr Ile Asp Leu Ala Lys Val Val Ser Gln Leu
                   885                 890                 895
   Val Asp Ala Asn Gly Val Leu Lys His Ser Ile Lys Leu Glu Val Ile
               900                 905                 910
   Gly Gly Asp Gly Asp Asp Val Val Leu Ala Asn Ala Ser Arg Ile His
           915                 920                 925
   Tyr Asp Gly Gly Ala Gly Thr Asn Thr Val Ser Tyr Ala Ala Leu Gly
       930                 935                 940
   Arg Gln Asp Ser Ile Thr Val Ser Ala Asp Gly Glu Arg Phe Asn Val
   945                 950                 955                 960
   Arg Lys Gln Leu Asn Asn Ala Asn Val Tyr Arg Glu Gly Val Ala Thr
                   965                 970                 975
   Gln Lys Thr Ala Tyr Gly Lys Arg Thr Glu Asn Val Gln Tyr Arg His
               980                 985                 990
   Val Glu Leu Ala Arg Val Gly Gln  Leu Val Glu Val Asp  Thr Leu Glu
           995                 1000                 1005
   His Val  Gln His Ile Ile Gly  Gly Ala Gly Asn Asp  Ser Ile Thr
       1010                 1015                 1020
   Gly Asn  Ala His Asp Asn Phe  Leu Ala Gly Gly Ala  Gly Asp Asp
       1025                 1030                 1035
   Arg Leu  Asp Gly Gly Ala Gly  Asn Asp Thr Leu Val  Gly Gly Glu
       1040                 1045                 1050
   Gly His  Asn Thr Val Val Gly  Gly Ala Gly Asp Asp  Val Phe Leu
       1055                 1060                 1065
   Gln Asp  Leu Gly Val Trp Ser  Asn Gln Leu Asp Gly  Gly Ala Gly
       1070                 1075                 1080
   Val Asp Thr Val Lys Tyr Asn Val His Gln Pro Ser Glu Glu Arg
   1085 1090 1095
   Leu Glu Arg Met Gly Asp Thr Gly Ile His Ala Asp Leu Gln Lys
   1100 1105 1110
   Gly Thr Val Glu Lys Trp Pro Ala Leu Asn Leu Phe Ser Val Asp
   1115 1120 1125
   His Val Lys Asn Ile Glu Asn Leu His Gly Ser Ser Leu Asn Asp
   1130 1135 1140
   Ser Ile Ala Gly Asp Asp Arg Asp Asn Glu Leu Trp Gly Asp Asp
   1145 1150 1155
   Gly Asn Asp Thr Ile His Gly Arg Gly Gly Asp Asp Ile Leu Arg
   1160 1165 1170
   Gly Gly Leu Gly Leu Asp Thr Leu Tyr Gly Glu Asp Gly Asn Asp
   1175 1180 1185
   Ile Phe Leu Gln Asp Asp Glu Thr Val Ser Asp Asp Ile Asp Gly
   1190 1195 1200
   Gly Ala Gly Leu Asp Thr Val Asp Tyr Ser Ala Met Ile His Ala
   1205 1210 1215
   Gly Lys Ile Val Ala Pro His Glu Tyr Gly Phe Gly Ile Glu Ala
   1220 1225 1230
   Asp Leu Ser Glu Gly Trp Val Arg Lys Ala Ala Arg Arg Gly Met
   1235 1240 1245
   Gly Tyr Tyr Asp Ser Val Arg Ser Val Glu Asn Val Ile Gly Thr
   1250 1255 1260
   Ser Met Lys Asp Val Leu Ile Gly Asp Ala Gln Ala Asn Thr Leu
   1265 1270 1275
   Met Gly Gln Gly Gly Asp Asp Thr Val Arg Gly Gly Asp Gly Asp
   1280 1285 1290
   Asp Leu Leu Phe Gly Gly Asp Gly Asn Asp Met Leu Tyr Gly Asp
   1295 1300 1305
   Ala Gly Asn Asp Thr Leu Tyr Gly Gly Leu Gly Asp Asp Thr Leu
   1310 1315 1320
   Glu Gly Gly Ala Gly Asn Asp Trp Phe Gly Gln Thr Pro Ala Arg
   1325 1330 1335
   Glu His Asp Val Leu Arg Gly Gly Ala Gly Val Asp Thr Val Asp
   1340 1345 1350
   Tyr Ser Gln Ala Gly Ala His Ala Gly Val Ala Thr Gly Arg Ile
   1355 1360 1365
   Gly Leu Gly Ile Leu Ala Asp Leu Gly Ala Gly Arg Val Asp Lys
   1370 1375 1380
   Leu Gly Glu Ala Gly Ser Ser Ala Tyr Asp Thr Val Ser Gly Ile
   1385 1390 1395
   Glu Asn Val Val Gly Thr Glu Leu Ala Asp Arg Ile Thr Gly Asp
   1400 1405 1410
   Ala Gln Ala Asn Val Leu Arg Gly Ala Gly Gly Ala Asp Val Leu
   1415 1420 1425
   Ala Gly Gly Glu Gly Asp Asp Val Leu Leu Gly Gly Glu Gly Asp
   1430 1435 1440
   Asp Gln Leu Ser Gly Asp Ala Gly Arg Asp Arg Leu Tyr Gly Glu
   1445 1450 1455
   Ala Gly Asp Asp Trp Phe Phe Gln Asp Ala Ala Asn Ala Gly Asn
   1460 1465 1470
   Leu Leu Asp Gly Gly Asp Gly Asn Asp Thr Val Asp Phe Ser Gly
   1475 1480 1485
   Pro Gly Arg Gly Leu Asp Ala Gly Ala Lys Gly Val Phe Leu Ser
   1490 1495 1500
   Leu Gly Lys Gly Phe Ala Ser Leu Met Asp Glu Pro Glu Thr Ser
   1505 1510 1515
   Asn Val Leu Arg His Ile Glu Asn Ala Val Gly Ser Val Arg Asp
   1520 1525 1530
   Asp Val Leu Ile Gly Asp Ala Gly Ala Asn Val Leu Asn Gly Leu
   1535 1540 1545
   Ala Gly Asn Asp Val Leu Ser Gly Gly Ala Gly Asp Asp Val Leu
   1550 1555 1560
   Leu Gly Asp Glu Gly Ser Asp Leu Leu Ser Gly Asp Ala Gly Asn
   1565 1570 1575
   Asp Asp Leu Phe Gly Gly Gln Gly Asp Asp Thr Tyr Leu Phe Gly
   1580 1585 1590
   Ala Gly Tyr Gly His Asp Thr Ile Tyr Glu Ser Gly Gly Gly His
   1595 1600 1605
   Asp Thr Ile Arg Ile Asn Ala Gly Ala Asp Gln Leu Trp Phe Ala
   1610 1615 1620
   Arg Gln Gly Asn Asp Leu Glu Ile Arg Ile Leu Gly Thr Asp Asp
   1625 1630 1635
   Ala Leu Thr Val His Asp Trp Tyr Arg Asp Ala Asp His Arg Val
   1640 1645 1650
   Glu Ala Ile His Ala Ala Asn Gln Ala Ile Asp Pro Ala Gly Ile
   1655 1660 1665
   Glu Lys Leu Val Glu Ala Met Ala Gln Tyr Pro Asp Pro Gly Ala
   1670 1675 1680
   Ala Ala Ala Ala Pro Pro Ala Ala Arg Val Pro Asp Thr Leu Met
   1685 1690 1695
   Gln Ser Leu Ala Val Asn Trp Arg
   1700 1705
<210> 8
   <211> 1706
   <212> PRT
   <213> Bordetella hinzii
<400> 8
   Met Gln Gln Ser His Gln Ala Gly Tyr Ala Asn Ala Ala Asp Arg Glu
   1 5 10 15
   Ser Gly Ile Pro Ala Ala Val Leu Asp Gly Ile Lys Ala Val Ala Lys
   20 25 30
   Glu Lys Asn Ala Thr Leu Met Phe Arg Leu Val Asn Pro His Ser Thr
   35 40 45
   Ser Leu Ile Ala Glu Gly Val Ala Thr Lys Gly Leu Gly Val His Ala
       50                  55                  60
   Lys Ser Ser Asp Trp Gly Leu Gln Ala Gly Tyr Ile Pro Val Asn Pro
   65                  70                  75                  80
   Asn Leu Ser Lys Leu Phe Gly Arg Ala Pro Glu Val Ile Ala Arg Ala
                   85                  90                  95
   Asp Asn Asp Val Asn Ser Ser Leu Ala His Gly His Thr Ala Val Asp
               100                 105                 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
                   165                 170                 175
   Ile Gly Asn Ala Ala Gly Ile Pro Leu Thr Ala Asp Ile Asp Met Phe
               180                 185                 190
   Ala Ile Met Pro His Leu Ser Asn Phe Arg Asp Ser Ala Arg Ser Ser
           195                 200                 205
   Val Thr Ser Gly Asp Ser Val Thr Asp Tyr Leu Ala Arg Thr Arg Arg
       210                 215                 220
   Ala Ala Ser Glu Ala Thr Gly Gly Leu Asp Arg Glu Arg Ile Asp Leu
   225                 230                 235                 240
   Leu Trp Lys Ile Ala Arg Ala Gly Ala Arg Ser Ala Val Gly Thr Glu
                   245                 250                 255
   Ala Arg Arg Gln Phe Arg Tyr Asp Gly Asp Met Asn Ile Gly Val Ile
               260                 265                 270
   Thr Asp Phe Glu Leu Glu Val Arg Asn Ala Leu Asn Arg Arg Ala His
           275                 280                 285
   Ala Val Gly Ala Gln Asp Val Val Gln His Gly Thr Glu Gln Asn Asn
       290                 295                 300
   Pro Phe Pro Glu Ala Asp Glu Lys Ile Phe Val Val Ser Ala Thr Gly
   305                 310                 315                 320
   Glu Ser Gln Met Leu Thr Arg Gly Gln Leu Lys Glu Tyr Ile Gly Gln
                   325                 330                 335
   Gln Arg Gly Glu Gly Tyr Val Phe Tyr Glu Asn Arg Ala Tyr Gly Val
               340                 345                 350
   Ala Gly Lys Ser Leu Phe Asp Asp Gly Leu Gly Ala Ala Pro Gly Val
           355                 360                 365
   Pro Ser Gly Arg Ser Lys Phe Ser Pro Asp Val Leu Glu Thr Val Pro
       370                 375                 380
   Ala Ser Pro Gly Leu Arg Arg Pro Ser Leu Gly Ala Val Glu Arg Gln
   385                 390                 395                 400
   Asp Ser Gly Tyr Asp Ser Leu Asp Gly Val Gly Ser Arg Ser Phe Ser
                   405                 410                 415
   Leu Gly Glu Val Ser Asp Met Ala Ala Val Glu Ala Ala Glu Leu Glu
               420                 425                 430
   Met Thr Arg Gln Val Leu His Ala Gly Ala Arg Gln Asp Asp Ala Glu
           435                 440                 445
   Pro Gly Val Ser Gly Ala Ser Ala His Trp Gly Gln Arg Ala Leu Gln
       450                 455                 460
   Gly Ala Gln Ala Val Ala Ala Ala Gln Arg Leu Val His Ala Ile Ala
   465                 470                 475                 480
   Leu Met Thr Gln Phe Gly Arg Ala Gly Ser Thr Asn Thr Pro Gln Glu
                   485                 490                 495
   Ala Ala Ser Leu Ser Ala Ala Val Phe Gly Leu Gly Glu Ala Ser Ser
               500                 505                 510
   Ala Val Ala Glu Thr Val Ser Gly Phe Phe Arg Gly Ser Ser Arg Trp
           515                 520                 525
   Ala Gly Gly Phe Gly Val Ala Gly Gly Ala Met Ala Leu Gly Gly Gly
       530                 535                 540
   Ile Ala Ala Ala Val Gly Ala Gly Met Ser Leu Thr Asp Asp Ala Pro
   545                 550                 555                 560
   Ala Gly Gln Lys Ala Ala Ala Gly Ala Glu Ile Ala Leu Gln Leu Thr
                   565                 570                 575
   Gly Gly Thr Val Glu Leu Ala Ser Ser Ile Ala Leu Ala Leu Ala Ala
               580                 585                 590
   Ala Arg Gly Val Thr Ser Gly Leu Gln Val Ala Gly Ala Ser Ala Gly
           595                 600                 605
   Ala Ala Ala Gly Ala Leu Ala Ala Ala Leu Ser Pro Met Glu Ile Tyr
       610                 615                 620
   Gly Leu Val Gln Gln Ser His Tyr Ala Asp Gln Leu Asp Lys Leu Ala
   625                 630                 635                 640
   Gln Glu Ser Ser Ala Tyr Gly Tyr Glu Gly Asp Ala Leu Leu Ala Gln
                   645                 650                 655
   Leu Tyr Arg Asp Lys Thr Ala Ala Glu Gly Ala Val Ala Gly Val Ser
               660                 665                 670
   Ala Val Leu Ser Thr Val Gly Ala Ala Val Ser Ile Ala Ala Ala Ala
           675                 680                 685
   Ser Val Val Gly Ala Pro Val Ala Val Val Thr Ser Leu Leu Thr Gly
       690                 695                 700
   Ala Leu Asn Gly Ile Leu Arg Gly Val Gln Gln Pro Ile Ile Glu Lys
   705                 710                 715                 720
   Leu Ala Asn Asp Tyr Ala Arg Lys Ile Asp Glu Leu Gly Gly Pro Gln
                   725                 730                 735
   Ala Tyr Phe Glu Lys Asn Leu Gln Ala Arg His Glu Gln Leu Ala Asn
               740                 745                 750
   Ser Asp Gly Leu Arg Lys Met Leu Ala Asp Leu Gln Ala Gly Trp Asn
           755                 760                 765
   Ala Ser Ser Val Ile Gly Val Gln Thr Thr Glu Ile Ser Lys Ser Ala
       770                 775                 780
   Leu Glu Leu Ala Ala Ile Thr Gly Asn Ala Asp Asn Leu Lys Ser Val
   785                 790                 795                 800
   Asp Val Phe Val Asp Arg Phe Val Gln Gly Glu Arg Val Ala Gly Gln
   805 810 815
   Pro Val Val Leu Asp Val Ala Ala Gly Gly Ile Asp Ile Ala Ser Arg
   820 825 830
   Lys Gly Glu Arg Pro Ala Leu Thr Phe Ile Thr Pro Leu Ala Ala Pro
   835 840 845
   Gly Glu Glu Gln Arg Arg Arg Thr Lys Thr Gly Lys Ser Glu Phe Thr
   850 855 860
   Thr Phe Val Glu Ile Val Gly Lys Gln Asp Arg Trp Arg Ile Arg Asp
   865 870 875 880
   Gly Ala Ala Asp Thr Thr Ile Asp Leu Ala Lys Val Val Ser Gln Leu
   885 890 895
   Val Asp Ala Asn Gly Val Leu Lys His Ser Ile Lys Leu Asp Val Ile
   900 905 910
   Gly Gly Asp Gly Asp Asp Val Val Leu Ala Asn Ala Ser Arg Ile His
   915 920 925
   Tyr Asp Gly Gly Ala Gly Thr Asn Thr Val Ser Tyr Ala Ala Leu Gly
   930 935 940
   Arg Gln Asp Ser Ile Thr Val Ser Ala Asp Gly Glu Arg Phe Asn Val
   945 950 955 960
   Arg Lys Gln Leu Asn Asn Ala Asn Val Tyr Arg Glu Gly Val Ala Thr
   965 970 975
   Gln Thr Thr Ala Tyr Gly Lys Arg Thr Glu Asn Val Gln Tyr Arg His
   980 985 990
   Val Glu Leu Ala Arg Val Gly Gln Leu Val Glu Val Asp Thr Leu Glu
   995 1000 1005
   His Val Gln His Ile Ile Gly Gly Ala Gly Asn Asp Ser Ile Thr
   1010 1015 1020
   Gly Asn Ala His Asp Asn Phe Leu Ala Gly Gly Ser Gly Asp Asp
   1025 1030 1035
   Arg Leu Asp Gly Gly Ala Gly Asn Asp Thr Leu Val Gly Gly Glu
   1040 1045 1050
   Gly Gln Asn Thr Val Ile Gly Gly Ala Gly Asp Asp Val Phe Leu
   1055 1060 1065
   Gln Asp Leu Gly Val Trp Ser Asn Gln Leu Asp Gly Gly Ala Gly
   1070 1075 1080
   Val Asp Thr Val Lys Tyr Asn Val His Gln Pro Ser Glu Glu Arg
   1085 1090 1095
   Leu Glu Arg Met Gly Asp Thr Gly Ile His Ala Asp Leu Gln Lys
   1100 1105 1110
   Gly Thr Val Glu Lys Trp Pro Ala Leu Asn Leu Phe Ser Val Asp
   1115 1120 1125
   His Val Lys Asn Ile Glu Asn Leu His Gly Ser Arg Leu Asn Asp
   1130 1135 1140
   Arg Ile Ala Gly Asp Asp Gln Asp Asn Glu Leu Trp Gly His Asp
   1145 1150 1155
   Gly Asn Asp Thr Ile Arg Gly Arg Gly Gly Asp Asp Ile Leu Arg
   1160 1165 1170
   Gly Gly Leu Gly Leu Asp Thr Leu Tyr Gly Glu Asp Gly Asn Asp
   1175 1180 1185
   Ile Phe Leu Gln Asp Asp Glu Thr Val Ser Asp Asp Ile Asp Gly
   1190 1195 1200
   Gly Ala Gly Leu Asp Thr Val Asp Tyr Ser Ala Met Ile His Pro
   1205 1210 1215
   Gly Arg Ile Val Ala Pro His Glu Tyr Gly Phe Gly Ile Glu Ala
   1220 1225 1230
   Asp Leu Ser Arg Glu Trp Val Arg Lys Ala Ser Ala Leu Gly Val
   1235 1240 1245
   Asp Tyr Tyr Asp Asn Val Arg Asn Val Glu Asn Val Ile Gly Thr
   1250 1255 1260
   Ser Met Lys Asp Val Leu Ile Gly Asp Ala Gln Ala Asn Thr Leu
   1265 1270 1275
   Met Gly Gln Gly Gly Asp Asp Thr Val Arg Gly Gly Asp Gly Asp
   1280 1285 1290
   Asp Leu Leu Phe Gly Gly Asp Gly Asn Asp Met Leu Tyr Gly Asp
   1295 1300 1305
   Ala Gly Asn Asp Thr Leu Tyr Gly Gly Leu Gly Asp Asp Thr Leu
   1310 1315 1320
   Glu Gly Gly Ala Gly Asn Asp Trp Phe Gly Gln Thr Gln Ala Arg
   1325 1330 1335
   Glu His Asp Val Leu Arg Gly Gly Asp Gly Val Asp Thr Val Asp
   1340 1345 1350
   Tyr Ser Gln Thr Gly Ala His Ala Gly Ile Ala Ala Gly Arg Ile
   1355 1360 1365
   Gly Leu Gly Ile Leu Ala Asp Leu Gly Ala Gly Arg Val Asp Lys
   1370 1375 1380
   Leu Gly Glu Ala Gly Ser Ser Ala Tyr Asp Thr Val Ser Gly Ile
   1385 1390 1395
   Glu Asn Val Val Gly Thr Glu Leu Ala Asp Arg Ile Thr Gly Asp
   1400 1405 1410
   Ala Gln Ala Asn Val Leu Arg Gly Ala Gly Gly Ala Asp Val Leu
   1415 1420 1425
   Ala Gly Gly Glu Gly Asp Asp Val Leu Leu Gly Gly Asp Gly Asp
   1430 1435 1440
   Asp Gln Leu Ser Gly Asp Ala Gly Arg Asp Arg Leu Tyr Gly Glu
   1445 1450 1455
   Ala Gly Asp Asp Trp Phe Phe Gln Asp Ala Ala Asn Ala Gly Asn
   1460 1465 1470
   Leu Leu Asp Gly Gly Asp Gly Arg Asp Thr Val Asp Phe Ser Gly
   1475 1480 1485
   Pro Gly Arg Gly Leu Asp Ala Gly Ala Lys Gly Val Phe Leu Ser
   1490 1495 1500
   Leu Gly Lys Gly Phe Ala Ser Leu Met Asp Glu Pro Glu Thr Ser
   1505 1510 1515
   Asn Val Leu Arg Asn Ile Glu Asn Ala Val Gly Ser Ala Arg Asp
   1520 1525 1530
   Asp Val  Leu Ile Gly Asp Ala  Gly Ala Asn Val Leu  Asn Gly Leu
       1535                 1540                 1545
   Ala Gly  Asn Asp Val Leu Ser  Gly Gly Ala Gly Asp  Asp Val Leu
       1550                 1555                 1560
   Leu Gly  Asp Glu Gly Ser Asp  Leu Leu Ser Gly Asp  Ala Gly Asn
       1565                 1570                 1575
   Asp Asp  Leu Phe Gly Gly Gln  Gly Asp Asp Thr Tyr  Leu Phe Gly
       1580                 1585                 1590
   Val Gly  Tyr Gly His Asp Thr  Ile Tyr Glu Ser Gly  Gly Gly His
       1595                 1600                 1605
   Asp Thr  Ile Arg Ile Asn Ala  Gly Ala Asp Gln Leu  Trp Phe Ala
       1610                 1615                 1620
   Arg Gln  Gly Asn Asp Leu Glu  Ile Arg Ile Leu Gly  Thr Asp Asp
       1625                 1630                 1635
   Ala Leu  Thr Val His Asp Trp  Tyr Arg Asp Ala Asp  His Arg Val
       1640                 1645                 1650
   Glu Ile  Ile His Ala Ala Asn  Gln Ala Val Asp Gln  Ala Gly Ile
       1655                 1660                 1665
   Glu Lys  Leu Val Glu Ala Met  Ala Gln Tyr Pro Asp  Pro Gly Ala
       1670                 1675                 1680
   Ala Ala  Ala Ala Pro Pro Ala  Ala Arg Val Pro Asp  Thr Leu Met
       1685                 1690                 1695
   Gln Ser  Leu Ala Val Asn Trp  Arg
       1700                 1705
<210> 9
   <211> 1705
   <212> PRT
   <213> Bordetella bronchiseptica
<400> 9
   Met Gln Gln Ser His Gln Ala Gly Tyr Ala Asn Ala Ala Asp Arg Glu
   1               5                   10                  15
   Ser Gly Ile Pro Ala Ala Val Leu Asp Gly Ile Lys Ala Val Ala Lys
               20                  25                  30
   Glu Lys Asn Ala Thr Leu Met Phe Arg Leu Val Asn Pro His Ser Thr
           35                  40                  45
   Ser Leu Ile Ala Glu Gly Val Ala Thr Lys Gly Leu Gly Val His Ala
       50                  55                  60
   Lys Ser Ser Asp Trp Gly Leu Gln Ala Gly Tyr Ile Pro Val Asn Pro
   65                  70                  75                  80
   Asn Leu Ser Lys Leu Phe Gly Arg Ala Pro Glu Val Ile Ala Arg Ala
                   85                  90                  95
   Asp Asn Asp Val Asn Ser Ser Leu Ala His Gly His Thr Ala Val Asp
               100                 105                 110
   Leu Thr Leu Ser Lys Glu Arg Leu Asp Tyr Leu Arg Gln Ala Gly Leu
           115                 120                 125
   Val Thr Gly Met Ala Asp Gly Val Val Ala Ser Asn His Ala Gly Tyr
       130                 135                 140
   Glu Gln Phe Glu Phe Arg Val Lys Glu Thr Ser Asp Gly Arg Tyr Ala
   145                 150                 155                 160
   Val Gln Tyr Arg Arg Lys Gly Gly Asp Asp Phe Glu Ala Val Lys Val
                   165                 170                 175
   Ile Gly Asn Ala Ala Gly Ile Pro Leu Thr Ala Asp Ile Asp Met Phe
               180                 185                 190
   Ala Ile Met Pro His Leu Ser Asn Phe Arg Asp Ser Ala Arg Ser Ser
           195                 200                 205
   Val Thr Ser Gly Asp Ser Val Thr Asp Tyr Leu Ala Arg Thr Arg Arg
       210                 215                 220
   Ala Ala Ser Glu Ala Thr Gly Gly Leu Asp Arg Glu Arg Ile Asp Leu
   225                 230                 235                 240
   Leu Trp Lys Ile Ala Arg Ala Gly Ala Arg Ser Ala Val Gly Thr Glu
                   245                 250                 255
   Ala Arg Arg Gln Phe Arg Tyr Asp Gly Asp Met Asn Ile Gly Val Ile
               260                 265                 270
   Thr Asp Phe Glu Leu Glu Val Arg Asn Ala Leu Asn Arg Arg Ala His
           275                 280                 285
   Ala Val Gly Arg Gln Asp Val Val Gln His Gly Thr Glu Gln Asn Asn
       290                 295                 300
   Pro Phe Pro Glu Ala Asp Glu Lys Ile Phe Val Val Ser Ala Thr Gly
   305                 310                 315                 320
   Glu Ser Gln Met Leu Thr Arg Gly Gln Leu Lys Glu Tyr Ile Gly Gln
                   325                 330                 335
   Gln Arg Gly Glu Gly Tyr Val Phe Tyr Glu Asn Arg Ala Tyr Gly Val
               340                 345                 350
   Ala Gly Lys Ser Leu Phe Asp Asp Gly Leu Gly Ala Ala Pro Gly Val
           355                 360                 365
   Pro Gly Arg Arg Ser Lys Ser Ser Pro Asp Val Leu Glu Thr Val Pro
       370                 375                 380
   Ala Ser Pro Gly Leu Arg Arg Pro Ser Leu Gly Ala Val Glu Arg Gln
   385                 390                 395                 400
   Asp Ser Gly Tyr Asp Ser Leu Asp Gly Val Gly Ser Arg Ser Phe Ser
                   405                 410                 415
   Leu Gly Glu Val Ser Asp Met Ala Ala Val Glu Ala Ala Glu Leu Glu
               420                 425                 430
   Met Thr Arg Gln Val Leu His Ala Gly Ala Arg Gln Asp Asp Ala Glu
           435                 440                 445
   Pro Gly Val Ser Gly Ala Ser Ala His Trp Gly Gln Arg Ala Leu Gln
       450                 455                 460
   Gly Ala Gln Ala Val Ala Ala Ala Gln Arg Leu Val His Ala Ile Ala
   465                 470                 475                 480
   Leu Met Thr Gln Phe Gly Arg Ala Gly Ser Thr Asn Thr Pro Gln Glu
                   485                 490                 495
   Ala Ala Ser Leu Ser Ala Ala Val Phe Gly Leu Gly Glu Ala Ser Ser
               500                 505                 510
   Ala Val Ala Glu Thr Val Ser Gly Phe Phe Arg Gly Ser Ser Arg Trp
           515                 520                 525
   Ala Gly Gly Phe Gly Val Ala Gly Gly Ala Met Ala Leu Gly Gly Gly
       530                 535                 540
   Ile Gly Ala Val Gly Ala Gly Met Ser Leu Thr Asp Asp Ala Pro Ala
   545                 550                 555                 560
   Gly Gln Lys Ala Ala Ala Gly Ala Glu Ile Ala Leu Gln Leu Thr Gly
                   565                 570                 575
   Gly Thr Val Glu Leu Ala Ser Ser Ile Ala Leu Ala Leu Ala Ala Ala
               580                 585                 590
   Arg Gly Val Thr Ser Gly Leu Gln Val Ala Gly Ala Ser Ala Gly Ala
           595                 600                 605
   Ala Ala Gly Ala Leu Ala Ala Ala Leu Ser Pro Met Glu Ile Tyr Gly
       610                 615                 620
   Leu Val Gln Gln Ser His Tyr Ala Asp Gln Leu Asp Lys Leu Ala Gln
   625                 630                 635                 640
   Glu Ser Ser Ala Tyr Gly Tyr Glu Gly Asp Ala Leu Leu Ala Gln Leu
                   645                 650                 655
   Tyr Arg Asp Lys Thr Ala Ala Glu Gly Ala Val Ala Gly Val Ser Ala
               660                 665                 670
   Val Leu Ser Thr Val Gly Ala Ala Val Ser Ile Ala Ala Ala Ala Ser
           675                 680                 685
   Val Val Gly Ala Pro Val Ala Val Val Thr Ser Leu Leu Thr Gly Ala
       690                 695                 700
   Leu Asn Gly Ile Leu Arg Gly Val Gln Gln Pro Ile Ile Glu Lys Leu
   705                 710                 715                 720
   Ala Asn Asp Tyr Ala Arg Lys Ile Asp Glu Leu Gly Gly Pro Gln Ala
                   725                 730                 735
   Tyr Phe Glu Lys Asn Leu Gln Ala Arg His Glu Gln Leu Ala Asn Ser
               740                 745                 750
   Asp Gly Leu Arg Lys Met Leu Ala Asp Leu Gln Ala Gly Trp Asn Ala
           755                 760                 765
   Ser Ser Val Ile Gly Val Gln Thr Thr Glu Ile Ser Lys Ser Ala Leu
       770                 775                 780
   Glu Leu Ala Ala Ile Thr Gly Asn Ala Asp Asn Leu Lys Ser Ala Asp
   785                 790                 795                 800
   Val Phe Val Asp Arg Phe Ile Gln Gly Glu Arg Val Ala Gly Gln Pro
                   805                 810                 815
   Val Val Leu Asp Val Ala Ala Gly Gly Ile Asp Ile Ala Ser Arg Lys
               820                 825                 830
   Gly Glu Arg Pro Ala Leu Thr Phe Ile Thr Pro Leu Ala Ala Pro Gly
           835                 840                 845
   Glu Glu Gln Arg Arg Arg Thr Lys Thr Gly Lys Ser Glu Phe Thr Thr
       850                 855                 860
   Phe Val Glu Ile Val Gly Lys Gln Asp Arg Trp Arg Ile Arg Asp Gly
   865                 870                 875                 880
   Ala Ala Asp Thr Thr Ile Asp Leu Ala Lys Val Val Ser Gln Leu Val
                   885                 890                 895
   Asp Ala Asn Gly Val Leu Lys His Ser Ile Lys Leu Glu Val Ile Gly
               900                 905                 910
   Gly Asp Gly Asp Asp Val Val Leu Ala Asn Ala Ser Arg Ile His Tyr
           915                 920                 925
   Asp Gly Gly Ala Gly Thr Asn Thr Val Ser Tyr Ala Ala Leu Gly Arg
       930                 935                 940
   Gln Asp Ser Ile Thr Val Ser Ala Asp Gly Glu Arg Phe Asn Val Arg
   945                 950                 955                 960
   Lys Gln Leu Asn Asn Ala Asn Val Tyr Arg Glu Gly Val Ala Thr Gln
                   965                 970                 975
   Lys Thr Ala Tyr Gly Lys Arg Thr Glu Asn Val Gln Tyr Arg His Val
               980                 985                 990
   Glu Leu Ala Arg Val Gly Gln Leu  Val Glu Val Asp Thr  Leu Glu His
           995                 1000                1005
   Val Gln  His Ile Ile Gly Gly  Ala Gly Asn Asp Ser  Ile Thr Gly
       1010                1015                1020
   Asn Ala  His Asp Asn Phe Leu  Ala Gly Gly Ala Gly  Asp Asp Arg
       1025                1030                1035
   Leu Asp  Gly Gly Ala Gly Asn  Asp Thr Leu Val Gly  Gly Glu Gly
       1040                 1045                 1050
   His Asn  Thr Val Val Gly Gly  Ala Gly Asp Asp Val  Phe Leu Gln
       1055                 1060                 1065
   Asp Leu  Gly Val Trp Ser Asn  Gln Leu Asp Gly Gly  Ala Gly Val
       1070                 1075                 1080
   Asp Thr  Val Lys Tyr Asn Val  His Gln Pro Ser Glu  Glu Arg Leu
       1085                 1090                 1095
   Glu Arg  Met Gly Asp Thr Gly  Ile His Ala Asp Leu  Gln Lys Gly
       1100                 1105                 1110
   Thr Val  Glu Lys Trp Pro Ala  Leu Asn Leu Phe Ser  Val Asp His
       1115                 1120                 1125
   Val Lys  Asn Ile Glu Asn Leu  His Gly Ser Ser Leu  Asn Asp Ser
       1130                 1135                 1140
   Ile Ala  Gly Asp Asp Arg Asp  Asn Glu Leu Trp Gly  Asp Asp Gly
       1145                 1150                 1155
   Asn Asp  Thr Ile His Gly Arg  Gly Gly Asp Asp Ile  Leu Arg Gly
       1160                 1165                 1170
   Gly Leu  Gly Leu Asp Thr Leu  Tyr Gly Glu Asp Gly  Asn Asp Ile
       1175                 1180                 1185
   Phe Leu  Gln Asp Asp Glu Thr  Val Ser Asp Asp Ile  Asp Gly Gly
       1190                 1195                 1200
   Ala Gly  Leu Asp Thr Val Asp  Tyr Ser Ala Met Ile  His Ala Gly
       1205                 1210                 1215
   Lys Ile  Val Ala Pro His Glu  Tyr Gly Phe Gly Ile  Glu Ala Asp
       1220                 1225                 1230
   Leu Ser  Glu Gly Trp Val Arg  Lys Ala Ala Arg Arg  Gly Met Asp
       1235                 1240                 1245
   Tyr Tyr  Asp Ser Val Arg Ser  Val Glu Asn Val Ile  Gly Thr Ser
       1250                 1255                 1260
   Met Lys  Asp Val Leu Ile Gly  Asp Ala Gln Ala Asn  Thr Leu Met
   1265 1270 1275
   Gly Gln Gly Gly Asp Asp Thr Val Arg Gly Gly Asp Gly Asp Asp
   1280 1285 1290
   Leu Leu Phe Gly Gly Asp Gly Asn Asp Met Leu Tyr Gly Asp Ala
   1295 1300 1305
   Gly Asn Asp Thr Leu Tyr Gly Gly Leu Gly Asp Asp Thr Leu Glu
   1310 1315 1320
   Gly Gly Ala Gly Asn Asp Trp Phe Gly Gln Thr Pro Ala Arg Glu
   1325 1330 1335
   His Asp Val Leu Arg Gly Gly Ala Gly Val Asp Thr Val Asp Tyr
   1340 1345 1350
   Ser Gln Ala Gly Ala His Ala Gly Val Ala Thr Gly Arg Ile Gly
   1355 1360 1365
   Leu Gly Ile Leu Ala Asp Leu Gly Ala Gly Arg Val Asp Lys Leu
   1370 1375 1380
   Gly Glu Ala Gly Ser Ser Ala Tyr Asp Thr Val Ser Gly Ile Glu
   1385 1390 1395
   Asn Val Val Gly Thr Glu Leu Ala Asp Arg Ile Thr Gly Asp Ala
   1400 1405 1410
   Gln Ala Asn Val Leu Arg Gly Ala Gly Gly Ala Asp Val Leu Ala
   1415 1420 1425
   Gly Gly Glu Gly Asp Asp Val Leu Leu Gly Gly Asp Gly Asp Asp
   1430 1435 1440
   Gln Leu Ser Gly Asp Ala Gly Arg Asp Arg Leu Tyr Gly Glu Ala
   1445 1450 1455
   Gly Asp Asp Trp Phe Phe Gln Asp Ala Ala Asn Ala Gly Asn Leu
   1460 1465 1470
   Leu Asp Gly Gly Asp Gly Asn Asp Thr Val Asp Phe Ser Gly Pro
   1475 1480 1485
   Gly Arg Gly Leu Asp Ala Gly Ala Lys Gly Val Phe Leu Ser Leu
   1490 1495 1500
   Gly Lys  Gly Phe Ala Ser Leu  Met Asp Glu Pro Glu  Thr Ser Asn
       1505                 1510                 1515
   Val Leu  Arg His Ile Glu Asn  Ala Val Gly Ser Val  Arg Asp Asp
       1520                 1525                 1530
   Val Leu  Ile Gly Asp Ala Gly  Ala Asn Val Leu Asn  Gly Leu Ala
       1535                 1540                 1545
   Gly Asn  Asp Val Leu Ser Gly  Gly Ala Gly Asp Asp  Val Leu Leu
       1550                 1555                 1560
   Gly Asp  Glu Gly Ser Asp Leu  Leu Ser Gly Asp Ala  Gly Asn Asp
       1565                 1570                 1575
   Asp Leu  Phe Gly Gly Gln Gly  Asp Asp Thr Tyr Leu  Phe Gly Ala
       1580                 1585                 1590
   Gly Tyr  Gly His Asp Thr Ile  Tyr Glu Ser Gly Gly  Gly His Asp
       1595                 1600                 1605
   Thr Ile  Arg Ile Asn Ala Gly  Ala Asp Gln Leu Trp  Phe Ala Arg
       1610                 1615                 1620
   Gln Gly  Asn Asp Leu Glu Ile  Arg Ile Leu Gly Thr  Asp Asp Ala
       1625                 1630                 1635
   Leu Thr  Val His Asp Trp Tyr  Arg Asp Ala Asp His  Arg Val Glu
       1640                 1645                 1650
   Ala Ile  His Ala Ala Asn Gln  Ala Ile Asp Pro Ala  Gly Ile Glu
       1655                 1660                 1665
   Lys Leu  Val Glu Ala Met Ala  Gln Tyr Pro Asp Pro  Gly Ala Ala
       1670                 1675                 1680
   Ala Ala  Ala Pro Pro Ala Ala  Arg Val Pro Asp Thr  Leu Met Gln
       1685                 1690                 1695
   Ser Leu  Ala Val Asn Trp Arg
       1700                 1705

## Description

The invention relates to polypeptides suitable for use in the delivery of one or more molecules into a cell.

In particular, the invention relates to polypeptides suitable for use in the delivery of one or more molecules which are able to elicit an immune response into a host, by targeting cells which express the CD11b/CD18 receptor (also referred to herein as "CD11 b expressing cells").

The invention is more particularly directed to polypeptides derived from an adenylate cyclase protein (CyaA), the latter being used either under the form of a toxin or of a detoxified protein or toxoid, which are mutant polypeptides. Said mutant polypeptides are capable of retaining the binding activity of native CyaA to a target cell expressing the CD11b/CD18 receptor and preferably of also retaining the translocating activity of native CyaA through its N-terminal domain into said cells and furthermore have a pore-forming activity which is reduced or suppressed as compared to that of the native CyaA toxin.

The invention relates in particular to the use of said polypeptides as proteinaceous vectors. Accordingly the mutant polypeptides are further combined with non-CyaA molecules, thereby giving rise to polypeptide derivatives, wherein said molecules have a preventive vaccinal and/or therapeutic interest when administered to a host.

The polypeptides according to the invention are suitable for use as proteinaceous vectors for the delivery of a molecule, in particular of a polypeptidic molecule having an amino acid sequence comprising one or more epitope(s), especially antigens, into a cell, in particular in CD11b expressing cells.

The invention thus also relates to a polypeptide derivative (a derivative of the mutant polypeptide of the invention) which comprises or consists of a mutant polypeptide according to the invention recombined to one or more molecules, in particular to one or more molecules suitable for eliciting an immune response, thus constituting a recombinant polypeptide or a fusion polypeptide. The invention also relates to polypeptide derivatives obtained by chemically grafting said molecule(s) to the mutant polypeptides.

According to an embodiment, the polypeptide derivatives according to the invention are suitable for use in prophylactic treatment and especially in vaccination and in therapy including in immunotherapy, in particular for eliciting an immune response in a subject.

The native CyaA used in the context of the present invention for the design of the polypeptides of the invention is the adenylate cyclase produced in *Bordetella Pertussis* and which has the following features and properties disclosed for the purpose of characterising said protein in the context of the invention.

The bi-functional RTX adenylate cyclase toxin-hemolysin (also designated herewith as the adenylate cyclase toxin (CyaA, ACT, or AC-Hly) is a key virulence factor of *Bordetella pertussis* which is the causative agent of whooping cough (1). Its 1706 residues-long polypeptide is a fusion of an N-terminal adenylate cyclase (AC) enzyme domain or part (∼400 residues) to a pore-forming RTX hemolysin (Repeat in ToXin cytolysin) of ∼1306 residues constituting the C-terminal part or domain (2). The latter harbors the sites of activation of proCyaA to CyaA by covalent post-translational palmitoylation of ε-amino groups of Lys⁸⁶⁰ and Lys⁹⁸³, as well as the numerous RTX repeats forming ∼40 calcium binding sites, the loading of which is required for cytotoxic activity of CyaA (3, 4). The CyaA protein is indeed synthesized as an inactive protoxin which is converted into an active toxin by post translational palmitoylation of two internal lysine residues (lysines 860 and 983). This post translational modification requires the expression with the *cyaA* gene, of an accessory gene i.e., cyaC which is located nearby cyaA on *B*. *pertussis* chromosome.

The toxin primarily targets host myeloid phagocytes expressing the α_{M}β₂ integrin receptor, known also as CD11b/CD18, CR3 or Mac-1 (5). Said toxin especially binds to the CD11b/CD18 receptor of cells expressing the same through a receptor binding site present in its C-terminal part. These cells are accordingly target cells for the native toxin and also for the polypeptides of the invention. CyaA inserts into cytoplasmic membrane of cells and translocates the AC enzyme domain into the cytosol of said target cells (6, 7). Inside cells, the AC is activated by calmodulin and catalyzes uncontrolled conversion of cellular ATP to cAMP, a key second messenger molecule provoking impairment of bactericidal functions of phagocytes (1). At high doses (>100 ng/ml), CyaA-catalyzed dissipation of ATP into cAMP becomes cytotoxic and promotes apoptosis or even rapid necrotic death and lysis of CD11b+ monocytes (8, 9).

Recently, the inventors showed that CyaA binds N-linked oligosaccharides of its CD11b/CD18 receptor (10). This suggests that low specificity interactions with glycans of ubiquitous cell surface proteins or glycolipids may account for the about two order of magnitude reduced but readily detectable capacity of CyaA to penetrate also cells lacking CD11b/CD18. Indeed, due to the extremely high specific catalytic activity of the AC domain, CyaA was found to substantially elevate cAMP also in mammalian and avian erythrocytes, lymphocytes, lymphoma, neuroblastoma, CHO, or tracheal epithelial cells (1, 11).

It has already been proposed in the prior art to provide detoxified toxin also called toxoid, wherein the adenylate cyclase activity is decreased, especially essentially suppressed. Such CyaA/AC⁻ toxoid may be used to achieve the preparation of the polypeptides of the invention.

Besides elevating cAMP, the toxin exhibits also a moderate hemolytic activity on mammalian and avian erythrocytes. This is due to the capacity to form small cation-selective pores of an estimated diameter of 0.6 to 0.8 nm, which permeabilize cellular membrane and eventually provoke colloid-osmotic cell lysis (12). Recently, the inventors and others have shown that the pore-forming activity of CyaA synergizes with its cell-invasive AC enzyme activity and contributes to the overall cytolytic potency of CyaA on CD11b⁺ cells (13, 14). Due to an intact pore-forming (hemolytic) capacity, in the absence of osmoprotectants such as serum, the enzymatically inactive CyaA/AC⁻ toxoid (15) still exhibits a full hemolytic activity on erythrocytes and a residual, about ten-fold reduced cytolytic activity on CD11b-expressing monocytes (8), which sets a limit to its use in therapy.

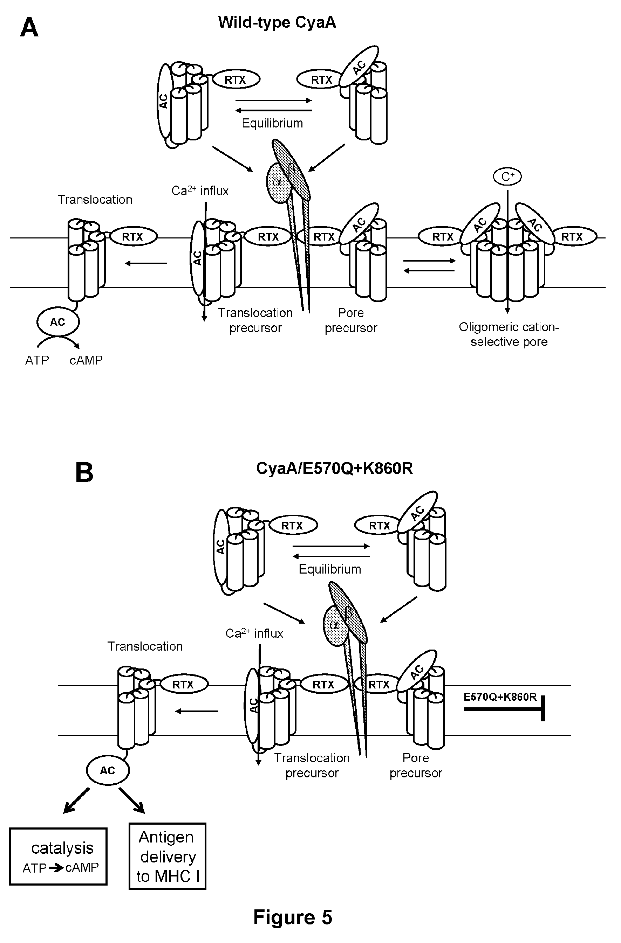

The hemolytic (pore-forming) and AC membrane translocation (cell-invasive) activities of CyaA were early on found to be dissociable by low calcium concentration, low temperature (16) and by the extent and nature of acylation of CyaA (4, 12, 17). Moreover, the two activities differ substantially in sensitivity to charge-reversing or neutral substitutions of glutamates at positions 509, 516, 570 and 581 within the hydrophobic domain (8, 13, 18). The cell-invasive and pore-forming activities of CyaA were thus proposed to be mutually independent and operating in parallel in target cell membrane. The model illustrated in figure 5A, suggests that two distinct CyaA conformers insert into target cell membrane in parallel, one being the translocation precursor, accounting for delivery of the AC domain across cellular membrane with concomitant influx of calcium ions into cells, the other being a pore precursor eventually forming oligomeric pores (13, 18, 19).

The inventors have now tested this model and refined it, showing that the pore-forming activity is not involved in translocation of the AC domain across target cell.

In the present invention, the inventors initially designed CyaA mutant polypeptides, based on the adenylate cyclase of *Bordetella pertussis,* either in the toxin or in the toxoid format, having a combination of substitutions within the pore-forming (E570Q) and acylation-bearing (K860R) domains and showed that this specific combination of substitutions selectively abolished the cell-permeabilizing activity of CyaA, thus eliminating the residual cytolytic activity of CyaA/AC- toxoids on CD11 b+ cells. At the same time, the E570Q+K860R construct retained a full capacity to translocate the AC domain into cytosol of cells to elevate cellular cAMP and its toxoid was fully capable to deliver epitopes containing molecules inserted within said construct to the cytosolic pathway of dendritic cells for MHC class I-restricted presentation and induction of specific cytotoxic T cell responses *in vivo.*

The CyaA/233OVA/E570Q+K860R mutant designed by the inventors, and in which an OVA antigenic peptide is inserted as described in the examples, is the first construct illustrative of the capacity of the CyaA mutant to provide an importantly reduced capacity to permeabilize cells while remaining fully capable of translocating the AC domain across cellular membrane.

The inventors have now designed particular constructs, illustrated especially as a CyaA/E570Q+K860R/AC⁻ toxoid and have shown that despite its much reduced cell-permeabilizing (cytolytic) activity, it remains fully active in antigen delivery into CD11b⁺ APCs. The inventors have further shown that the overall cytolytic activity of the illustrative CyaA/E570Q+K860R/AC⁻ toxoid is very low. It is thus devoid of residual toxicity in an animal or human host and is therefore highly suitable for use in therapy.

The invention thus provides new polypeptides, which are toxoids and have an enhanced safety profile and can be used as proteinaceous vectors for the delivery of molecules of interest, in particular of immunogenic peptidic sequences, to cells of a patient in need of a treatment, and more particularly to cells expressing CD11b.

Based on the experiments carried out by the inventors it has thus been possible to define and provide a polypeptide which is a mutant of the adenylate cyclase protein (CyaA) of *Bordetella pertussis* having the amino acid sequence disclosed as SEQ ID NO: 1 and is capable of binding to cells and of translocating its N-terminal adenylate cyclase enzyme domain into said cells wherein said cells express the CD11b/CD18 receptor and wherein binding to said cells occurs through binding to said CD11b/CD18 receptor, and which polypeptide has a pore-forming activity which is reduced or suppressed as compared to that of the native CyaA toxin, and which has an amino acid sequence which differs from the amino acid sequence disclosed as SEQ ID NO: 1 by:
(i) the substitution of the glutamic acid residue corresponding to the position 570 in SEQ ID NO: 1 by a glutamine residue or by a conservative amino acid residue selected from the group consisting of Asn, Met, Thr, Ser, Gly, Arg, Lys, Val, Leu, Cys, Ile and Asp; and
(ii) the substitution of the lysine residue corresponding to the position 860 in SEQ ID NO: 1 by an arginine residue or by a conservative amino acid residue selected from the group consisting of Asn, Gln, Met, Thr, Ser, Gly, Val, Leu, Cys and Ile; and
(iii) optionally from 1 to 10 amino acid residue substitutions, deletions, and/or insertions at locations other than the locations recited in (i) and (ii), said obtained polypeptide having an amino acid sequence which shares at least 99% identity with the sequence set forth in SEQ ID NO: 1.

For the purpose of the invention, the N-terminal domain of the described fragment is the amino acid sequence of the fragment which includes the contiguous amino acid residues of the N-terminal part of the native CyaA protein, e.g. the N-terminal part of the fragment is all or part of the contiguous residues forming the sequence of 400 amino acid residues of the N-terminal domain of the *Bordetella pertussis* CyaA protein.

Herein, "E570Q" encompasses substitution of the glutamic acid residue at position 570 of native CyaA of *Bordetella pertussis* by a glutamine residue or by another conservative residue, in particular a residue whose side chain size and hydrophilic nature is close to that of glutamic acid. The glutamic acid residue at position 570 is preferably substituted by an amino acid residue selected from Gln, Asn, Met, Thr, Ser, Gly, Arg, Lys, Val, Leu, Cys, Ile, Asp.

Herein, "K860R" encompasses substitution of the lysine residue at position 860 of native CyaA of *Bordetella pertussis* by an arginine residue or by another conservative residue, in particular a residue whose side chain size and hydrophilic nature is close to that of lysine. The lysine residue at position 860 is preferably substituted by an amino acid residue selected from Arg, Asn, Gln, Met, Thr, Ser, Gly, Val, Leu, Cys, Ile.

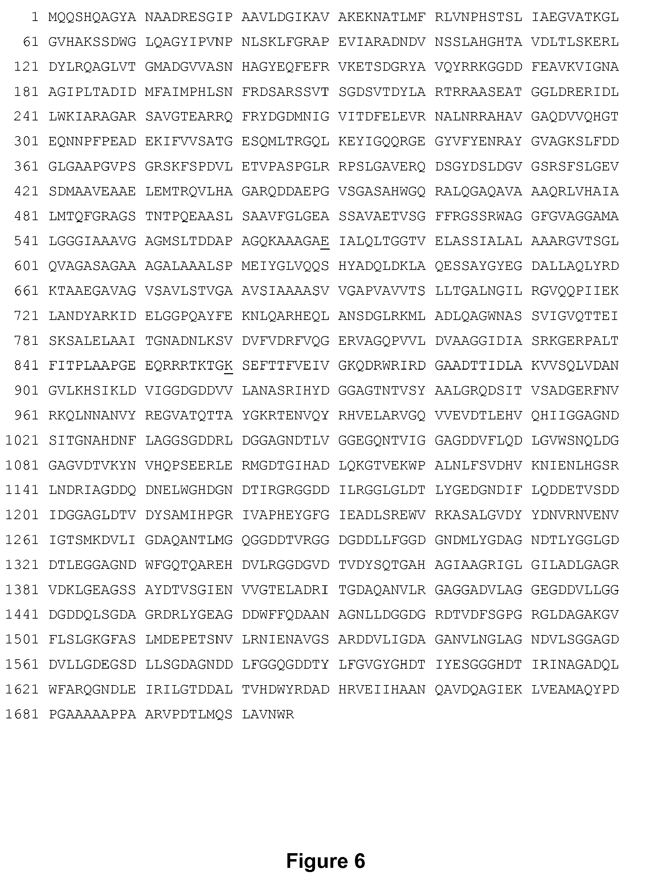

The native CyaA of *Bordetella pertussis* has also been described as an amino acid sequence and a nucleotide sequence by Glaser, P. et al, 1988, Molecular Microbiology 2(1), 19-30. This sequence is referred to as SEQ ID N°1 as illustrated in figure 6. Accordingly, when amino acid residues or sequences or nucleotides or nucleotide sequences of the CyaA protein of *B*. *pertussis,* are cited in the present invention their positions are given with respect to the sequences disclosed in said publication of Glaser et al. 1988.

In an embodiment of the present invention the amino sequence of the *Bordetella pertussis* adenylate cyclase is the sequence disclosed as SEQ ID N°1.

When reference is made to SEQ ID N°1 or to SEQ ID N°2 herein, it is especially pointed out that, unless it is technically not relevant, the disclosed features would similarly apply to a sequence modified by insertion of residues in SEQ ID N°1 or SEQ ID N°2 in order to detoxify the CyaA protein. In such a case, the numbering of the amino acid residues should be adapted (especially insofar as positions 570 and 860 of the native sequence are concerned).

Advantageously, the CyaA protein or a fragment thereof is a protein or a fragment thereof, which is the result of the co-expression in a cell, especially in a recombinant cell, of both cyaA and cyaC genes. It has been indeed shown that in order to have invasive properties for target cells, CyaA has to undergo post-translational modifications which are enabled by the expression of both cyaA and cyaC genes (WO 93/21324).

As described herein, the CyaA protein is a bacterial protein. As described herein, CyaA protein is derived from a *Bordetella species.*

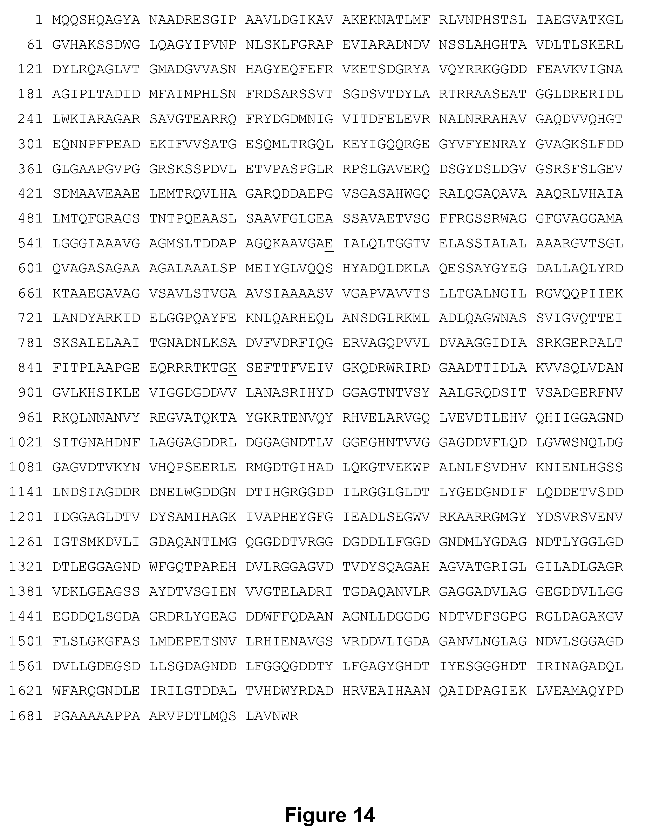

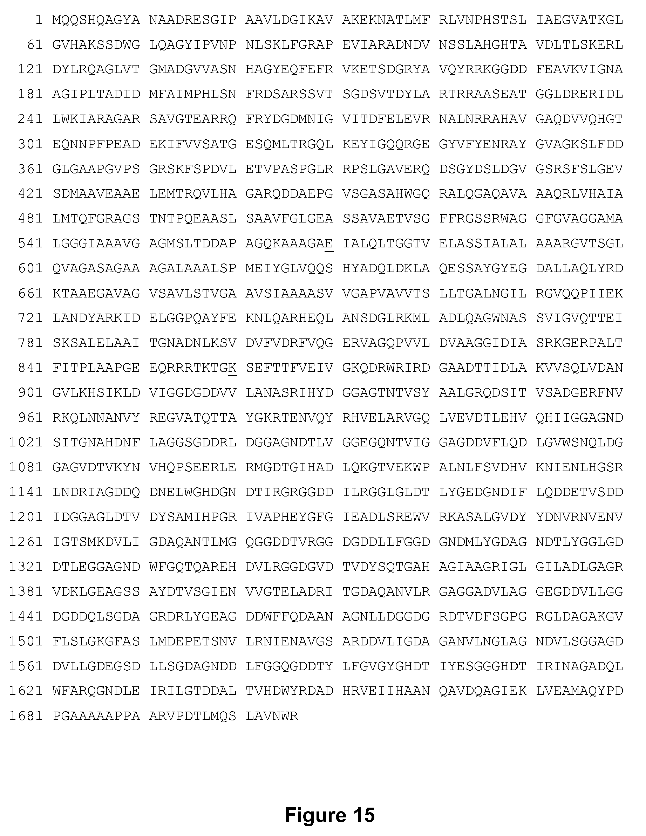

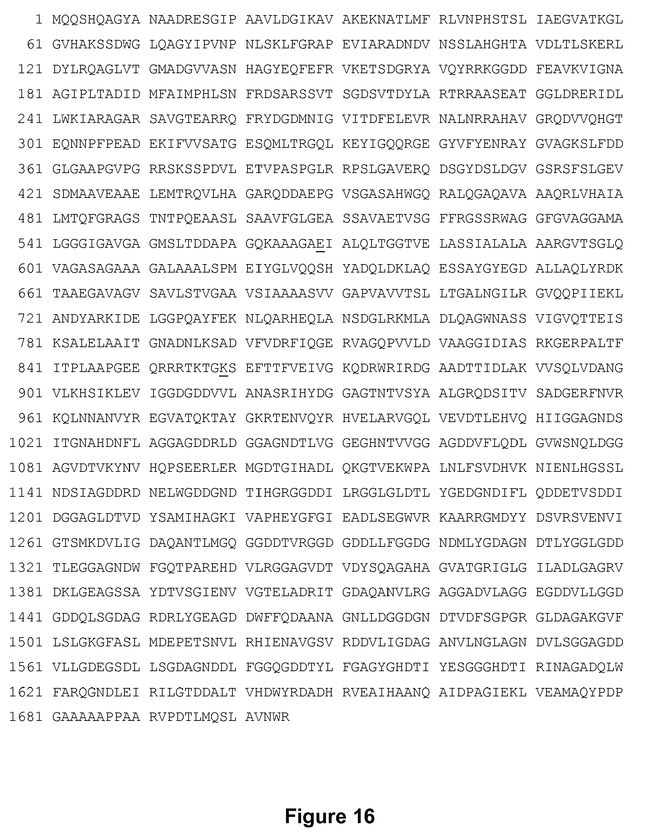

The *Bordetella species* of the invention is *Bordetella pertussis.* Other *Bordetella* strains are those of *Bordetella parapertussis, Bordetella hinzii* or *Bordetella bronchiseptica.* The sequence of CyaA protein of *B*. *parapertussis* has been disclosed especially under accession number NC 002928.3 (as a sequence of 1740 amino acids) and in Parkhill J. et al (Nat. Genet. DOI, 10 (2003)), for *B*. *hinzii* in Donato G.M. et al. (J. Bacteriol. 2005 Nov, 187(22):7579-88) and for *B*. *bronchiseptica* in Betsou F. et al (Gene 1995, August 30; 162(1): 165-6). The sequence of *Bordetella parapertussis* has been disclosed especially under accession number CAB76450, referred to herein as SEQ ID N°7 as illustrated in figure 14. The sequence of *Bordetella hinzii* has been disclosed especially under accession number AAY57201, referred to herein as SEQ ID N°8 as illustrated in figure 15. The sequence of *Bordetella bronchiseptica* has been disclosed especially under accession number CAA85481, referred to herein as SEQ ID N°9 as illustrated in figure 16. Accordingly, when amino acid residues or sequences of the CyaA protein of *Bordetella parapertussis, Bordetella hinzii* or *Bordetella bronchiseptica,* are cited in the present disclosure their positions are given with respect to the sequences disclosed in SEQ ID N°7, 8 and 9 respectively.

The expression "polypeptide mutant of the adenylate cyclase protein" excludes the native adenylate cyclase as expressed by *Bordetella*. As stated above, it is characterised by a primary difference with the native protein, lying in the combined substitution of two specific amino acid residues. It may be further modified with respect to said native protein and it may especially be a fragment of the thus mutated protein, such as for example a truncated variant of said mutated protein, wherein residues at either or both terminal ends are deleted. In particular residues at the C-terminal end may be deleted to the extent that it does not affect the recognition and binding site for the CD11b/CD18 cell receptor. Alternatively or in addition residues may be deleted at the N-terminal end provided that it does not affect the translocation ability of the obtained mutant polypeptide. It may also be a fragment obtained after internal deletions of one or more residues of the native mutated CyaA protein.

The disclosure also relates to a polypeptide mutant which is a fragment as stated herein, said fragment which necessarily comprises the mutated residues E570Q and K860R (when reference is made to the amino sequence of the CyaA protein of *Bordetella pertussis)* also retains the ability of the mutated full-length CyaA to bind cells and to translocate its N-terminal domain into the cytosol of target cells, especially of CD11b/CD18 expressing cells.

The invention provides thus mutant polypeptides suitable for use in the design of means for the delivery of one or more molecules into a cell, especially a target cell expressing the CD11b/CD18 receptor.

In particular the invention provides mutant polypeptides of a CyaA protein, where said protein is either derived from the CyaA toxin or is preferably derived from a toxoid thereof, especially a CyaA/AC⁻ toxoid. The mutant polypeptides are capable of binding to a cell, especially a target cell expressing the CD11b/CD18 receptor, are capable of translocating their N-terminal domain or the molecule inserted in said domain or grafted on it into the cell and their pore-forming activity is totally or partially suppressed as compared to that of the CyaA toxin or toxoid.

The capacity of the mutant polypeptide to target CD11b/CD18 cells can be assayed especially according to the methods disclosed in EP 03291486.3 and El-Azami-El-Idrissi M. et al, J. Biol. Chem., 278(40)38514-21 or in WO 02/22169. Furthermore, the capacity of the mutant polypeptide to translocate the epitope(s) or polypeptide(s) containing said epitope(s) into the cytosol of target cell can be assayed by applying the method described in WO 02/22169.

Total or partial suppression of the CyaA toxin or toxoid pore-forming activity, or cell-permeabilizing capacity, is to be understood as the total or partial suppression of the ability to form pores, in particular cation selective pores of an estimated diameter of 0.6 to 0.8 nm, which permeabilize a cellular membrane and eventually provoke colloid-osmotic cell lysis. The pore-forming activity can be measured using the single whole cell patch-clamp experiment as described in examples.

The pore-forming activity of the CyaA toxin contributes to its overall cytolytic or haemolytic activity on cells. Indeed in the context of the present invention, the overall cytolytic or haemolytic activity of CyaA (or its "overall cytotoxic activity") is to be understood as the resultant of at least the adenylate cyclase and pore-forming activities of the CyaA toxin. Thus total or partial suppression of the CyaA toxin pore-forming activity allows at least a partial suppression of its cytolytic activity.

In a preferred embodiment, the overall cytolytic activity of the polypeptide according to the invention, in particular on cells which express the CD11b/CD18 receptor, is totally or partially reduced as compared to that of the *Bordetella pertussis* CyaA toxin. The cytolytic activity of the inventive polypeptide can be determined by measuring the amount of hemoglobulin (for erythrocytes) or of lactate dehydrogenase (for monocytes) released by the cells when incubated with the tested polypeptide as described in examples.

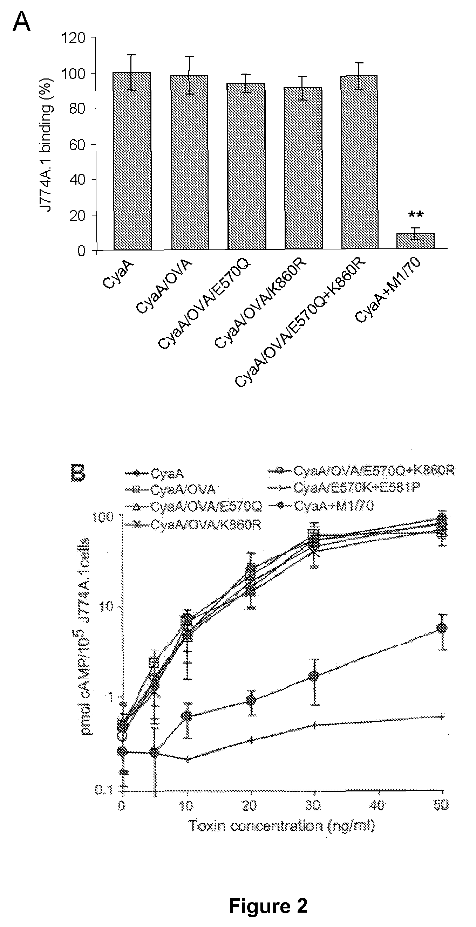

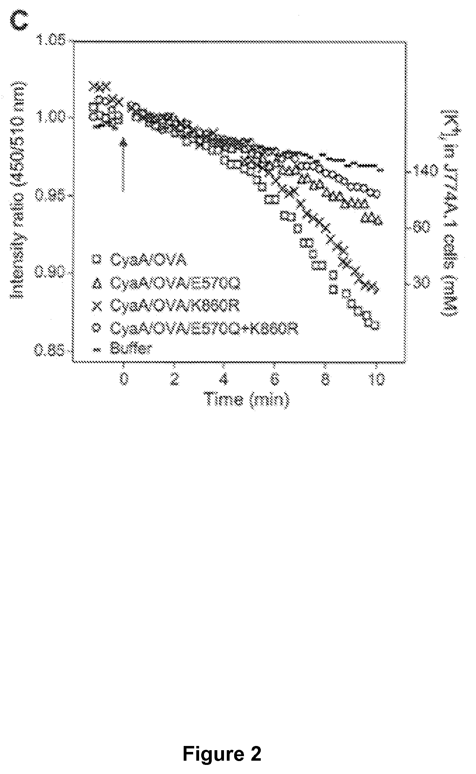

In a preferred embodiment, the overall cytolytic activity of the polypeptide according to the invention on cells which express the CD11b/CD18 receptor is at least 75% lower, preferably still at least 80%, 85%, 90% or 95% lower, than that the *Bordetella pertussis* CyaA toxin, or than that of a *Bordetella pertussis* CyaA protein whose adenylate cyclase activity is partly or totally suppressed (or "CyaA toxoid"). In a particularly preferred embodiment, the overall cytolytic activity of the polypeptide according to the invention on cells which express the CD11b/CD18 receptor is at least 75% lower, preferably still at least 80% or 85% lower, than that the *Bordetella pertussis* CyaA toxoid whose amino acid sequence is shown in figure 2 (SEQ ID N°2).

In a preferred embodiment, the invention relates to a polypeptide which is a mutant of an adenylate cyclase and whose amino acid sequence comprises or consists of an amino acid sequence (i) which is mutated with respect to the amino acid sequence disclosed in SEQ ID N°1 said mutations comprising at least the substitutions E570Q and K860R and wherein the polypeptide is capable of binding to a target cell and of translocating its N-terminal domain into the cell.

As described herein, the fragment including a substitution of the glutamic acid residue at position 570 of SEQ ID N°1 by a glutamine residue (referred to as "E570Q"), and the substitution of the lysine residue at position 860 of SEQ ID N°1 by an arginine residue (referred to as "K860R") encompasses at least the amino acid sequence of the CyaA protein starting with the first N-terminal residue or from one of the amino acid residues comprised between the positions 1 and 400, preferably between the positions 1 and 380 and extending up to the residues forming the recognition and binding site for the CD11b/CD18 cell receptor and said fragment contains residues corresponding to the mutated E570Q and K860R residues or consists of said amino acid sequence. As described herein, the fragment including the E570Q and K860R substitutions does not comprise the amino acid sequence running from the amino acid at position 1 of SEQ ID N°1 to the amino acid at position 372 of SEQ ID N°1.

As described herein, the fragment which is thus prepared has essentially lost the adenyl cyclase enzyme activity (AC activity).

In a preferred embodiment, the mutant polypeptide of the invention is produced by co-expression in a recombinant cell of a mutated gene encoding the E570Q and K860R mutated CyaA amino acid sequence and of the cyaC gene, followed by recovery of the selected expressed fragment of mutant CyaA.

Preferably, the mutant polypeptide of the invention has a lysine residue which corresponds to the lysine residue at position 983 of the CyaA amino acid sequence as set forth in SEQ ID N°1 and which is acylated, in particular which is palmytoylated or palmitoleilated.

Alternatively, the mutant polypeptide of the invention has a lysine residue which corresponds to the lysine residue at position 983 of the CyaA amino acid sequence as set forth in SEQ ID N°1 which is not acylated.

In a specific embodiment, the mutant polypeptide of the invention has an amino acid sequence derived from the CyaA amino acid sequence disclosed in SEQ ID N°1 by mutation of residues resulting in E570Q and K860R and has an amino acid sequence which shares at least 99% identity with the sequence set forth in SEQ ID N°1.

In another specific embodiment, the mutant polypeptide of the invention has an amino acid sequence which differs from the CyaA amino acid sequence as set forth in SEQ ID N°1 by mutation of residues resulting in E570Q and K860R and by further mutations resulting in 1 to 10 or 1 to 5 amino acid residue substitutions, deletions, and/or insertions including the E570Q and K860R substitutions, said obtained polypeptide having an amino acid sequence which shares at least 99% identity with the sequence set forth in SEQ ID NO: 1.

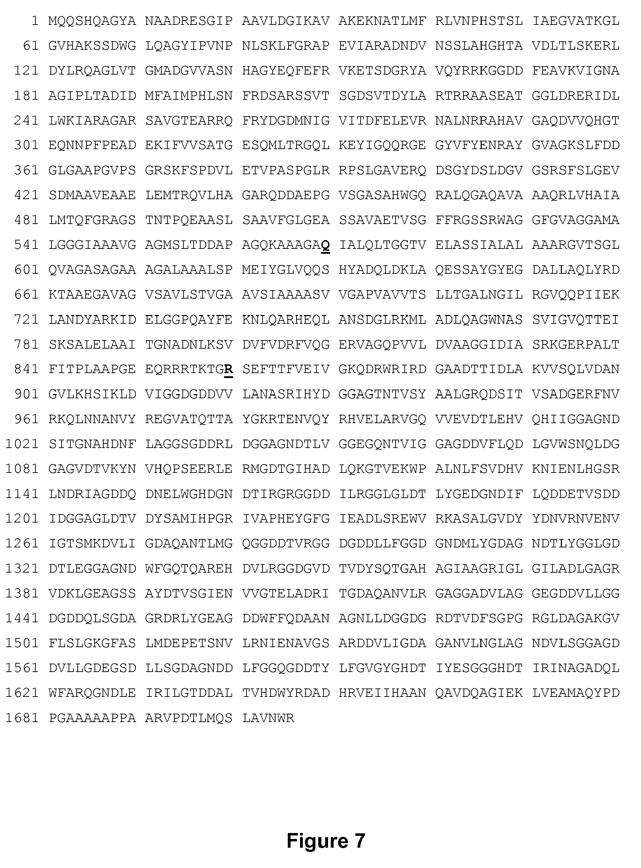

In a specific embodiment, the mutant polypeptide of the invention does not carry any amino acid residue substitutions, deletions, and/or insertions as compared to the *Bordetella pertussis* CyaA amino acid sequence other than the E570Q and K860R substitutions. In a specific embodiment, the mutant polypeptide has amino acid sequence of SEQ ID N°2 as illustrated in figure 7. In another specific embodiment, the only further amino acid substitutions, deletions, and/or insertions as compared to the amino acid sequence of SEQ ID N°2 consist in amino acid substitutions, deletions, and/or insertions which totally or partially suppress the adenyl cyclase enzymatic activity of the CyaA protein, such as in particular the insertion of a dipeptide, for example an "LQ" or "GS" dipeptide between the amino acids corresponding to the positions 188 and 189.

In a particular embodiment, the mutant polypeptide of the invention differs from the CyaA amino acid sequence as set forth in SEQ ID N°1 by 1, 2, 3, 4, 5, 6, 7, 8, 9 or 10 amino acid residue substitutions, deletions, and/or insertions in addition to the E570Q and K860R substitutions.

In a particular embodiment, in addition to the E570Q and K860R substitutions, the leucine residue at position 247 of the native CyaA protein of *Bordetella pertussis* is substituted by a glutamine residue (L247Q) or by another amino acid residue in particular a conservative amino acid residue.

The disclosure describes a mutant polypeptide which is a fragment as disclosed herein of the amino acid sequence disclosed in SEQ ID N°1 is to be understood as a sequence which comprises one or more fragments having at least about 350 amino acid residues and up to about 1705 amino acid residues of the SEQ ID N°1 amino acid sequence, in particular fragments comprising a stretch of at least 400, 500, 600, 700, 800, 900, 1000, 1100, 1200, 1300, 1400, 1500, 1600 amino acid residues of SEQ ID N°1, encompassing residues E570Q and K860R. A mutant polypeptide as described herein can also be defined as a fragment of the amino acid sequence disclosed in SEQ ID N°2 which comprises one or more fragments having at least about 350 amino acid residues and up to about 1705 amino acid residues of the SEQ ID N°2 amino acid sequence, in particular fragments comprising a stretch of at least 400, 500, 600, 700, 800, 900, 1000, 1100, 1200, 1300, 1400, 1500, 1600 amino acid residues of SEQ ID N°2, encompassing residues 570 and 860. Said fragments preferably retain the capacity of binding to the CD11b/CD18 cell receptor and the ability to translocate their N-terminal domain into target cells. Preferably, the disclosure describes a mutant polypeptide which is such a fragment which has a stretch of amino acids comprising amino acid residues 570 as E570Q to 860 as K860R or 1 to 860, or 2 to 860 of SEQ ID N°1 to the extent that the E570Q and K860R mutations are observed with respect to the original SEQ ID N°1.

As described herein, the fragment further comprises amino acid residues 1166 to 1281 or amino acid residues 1208 to 1243 of the CyaA amino acid sequence as set forth in SEQ ID N°1 of CyaA protein for interaction with CD11b/CD18 target cells.

As described herein, a particular fragment thus encompasses all or part of the C-terminal part of the native protein which part is responsible for the binding of the polypeptide of the invention to target cell membrane and/or CD11b/CD18 receptor, and for the subsequent delivery of the N-terminal domain of the polypeptide into the cell cytosol. A particular polypeptide as described herein is the fragment of CyaA protein which contains amino acid residues 373 to 1706 of CyaA protein especially of the SEQ ID N°1, to the extent that residues 570 and 860 are mutated as E570Q and K860R.

As described herein, the mutant polypeptide which is such a fragment comprises :
a) a first amino acid sequence which corresponds to a stretch of at least 100 contiguous amino acid residues from SEQ ID N°1 comprising amino acid residues 570 as E570Q, and further including 0, 1, 2, 3, 4 or 5 deletions, substitutions or insertions as compared to SEQ ID N°1 and
b) a second amino acid sequence which corresponds to a stretch of at least 100 contiguous amino acid residues from SEQ ID N°1 comprising amino acid residues 860 as K860R, and further including 0, 1, 2, 3, 4 or 5 deletions, substitutions or insertions as compared to SEQ ID N°1 and preferably,
c) a third amino acid sequence comprising amino acid residues 1166 to 1281 or amino acid residues 1208 to 1243 of the CyaA amino acid sequence as set forth in SEQ ID N°1 of CyaA protein for interaction with CD11b/CD18 target cells.

Another particular polypeptide as described herein is a fragment which is one which corresponds to the E570Q and K860R mutated CyaA protein wherein amino acid residues 225 to 234 have been deleted, thus providing a fragment containing residues 1 to 224 and 235 to 1706 of the mutated protein.

As described herein, the polypeptide fragment binds to a cell which expresses the CD11b/CD18 receptor as a result of specific binding to said receptor.

In a preferred embodiment, adenylate cyclase activity of the polypeptide in a cell is partly or totally suppressed as compared to that of the *Bordetella pertussis* CyaA toxin. As stated above, the expression "CyaA protein" relates either to the toxin form or preferably to the toxoid form of the protein. Accordingly each embodiment of the invention relating to the polypeptide which is a mutant of the CyaA protein applies to each of the toxin or toxoid form of the protein.

Total or partial suppression of CyaA adenylate cyclase or enzymatic activity is to be understood as the total or partial suppression of the ability to convert ATP into cAMP in a cellular environment as compared to that of a CyaA toxin produced by co- expression of the cyaA and cyaC genes in a cell. The ability to convert ATP into cAMP can be determined by measuring the level of intracellular cAMP as described in the examples.

Such total or partial suppression can be obtained as a result of genetic inactivation, for example by introduction of a short amino acid sequence, comprising for example from one to ten amino acids, in particular a dipeptide in a site of the amino acid sequence of CyaA which is part of the catalytic site, i.e. in a site located within the first 400 amino acids (AC domain) of SEQ ID N°1 or by deletion or substitution of a part of the CyaA amino acid sequence as set forth in SEQ ID N°1 which is essential for enzymatic activity. In a preferred embodiment, total or partial suppression of the CyaA enzymatic activity is obtained by insertion of a dipeptide, for example an "LQ" or "GS" dipeptide, between the amino acids corresponding to the positions 188 and 189 of the CyaA sequence as set forth in SEQ ID N°1. This can be achieved by inserting an oligonucleotide, such as "CTG CAG" or "CGATCC", at the EcoRV site at position 564 of the coding phase of the cyaA gene. See Ladant et al., 1992. Alternatively, total or partial suppression of the enzymatic activity can also be obtained by directed mutagenesis, for example, by replacing the lysine residue at position 58 or 65 of the native CyaA *Bordetella pertussis* protein (Glaser et al., 1989) by a Gln residue.

The polypeptide according to the invention can also be defined as a polypeptide which may be obtained from a CyaA polypeptide having an amino acid sequence according to SEQ ID N° 1, by:
(i) substituting the glutamic acid residue at position 570 of SEQ ID N° 1 by a glutamine residue or by a conservative amino acid residue,
(ii) substituting the lysine residue at position 860 of SEQ ID N° 1 by an arginine residue or by a conservative amino acid residue, and
(iii) optionally, carrying out from one to 10 amino acid residue substitutions, insertions and/or deletions at locations other than the locations recited in i) and ii), provided that the polypeptide thus obtained has the capacity of the CyaA protein of *Bordetella pertussis* to bind to a target cell and translocate its N-terminal adenylate cyclase enzyme domain or part thereof into said cell.

In step i), the glutamic acid residue is substituted by an amino acid residue selected from Gln, Asn, Met, Thr, Ser, Gly, Arg, Lys, Val, Leu, Cys, Ile, Asp, most preferably by Gln. In step ii), the lysine residue is substituted by an amino acid residue selected from Arg, Asn, Gln, Met, Thr, Ser, Gly, Val, Leu, Cys, Ile, most preferably by Arg.

In a specific embodiment, no further amino acid residue substitutions, insertions and/or deletions are carried out in step iii).

In particular, step iii) may comprise truncation of either or both terminal ends. In particular, residues at the C-terminal end may be deleted to the extent that it does not affect the recognition and binding site for the CD11b/CD18 cell receptor. Alternatively or in addition, residues may be deleted at the N-terminal end provided that it does not affect the translocation ability of the obtained mutant polypeptide. Internal deletions of one or more residues of the native CyaA protein located at positions other than those recited in steps i) and ii) may also be performed. As described herein, step iii) comprises the deletion of up to 380 or up to 400 amino acids in the N-terminal amino acid sequence of the CyaA polypeptide, preferably the deletion of the stretch of amino acids running from the amino acid at position 1 of SEQ ID N°1, 7, 8 or 9 to the amino acid at position 372 of SEQ ID N°1, 7, 8 or 9.

Preferably, after performing step iii), the obtained polypeptide encompasses all or part of the C-terminal part of the native protein which part is responsible for the binding of the polypeptide of the invention to the membrane of the target cell and/or CD11b/CD18 receptor, and for the subsequent delivery of the N-terminal domain of the polypeptide into the cell cytosol. In a particular embodiment, in step iii), amino acid residues 373 to 1706 of SEQ ID N°1 are not deleted. In a preferred embodiment, in step iii), amino acid residues 1208 to 1243 of SEQ ID N°1 are not deleted.

In a preferred embodiment, step iii) comprises amino acid substitutions, deletions, and/or insertions which totally or partially suppress the adenyl cyclase enzymatic activity of the CyaA protein. Such total or partial suppression can be obtained by introduction of a short amino acid sequence, comprising from one to ten amino acids, in particular a dipeptide in a site located within the first 400 amino acids (AC domain) of SEQ ID N°1 or by deletion or substitution of a part of the CyaA amino acid sequence as set forth in SEQ ID N°1 which is essential for enzymatic activity. In a preferred embodiment, total or partial suppression of the CyaA enzymatic activity is obtained by insertion of a dipeptide, for example an "LQ" or "GS" dipeptide, between the amino acids at positions 188 and 189 of the CyaA sequence as set forth in SEQ ID N°1. Alternatively, total or partial suppression of the enzymatic activity can also be obtained by directed mutagenesis, for example, by replacing the lysine residue at position 58 or 65 of the native CyaA *Bordetella pertussis* protein (Glaser et al., 1989) by a Gln residue.

Preferably, in step iii) the lysine residue at position 983 of the CyaA amino acid sequence as set forth in SEQ ID N°1 is neither substituted nor deleted. In one embodiment, this lysine residue is acylated, in particular it is palmytoylated or palmitoleilated. Alternatively, this lysine residue is not acylated.

Preferably, after performing step iii), the obtained polypeptide has an amino acid sequence which shares at least 99% identity with the sequence set forth in SEQ ID N°1.

Still preferably, after performing step iii), the obtained polypeptide has an amino acid sequence which differs from the sequence set forth in SEQ ID N°1 by the amino acid residue substitutions resulting from steps i) and ii), and by 1 to 10 or 1 to 5 further amino acid residue substitutions, deletions, and/or insertions. In a particular embodiment, after performing step iii), the obtained polypeptide differs from the CyaA amino acid sequence as set forth in SEQ ID N°1 by 1, 2, 3, 4, 5, 6, 7, 8, 9 or 10 amino acid residue substitutions, deletions, and/or insertions in addition to the substitutions carried out in steps i) and ii).

The invention is also directed to a polypeptide derivative comprising or consisting of the mutant polypeptide according to the invention which is further combined with one or more molecules of interest. In a preferred embodiment, a molecule of interest is a biologically active molecule either when taken alone or when combined to the polypeptide of the invention. Said molecules may especially be of prophylactic value or therapeutic value i.e., may have a prophylactic or a therapeutic activity, or may enhance a prophylactic or therapeutic activity.

In specific embodiments, the molecules of interest are selected in the group comprising: peptides, glycopeptides, lipopeptides, polysaccharides, oligosaccharides, nucleic acids, lipids and chemicals.

In a specific embodiment, the one or more molecules of interest are polypeptidic molecules or contain polypeptidic molecules. Their amino acid sequence may comprise 2 to 1000, preferably 5-800, 5 to 500, 5 to 200, 5 to 100, 8 to 50, 5 to 25, 5 to 20 or 8 to 16, or 300-600, 400-500, amino acid residues.

In a preferred embodiment, the one or more molecules of interest are heterologous amino acid sequences suitable for eliciting an immune response (also referred to as "heterologous antigens"), in particular amino acid sequences which comprise or consist of an epitope, including antigens. As used herein, the term "heterologous" refers to an antigen other than the mutant polypeptide which is used in the vector itself. As used herein, the term "epitope" refers to a heterologous molecule and especially a heterologous peptide that can elicit an immune response, when presented to the immune system of a host. In particular, such an epitope can comprise or consist of a stretch of 8, 9, 10, 11, 12, 13, 14, 15 or 16 amino acid residues. It may alternatively consist in a full-length antigen or consist in antigen(s) fragment(s).

In a specific embodiment, a polypeptide derivative according to the invention can be encoded by a plasmid which corresponds to the OVA-QR-AC-plasmid deposited under accession number CNCM I-4137 (figure 12) in which the DNA sequence encoding the "OVA" antigenic sequence is replaced by a DNA sequence encoding an antigenic sequence comprising one or more epitopes.

The polypeptidic molecule suitable for eliciting an immune response is especially one eliciting a T-cell immune response, including as an example a CTL response. The polypeptidic molecule suitable for eliciting an immune response can also be one eliciting a B-cell immune response.

In specific embodiments, the heterologous antigen is selected from the group consisting of an antigen of a bacterial pathogen, a tumoral cell antigen, a viral antigen, a retroviral antigen, a fungus antigen or a parasite cell antigen.

A molecule of interest can be especially an antigen selected from the group consisting of: a Chlamidia antigen, a Mycoplasma antigen, a hepatitis virus antigen, a poliovirus antigen, an HIV virus antigen, an influenza virus antigen, a choriomeningitis virus antigen, a tumor antigen, or a part of any of these antigens which comprises at least an epitope.

In a preferred embodiment of the polypeptide derivative of the invention, the amino acid sequence of each of said molecule(s) suitable for eliciting an immune response comprises or consists of an amino acid sequence of a Chlamidia antigen, a Mycoplasma antigen, a hepatitis virus antigen, a poliovirus antigen, an HIV virus antigen, an influenza virus antigen, a choriomeningitis virus sequence, a tumor antigen, or comprises or consist of a part of an amino acid sequence of any these antigens which comprises at least one epitope.

In a particularly preferred embodiment, the molecule of interest is a tumor associated antigen (TAA). Tumor-associated antigens have been characterized for a number of tumors such as for example: Melanoma, especially metastatic melanoma; Lung carcinoma; Head & neck carcinoma; cervical carcinoma, Esophageal carcinoma; Bladder carcinoma, especially infiltrating Bladder carcinoma; Prostate carcinoma; Breast carcinoma; Colorectal carcinoma; Renal cell carcinoma; Sarcoma; Leukemia; Myeloma. For these various histological types of cancers, it has been shown that antigenic peptides are specifically expressed on tumor samples and are recognized by T cells, especially by CD8⁺ T cells or CD4⁺ T cells.

A review of peptides found as tumor-associated antigens in these types of tumors is made by Van der Bruggen P. et al (Immunological Reviews, 2002, vol 188:51-64). Especially, the disclosure of the peptides contained in table 3 of said review is referred to herein as providing examples of such tumor-associated antigens and said table 3 is incorporated by reference to the present application.

The following antigens are cited as examples of tumor-associated antigens recognized by T cells, according to the publication of Kawakami Y. et al (Cancer Sci, October 2004, vol.95, no. 10, p784-791) that also provides methods for screening these antigens or further one: antigens shared by various cancers, including MAGE (especially in Melanoma), NY-ESO-1, Her2/neu, WT1, Survivin, hTERT, CEA, AFP, SART3, GnT-V, antigens specific for some particular cancers such as βbeta-catenin, CDK4, MART-2, MUM3, gp100, MART-1, tyrosinase for Melanoma; bcr-abl, TEL-AML1 for Leukemia; PSA, PAP, PSM, PSMA for prostate cancer; Proteinase 3 for myelogenous leukemia; MUC-1 for breast, ovarian or pancreas cancers; EBV-EBNA, HTLV-1 tax for lymphoma, ATL or cervical cancer; mutated HLA-A2 for Renal cell cancer; HA1 for leukemia/lymphoma. Tumor-associated antigens in animals have also been described such as Cycline D1 and Cycline D2 in tumors affecting cats or dogs.

Tumor-associated antigens recognized by T cells have also been disclosed in Novellino L. et al (Immunol Immunother 2004, 54:187-207).

More generally, TAA of interest in the present invention are those corresponding to mutated antigens, or to antigens that are overexpressed on tumor cells, to shared antigens, tissue-specific differenciation antigens or to viral antigens.

In a particular embodiment of the invention, the tumor-associated antigen is an antigen of papillomavirus (HPV) or is tyrosinase.

According to another particular embodiment of the invention, the amino acid sequences of the polypeptidic molecules which comprise or consist of an epitope have been modified with respect to their native amino acid sequence, for example in order to decrease the number of negatively charged amino acid residues within the sequence. Such a modification can be obtained by removing some of these negatively charged amino acid residues or also by adding some positively charged amino acid residues, especially as flanking residues of the epitopes. Polypeptides thus comprising less negatively charged residues might favour the translocation of the catalytic domain of the polypeptide derivative of the invention in the cytosol of target cells.

The amino acid sequences of the polypeptidic molecules which comprise or consist of an epitope or an antigen can also be designed in such a way that they are unfolded when they are inserted in the polypeptide derivative of the invention, which improves efficiency of the internalization of the molecule(s) of interest according to the invention into the target cells. Such unfolding in polypeptides which undergo folding as a consequence of their amino acid content, can be obtained for instance, by removing or substituting cystein residues in order to avoid formation of disulfide bonds that may be involved in folding of polypeptides. In some cases, it is possible to prevent folding of the polypeptides by preparing them in the presence of reducing agents to enable avoiding *in vivo* refolding.

In a particular embodiment, the amino acid sequences, especially the antigen, can comprise or consist of cryptic epitopes.

The inventors have indeed determined that polypeptide derivative constructs, which comprise (i) a polypeptide of the invention which is a mutant of a CyaA protein (polypeptide mutant) according to the definitions disclosed herein and (ii) a polypeptidic molecule having an amino acid sequence bearing one or several antigenic fragments of one or several antigens, enable cryptic epitopes of said antigens to become immunogenic as a result of their presentation in the constructs. Especially, said constructs involving mutant polypeptides as defined in the present invention comprising polypeptidic molecule(s) derived from antigens of interest for especially prophylactic or therapeutic applications, including immunotherapeutic vaccination, purposes are processed in target cells where the polypeptidic molecule(s) is allowed to be internalized as a result of the translocation of the N-terminal domain of the mutant polypeptide. Such processing enables epitopes presentation through the class I MHC molecules of the target cells, and said epitopes can comprise cryptic epitopes of the antigen which are allowed to become immunogenic and in particular to raise a T-cell response in a host, especially a CTL response.

The invention thus also relates to a polypeptide derivative, in particular to a recombinant protein comprising one or several polypeptidic molecules having an amino acid sequence bearing one or several epitopes of one or several antigens, or bearing said antigen(s) said amino acid sequence(s) of said polypeptidic molecule(s) being inserted in the same or in different sites, especially in different permissive sites of a mutant polypeptide according to the invention, said recombinant protein retaining the property of the CyaA toxin to target antigen presenting cells (APC), wherein at least one of said epitope(s) is a subdominant cryptic T-cell epitope and wherein said polypeptide derivative, especially said recombinant protein, is capable of eliciting an antigen-specific response against said polypeptidic molecule(s).

In a specific embodiment of the polypeptide derivative according to the invention, the one or more amino acid sequences are inserted into one or more sites, especially permissive sites.

For the present invention, a "permissive site" is a site of the sequence of the CyaA protein where a polypeptide can be inserted without substantially affecting the desired functional properties of the CyaA protein especially without substantially affecting the targeting of cells, particularly the targeting of antigen presenting cells (APC) by CyaA, including without substantially affecting the specific binding to the CD11b/CD18 receptor and advantageously without substantially affecting the domains of the protein involved in the process of translocation of the CyaA N-terminal domain into a target cell.

Methods to select for permissive sites are presented for example in WO93/21324, in Ladant et al., 1992, and in Osicka et al., 2000 (Infection and Immunity, 2000, 68(1):247-256). In particular, a methodology using a double selection (resistance to an antibiotic and colorimetric test on dishes by α-complementation) enables to identify readily oligonucleotides insertions (which preserve the reading frame) in the portion of the gene coding for the N-terminal catalytic domain of the toxin. The functional consequences of these mutations on the catalytic activity of the toxin may be readily analysed, both genetically (functional complementation of an *E. coli cya⁻* strain) and biochemically (characterization of the stability of the modified adenylcyclases, of their enzymatic activity, of their interaction with caM, etc.). This methodology has enabled a large number of mutations to be screened in order to identify the sites which are potentially advantageous for the insertion of antigenic determinants.

Permissive sites of the *Bordetella pertussis* adenylate cyclase allowing translocation of CyaA catalytic domain and hence translocation of amino acid sequences inserted into such permissive sites include, but are not limited to, residues 137-138 (Val-Ala), residues 224-225 (Arg-Ala), residues 228-229 (Glu-Ala), residues 235-236 (Arg-Glu), and residues 317-318 (Ser-Ala) (Sebo et al., 1995). The following additional permissive sites are also included in embodiments of the invention: residues 107-108 (Gly-His), residues 132-133 (Met-Ala), residues 232-233 (Gly-Leu), and 335-336 (Gly-Gln) and 336-337. However, other permissive sites may be used in the present invention, that can be identified for example by use of the methodology indicated above, especially sites between residues 400 and 1700 of the CyaA protein.

For other *Bordetella* species corresponding permissive sites can be defined by comparison of sequences and determination of corresponding residues.

According to another embodiment, the one or more amino acid sequence polypeptide can also or alternatively be inserted at one and/or the other extremities (ends) of the polypeptide of the invention, preferably at the N-terminal end of the mutant CyaA polypeptide lacking all or part of the N-terminal catalytic domain of the *Bordetella pertussis* CyaA protein, and more particularly lacking residues 1-373.

According to a specific embodiment, the one or more amino acid sequences suitable for eliciting an immune response, is grafted onto an amino acid residue of said polypeptide.

According to the invention, the "combination" (or insertion) of an amino acid sequence with the CyaA mutant polypeptide to provide a so-called polypeptide derivative, also referred to as a "recombinant protein" or a "hybrid protein", encompasses genetic insertion especially by available DNA technology. Alternatively, "combination" also encompasses non genetic insertion, including chemical insertion for instance covalent coupling carried out especially at one extremity of the amino acid sequence, or non covalent coupling. Non-genetic insertion can especially be of interest when the amino acid sequence to be inserted is synthetic or semi-synthetic. Methods for coupling a drug to a polypeptide are well known in the Art and comprise for example disulfide linkage by using N-pyridyl sulfonyl-activated sulfhydryl.

In particular, it is possible to graft molecules to the polypeptides of the invention by a chemical linkage or by genetic insertion for *in vivo* targeting to CyaA target cells, such as APC, for example CD11b/CD18 positive cells and particularly to the cytosol of said cells. Indeed, when coupling a molecule corresponding to a given CD8+ T-cell epitope to the catalytic domain of detoxified CyaA, either by means of a disulfide bond or by genetic insertion, it has been found that the engineered molecule can elicit *in vivo* specific CTL response, thereby showing that said CD8+ T-cell epitope is translocated into the cytosol of CD11b-expressing cells.

In a preferred embodiment of the invention, the mutant CyaA polypeptide is used in the manufacturing of a proteinaceous vector or in the preparation of a composition specifically designed to prime CD8+ cytoxic T-cell response (CTL response) when said response follows the targeting of the mutant CyaA polypeptide modified (especially recombined or conjugated) with a molecule of interest to CD11b expressing cells, followed by the translocation of the molecule of interest to the cytosol of said CD11b expressing cells, and in particular to myeloid dendritic cells. In this context, the molecule of interest is or comprises preferably an epitope or an antigen.

In another preferred embodiment of the invention, the mutant CyaA polypeptide is used in the manufacturing of the proteinaceous vector or in the preparation of a composition specifically designed to prime CD4+ cells response when said response follows the targeting of the adenylcyclase modified (especially recombined or conjugated) with a molecule of interest to CD11b expressing cells, in particular myeloid dendritic cells. In this context, the molecule of interest is or comprises preferably an epitope or an antigen.

The mutant polypeptides can also be used in the manufacturing of a proteinaceous vector for targeting of a prophylactic or a therapeutic compound to CD11b expressing cells. In this context, in one specific embodiment of the invention, the so-called molecule of interest has a prophylactic or therapeutic value and in particular is a drug. Said prophylactic or therapeutic compound and in particular said drug may be chemically or genetically coupled to the mutant polypeptide. Method for coupling a compound to a polypeptide are well known in the Art and comprise for example disulfide linkage by using N-pyridyl sulfonyl-activated sulfhydryl. In one embodiment, a molecule of interest is an antiinflammatory compound which is, when coupled to the mutant polypeptide, specifically targeted to the surface of the cells involved of the inflammatory response, such as dentritic cells or neutrophils.

More specifically, antigen presentation for selective CD8+ cytotoxic cells priming is mainly performed by myeloïd dendritic cells.

Accordingly, in a specific embodiment, the mutant CyaA polypeptide used for the manufacturing of proteinaceous vector is a genetically modified adenylcyclase containing one or more molecule(s) chemically coupled by means of a disulfide bond to genetically inserted cysteine residue(s) located within the catalytic domain of the mutant CyaA polypeptide. Indeed, multiple molecules can be chemically coupled to the mutant CyaA polypeptide by means of a disulfide bond to different cysteine residues located at different permissive sites within the catalytic domain.

The mutant polypeptides or polypeptide derivatives according to the invention are suitable for use in therapy or prophylaxis.

By therapy or therapeutic effect it is intended any effect which is beneficial to the condition of a patient, be it curative or sufficient to limit the symptoms or the consequences of a pathological condition, including limiting the progression of a pathological condition. By therapy or therapeutic effect is also encompassed the prevention of the onset of pathological condition.

The mutant polypeptides or polypeptide derivatives according to the invention are in particular suitable to elicit a cell-mediated immune response such as a T-cell immune response or a B-cell immune response in a host in need thereof. It includes CTL and Th, especially Th1 response, including CD4⁺ T cell response and/or CD8⁺ T cell response.

The ability of a polypeptide derived from CyaA protein to elicit a cell-mediated immune response may be sufficient to prevent tumor growth *in vivo* or even to enable tumor regression in an animal. It may also be enhanced by activation of innate component of the immune response through TLR activation and by down activating the regulatory component of the immune response through the use of chemotherapeutic agents. The invention provides means which should enable such results to be obtained in improved safety conditions as a result of the combined mutations E570Q and K860R, which have been selected.

The disclosure describes therapeutic methods comprising administration to an animal or human patient of the mutant polypeptide or polypeptide derivative according to the invention to a patient to elicit a T-cell immune response or a B-cell immune response in a host in need thereof.

The mutant polypeptides or polypeptide derivatives according to the invention can in particular be used for the prevention or the treatment of a disease selected from neoplasia, cancers and infectious diseases selected from viral-, retroviral-, bacterial- or fungal- induced diseases. In particular, the polypeptide derivatives can be used for the treatment of HIV infections in a patient.

It is especially provided that in a particular embodiment of the invention, the CyaA mutant polypeptide or polypeptide derivative is suitable for the treatment of infiltrating or vascularized tumors versus superficial tumors or for the treatment of metastatic tumors versus primary tumors, in accordance with the acknowledged clinical criteria for the classification of tumors.

Solid tumors are especially a target for the treatment through the use of the polypeptide derivative of the invention.

Among tumors which may be candidates for the treatment with the polypeptide derivative of the invention, the following, for which tumor-associated antigens have been characterized, are described as examples:
Melanoma, especially metastatic melanoma; Lung carcinoma; Head & neck carcinoma; cervical carcinoma, Esophageal carcinoma; Bladder carcinoma, especially infiltrating Bladder carcinoma; Prostate carcinoma; Breast carcinoma; Colorectal carcinoma; Renal cell carcinoma; Sarcoma; Leukemia; Myeloma. For these various histological types of cancers, it has been shown that antigenic peptides are specifically expressed on tumor samples and are recognized by T cells, especially by CD8⁺ T cells or CD4⁺T cells.

The invention further relates to the use of a polypeptide derivative according to the invention, for the preparation of a therapeutic composition for the treatment of a disease selected from neoplasia, cancers and infectious diseases selected from viral- or retroviral-induced diseases.

In a preferred embodiment, the polypeptide or polypeptide derivative according to the invention can be administered to the patient in combination with an adjuvant and/or in combination with another therapeutically active molecule or agent.

In the context of the present invention said "another therapeutically active molecule or agent" is one which may be beneficial to the condition of a patient to whom it is administered. It is especially an active principle suitable for use in the manufacturing of a drug. It may be a compound suitable to either, potentiate increase or modulate the effect of a therapeutically active principle.

The mutant CyaA poplypeptide or the poplypeptide derivative thereof can be administered with a therapeutically active molecule or agent, in particular one suitable for eliciting an immune response in a patient.

In particular, mutant CyaA poplypeptide or the poplypeptide derivative thereof can be administered with a therapeutically active agent suitable for modulating a cell response in a patient, in particular by lowering or blocking regulatory T cells immunosuppressive capacity.

According to a particular embodiment of the invention, such an effect on a regulatory cell response may be obtained with an agent modulating a regulatory T cell and/or modulating another cell suppressive response, such as the myeloid suppressive cells response, said agent targeting said regulatory cells, especially T cells, by depleting or inactivating these cells (such as with CD25-specific antibody, or cyclophosphamide), altering trafficking of said cells, especially regulatory T cells (such as CCL22-specific antibody) or altering differentiation and signalling of said cells (such as by blocking FOXP3 (forkhead box P3) signal).

According to a particular embodiment of the invention, the agent modulating a regulatory cell response targets suppressive molecules, especially such molecules present on APCs (such as B7-H1, B7-H4, IDO (indoleamine 2,3-dioxygenase) or arginase) or on T cells (such as CTLA4 (cytotoxic T-lymphocyte-associated antigen 4) or PD1 (programmed cell death 1)), or targets soluble immunosuppressive molecules (such as TGF beta (transforming growth factor), IL-10, VEGF (vascular endothelial growth factor), COX2 (cyclooxygenase 2)).

As examples of agents having an effect on a regulatory cell response, cytotoxic agents are proposed, that can kill regulatory T cells or other immunosuppressive cells, or that can block their activity and/or development and/or accumulation.

In a particular embodiment of the invention, the agent modulating the regulatory cell response, especially a regulatory T cell response, is a chemotherapeutic agent. Especially it is selected among chemotherapeutic agents known as anticancer agents and used in chemotherapy. Such agents include those helping to reduce the tumor burden, those acting by increasing sensitivity of tumor cells to treatment or those enabling killing or inactivating immune regulatory cells. The chemotherapeutic agents used within the frame of the invention thereby enhance antitumor immunity.

In a particular embodiment of the invention, the chemotherapeutic agent is an alkylating agent. Especially, it is Cyclophosphamide (CTX) (Sigma, Steinheim, Germany). Cyclophosphamide is capable of depleting or inactivating regulatory T cells.

In another particular embodiment of the invention, the chemotherapeutic agent is an intercalating agent.

In a particular embodiment, the chemotherapeutic agent is Doxorubicin (DOX) (Calbiochem, La Jolla, CA, USA).

The chemotherapeutic agent is advantageously administered by low doses.

The mutant CyaA poplypeptide or the poplypeptide derivative thereof can also be administered with an adjuvant component, suitable for activating the innate immune response primed by a tumor in a patient.

In a particular embodiment of the invention, the adjuvant component is selected in the group of components consisting of nucleic acids, peptidoglycans, carbohydrates, peptides, cytokines, hormones and small molecules, wherein said adjuvant component is capable of signaling through pattern-recognition receptors (PRRs).

PRRs are known to mediate the innate immune response to pathogens, and to tumors, by recognition of so-called evolutionarily conserved signatures from pathogens (pathogen-associated molecule patterns, PAMPs). PRRs are present on a variety of immune cells including dendritic cells, natural killer cells, B cells, and also on some non immune cells such as epithelial cells or endothelial cells. PRRs and their involvement in the innate immune response are described in Pashine A. et al (Nature medicine supplement volume 11, N° 4, April 2005).

In particular an adjuvant component for the activation of the innate immune response can target PRRs and therefore activate signaling through PRRs, wherein said PRRs encompass Toll-like receptors or nucleotide-binding oligomerization domain (NOD) or C type lectin.

In a particular embodiment of the invention, the adjuvant component is a Toll-like receptor (TLR) agonist. The Toll-like receptor agonist is especially formulated to efficiently activate the innate immune system of a patient. Said TLR agonist is capable of binding the TLR, i.e., is a ligand of the TLR and is furthermore capable of enhancing the immune response elicited under the control of said TLR.

For illustration, TLR agonists are selected from the group of TLR-9, TLR-8, TLR-3 and TLR-7 agonists. However agonists of other TLR receptors may be used to perform the invention, such as agonists of the TLR2, TLR4, TLR5 receptors.

The TLR agonist used in the invention can be a natural or a synthetic agonist. It can be a combination of different agonists of the same or of different toll-like receptors.

According to a particular embodiment of the invention, the TLR agonist is an immunostimulatory nucleotide sequence, especially a stabilized nucleotide sequence, for example stabilized as a result of structure modification such as phosphorothioate modification. The nucleotide sequence can also be protected against degradation by specific formulation. Especially liposome formulation thereof, e.g. liposome suspension, can be advantageous for the efficient administration of the immunostimulatory nucleotide sequence.

In a particular embodiment of the invention, the immunostimulatory nucleic acid sequence is a single-stranded RNA.

In a particular embodiment of the invention, the immunostimulatory nucleotide sequence comprises a CpG motif and especially is a CpG oligonucleotide (CpG ODNs). As an example of suitable CpG oligonucleotides the invention provides TLR-9 ligands such as Type A CpG ODN, i.e., CpG 2216 having nucleotide sequence 5'-GGGGGACGATCGTCGGGGGG-3' or Type B CpG ODN, i.e., CpG 1826 having nucleotide sequence 5'-TCCATGACGTTCCTGACGTT-3'.

CpG oligonucleotide can be used after being complexed with DOTAP (Roche Manheim, Germany), in order to protect it against degradation and to facilitate its uptake.

According to another particular embodiment of the invention, the TLR agonist is a small molecule. Small molecules suitable as TLR agonists are for example imidazoquinoline amine derivatives, such as the one named R848 (resiquimod), i.e., 4-amino-2-ethoxymethyl-a,a, dimethyl-1-H-imidazo[4,5c]quinoline -1-ethanol available from Invivogen, as TLR-7 ligand, or the one named R837 (imiqimod) available from Aldara as TLR-7 agonist.

Other molecules suitable as TLR agonists are polyuridine (pU) as TLR-3 ligand, or polycytidylic acid (PIC) as TLR-7 ligand.

These molecules can be formulated to facilitate their uptake and/or to protect them from degradation. These molecules can also be prepared as a liposome formulation, especially as a liposome suspension, for administration to a patient.

According to another particular embodiment of the invention, the adjuvant component can be a cell-based adjuvant component. An example thereof is dendritic cells that are known to be able to prime lymphocyte response, such dendritic cells being possibly conditioned *ex vivo* prior to their administration, in order to increase their activity of stimulation of the T cell response. Dendritic cells can hence be stimulated with adjuvants interacting with the PRRs, including TLR ligands or agonists (Pashine A. et al Nature Medicine Supplement Volume 11, N°4, April 2005 p S63-S68)

Alternatively, the polypeptide or polypeptide derivative according to the invention can be administered to the patient without an adjuvant.

Indeed the inventors have previously shown that CTL specific for the vectorized antigen can be primed *in vivo* after a single intravenous injection of the recombinant toxin, especially with no need to provide an heterologous adjuvant. These results and in particular the specific targeting of the epitope to myeloid dendritic cells enable to bypass the requirement for adjuvant and CD4+ T cell help.

Therefore, the invention also relates to the use of a mutant CyaA polypeptide recombined with a molecule and especially a peptide of interest for the preparation of a composition formulated for intravenous administration and enabling a CD8+ T cell immune response *in vivo,* said composition being free of a heterologous adjuvant. The invention also concerns this composition as such.

The disclosure describes therapeutic methods comprising administration of the mutant polypeptide or polypeptide derivative according to the invention to an animal or human patient suffering from a disease selected from neoplasia, cancers and infectious diseases selected from viral-, retroviral-, bacterial- or fungal-induced diseases.

The mutant polypeptide or polypeptide derivative can in particular be administered with a therapeutically active molecule and/or an adjuvant.

The mutant CyaA polypeptide or the polypeptide derivative, the therapeutically active molecule and/or an adjuvant can be administered together as part of a pharmaceutical composition which further comprises a pharmaceutically acceptable carrier or excipient(s).

Alternatively, the various types of molecules described herein to carry out the invention used, can be administered separately either simultaneously in time (especially for the mutant CyaA polypeptide or the polypeptide derivative and the adjuvant) or separately in time (especially for the mutant CyaA polypeptide).

The administration of the therapeutically active molecule can alternatively be carried out prior and after the administration of the mutant CyaA polypeptide or the polypeptide derivative and/or the adjuvant. It can also be sequential in time.

A particular regimen that may be adopted is a repeated administration protocol, especially in a protocol which encompasses two rounds or more of administration of at least one of the compounds selected from the mutant CyaA polypeptide or the polypeptide derivative, the therapeutically active agent and/or the adjuvant.

The invention is also directed to a pharmaceutical composition which comprises a mutant CyaA polypeptide or a polypeptide derivative according to the invention, a pharmaceutically acceptable carrier or excipient(s), and optionally an adjuvant and/or another therapeutically active molecule.

The disclosure describes a kit of parts comprising the mutant CyaA polypeptide or the polypeptide derivative, a therapeutically active molecule and/or an adjuvant.

The compounds of the kit of parts or the composition of the invention can especially be given to the patient through intravenous administration, intratumoral administration or subcutaneous administration.

The kit of parts or the composition has the ability to target (i) the adaptive immune response, through the mutant CyaA polypeptide or the polypeptide derivative disclosed in the present application, (ii) to downregulate the regulatory immune response through the therapeutically active agent, and if the adjuvant is present, to target (iii) the innate component of the immune response, by activating said innate response through the adjuvant.

The disclosure describes a method of treatment of a patient in need thereof, either a human or an animal patient, comprising the step of administering the components of the kit of parts or of the composition herein disclosed.

The invention in particular also relates to a new immunogenic composition formulated for administration, especially intravenous administration, in an animal or human host, characterized in that it comprises a recombinant CyaA polypeptide derivative which comprises an antigen inserted in the catalytic domain.

The invention further relates to a pharmaceutical composition for administration in a human or an animal formulated for targeting a molecule of interest specifically to CD11b expressing cells characterized in that said molecule of interest is coupled to a mutant CyaA polypeptide as described herein.

In another specific embodiment, the pharmaceutical or immunogenic composition comprises a nucleic acid construction encoding the recombinant CyaA polypeptide derivative comprising a CyaA mutant polypeptide as defined herein coupled to a molecule of interest.

Furthermore, the invention also relates to the use of the immunogenic composition as defined above for the preparation of a vaccine or an immunotherapeutic composition, for administration to an animal or human host.

As used herein, the term "immunotherapeutic composition" relates to a composition which leads to an immunological response and which is associated to therapeutic treatments, such as treatment against neoplasia, cancers, viral infections, fungal infections, parasites infections or bacterial infections.

The disclosure describes a method to immunize an animal or human host, wherein said method comprises the steps of:
a) providing an immunogenic composition as defined above;
b) administering said immunogenic composition, preferably via intravenous route, to said host in order to promote an immune response.

In particular, the immunogenic compositions of the invention are capable of inducing or stimulating, *in vivo* or *in vitro* an immune cell response involving specifically dendritic cells. The immunogenic compositions of the invention can in particular be used for preventive or therapeutic vaccination of a patient.

As a consequence, in a specific embodiment, the immunogenic or pharmaceutical composition is advantageously devoid of priming adjuvants commonly used in the Art, such as aluminium hydroxide.

The invention further relates to a method for the preparation of a proteinaceous vector suitable for the delivery of a molecule of interest into a cell comprising binding the molecule of interest to a CyaA mutant polypeptide as defined herein.

The disclosure describes nucleic acid molecules, in particular DNA or RNA molecules, which encode a polypeptide or polypeptide derivative as defined above.

The disclosure describes eukaryotic or prokaryotic cells which comprise the nucleic acid molecules as defined above.

The disclosure describes eukaryotic cells, preferably mammalian cells, which comprise a mutant CyaA polypeptide or polypeptide derivative as defined above. As described herein, the cells are human cells.

The disclosure describes eukaryotic cells, preferably mammalian cells, transformed with the proteinaceous vector as defined above.

### Figures

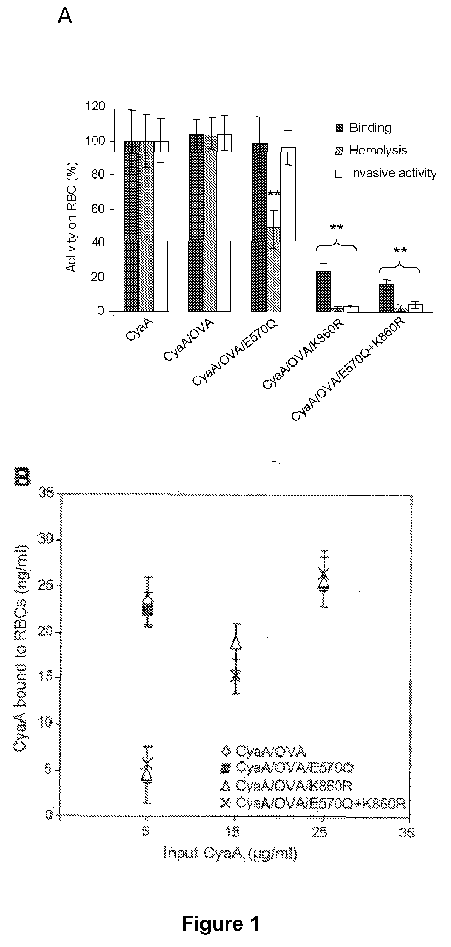

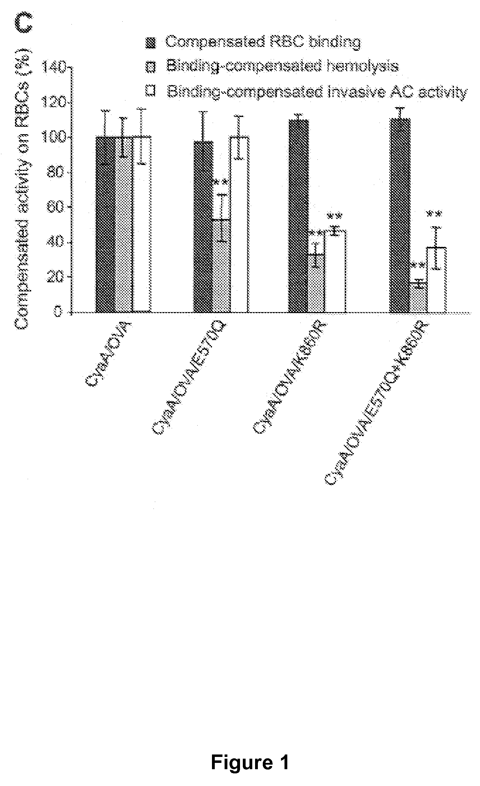

**Fig. 1****. Substitutions in the pore-forming and acylation domains synergize in decreasing the specific hemolytic activity of CyaA. (A)** Sheep erythrocytes (5x10⁸/ml) in TNC buffer were incubated with 5 µg/ml of enzymatically active CyaA proteins at 37°C. After 30 min, aliquots of cells suspensions were washed repeatedly to remove unbound CyaA and used to determine the amount of cell-associated and cell-invasive AC activity. Hemolytic activity was measured after 5 hours of incubation as the amount of released hemoglobin by photometric determination (A₅₄₁). Activity of intact CyaA was taken as 100%. (B) Erythrocytes were incubated as above with the indicated concentrations of the enzymatically active CyaA-derived proteins for 30 min, washed, and the amount of cell-associated AC enzyme activity was determined. **(C)** The reduced cell binding activity of proteins with the K860R substitution was compensated for by increasing their concentration from 5 µg/ml to 25 µg/ml. Activities of CyaA/233OVA (CyaA/OVA) in the presence were taken as 100% value. The results represent average values from at least three independent experiments performed in duplicates. The asterisks indicate statistically significant differences (**, *p<* 0.001) from activities of CyaA (Fig. 1A) or CyaA/OVA (Fig. 1C).
**Fig. 2****. CyaA/OVA/E570Q+K860R binds and translocates into CD11b**⁺ **monocytes. (A)** J774A.1 cells (10⁶/ml) were incubated in D-MEM for 30 min at 4°C with 2.5 µg/ml of CyaA, washed repeatedly, and the amount of cell-associated AC activity was determined in cell lyzates. To block the CD11b/CD18 receptor, cells were incubated for 30 min with the CD11b-specific antibody M1/70 (Exbio, Czech Republic) at a final concentration of 10 µg/ml prior to addition of CyaA (**, *p<* 0.001). **(B)** The AC domain translocation capacity of constructs was assessed as the capacity to penetrate cells and convert cytosolic ATP to cAMP. J774A.1 cells were incubated with CyaA constructs for 30 minutes at 37°C and the amounts of cAMP accumulated in cell lyzates were determined (41). As a control, the CD11b/CD18 receptor was blocked with the anti-CD11 b antibody M1/70 as above. Membrane penetration of CD11b/CD18 -bound and endocytosed toxin was controlled by using the doubly mutated CyaA/E570K+E581 P variant, which is intact for receptor binding but fails to translocate the AC domain across cell membrane and elevate cytosolic cAMP concentrations (Vojtova-Vodolanova *et al.,* 2009). **(C)** J774A.1 cells were loaded with the K⁺ sensitive fluorescent probe PBFI/AM at 9.5 µM final external concentration and 25°C for 45 min in the presence of Pluronic F-127 [0.05% (w/w)]. Cells were washed in HBSS before 3 µg ml⁻¹ of the indicated toxins were added. Fluorescence intensity ratio of PBFI (excitation wavelength 340, emission wavelengths 450 and 510 nm) reflecting the intracellular K⁺ concentration was recorded every 15 s. The right scale shows intracellular [K⁺] values derived from calibration experiments (see *Experimental procedures*). No cell lysis, assessed as lactate dehydrogenase release, was observed within the time interval of the assay. Results representative of three independent determinations performed in duplicates are shown.

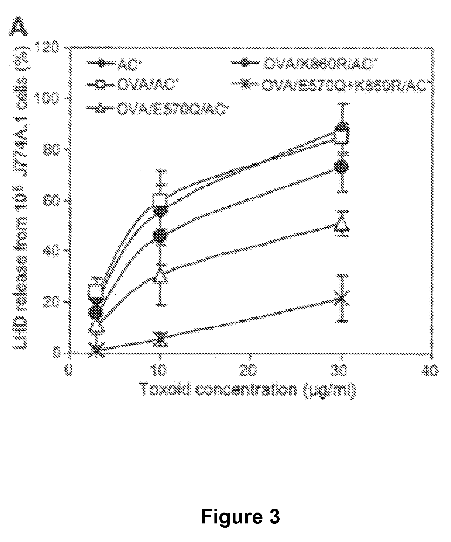

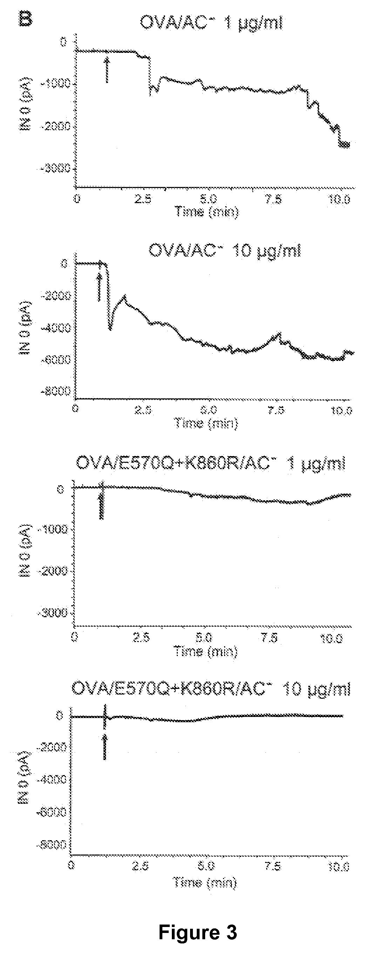

**Fig. 3****. E570Q**+**K860R toxoid does not permeabilize J774A.1 cells. (A)** Lysis of J774A.1 cells was determined as the amount of lactate dehydrogenase released from 10⁵ cells upon 3 h of incubation with 3, 10 and 30 µg ml⁻¹ of the indicated protein at 37% in DMEM without serum. The results represent the average of values obtained in two experiments performed in duplicates. **(B)** Whole-cell patch-clamp measurements were performed on single J774A.1 cells at room temperature exposed to 1 or 10 µg/ ml of CyaA/233OVA/AC⁻ or CyaA/233OVA/E570Q+K860R/AC⁻ proteins as described in Materials and Methods. The shown curves are representative of six determinations in 3 independent experiments.

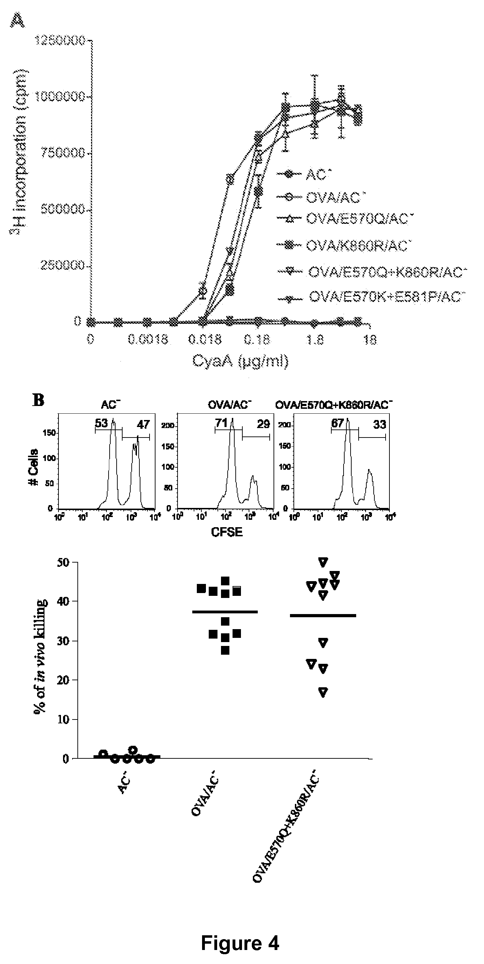

**Fig. 4****. Toxoid with E570Q+K860R substitutions delivers the OVA T-cell epitope for presentation by MHC class I molecules and induction of CD8⁺ CTLs**. **(A)** BMDC (3 x 10⁵ cells/well) used as APCs were incubated in the presence of indicated concentrations (0 to 60 nM) of the toxoids harboring the OVA epitope or with mock CyaA/AC⁻. Upon co-culture for 24 hours with B3Z T cells (1 x 10⁵ cells/well), IL-2 secretion by the stimulated B3Z cells was determined by the CTLL proliferation method. Results are expressed as Δcpm of incorporated [³H]thymidine (cpm in the presence of toxoid - cpm in the absence of toxoid) ±SD and are representative of five independent experiments. **(B)** Analysis of the induction of OVA (SIINFEKL)-specific CTL responses by in vivo killing assay. On day 0, mice received 50 µg i.v. of mock AC- or OVA/AC- toxoids and on day 7, they were i.v. injected with a mixture (1:1) of OVA (SIINFEKL) peptide-loaded CFSE^{high} and unloaded CFSE^{low} splenocytes. The number of CFSE-positive cells remaining in the spleen after 20 h was determined by FACS analysis, as documented for one representative *in vivo* killing assay in the upper panel assembly of plots, where percentages of cells in the gates are indicated. The lower panel shows pooled results of *in vivo* killing assays for three independent experiments. Statistical significance was determined by the Student t test (p= 0.75 for OVA/AC⁻ vs. OVA/E570Q+K860R/AC⁻).
**Fig. 5****. Model of CyaA action on the membrane. (A)** The model predicts an equilibrium between two conformers of CyaA in solution, each of them inserting into cell membrane in different a conformation. One would yield a monomeric CyaA translocation precursor, delivery of the AC domain into cytosol and concomitant influx of calcium ions into cells. The conformer would insert as pore precursor oligomerizing into a CyaA pore. **(B)** The synergic effect of the E570Q and K860R substitutions would selectively block the capacity of CyaA pore precursors to oligomerize into a pore, while the capacity of translocation precursors to deliver the AC domain across membrane would remain unaffected.
Fig. 6. Amino acid sequence of the *Bordetella pertussis* CyaA toxin (SEQ ID N°1)
Fig. 7. Amino acid sequence of the *Bordetella pertussis* CyaA/E570Q+K860R mutant (SEQ ID N°2)

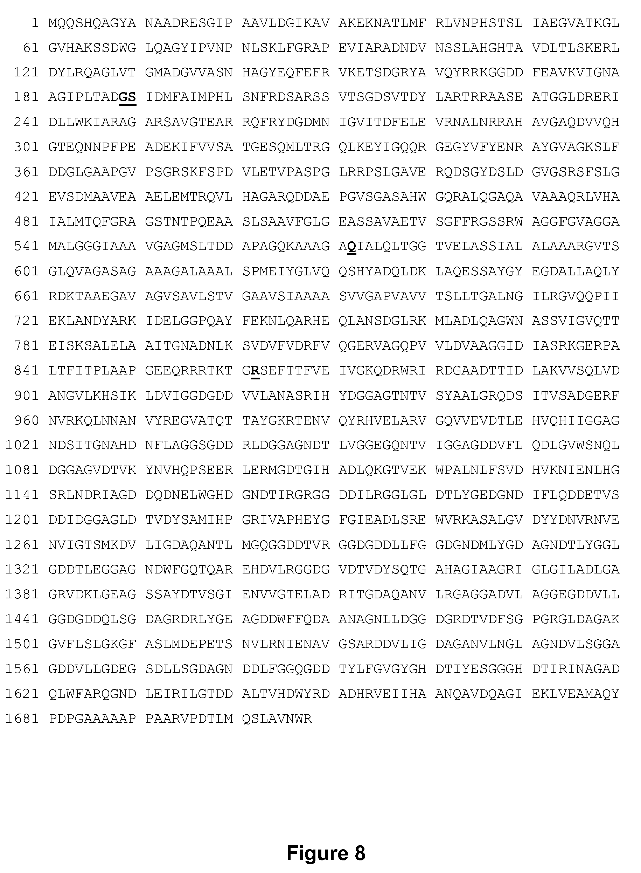

Fig. 8. Amino acid sequence of the *Bordetella pertussis* CyaA/E570Q+K860R/AC⁻ mutant (SEQ ID N° 3)

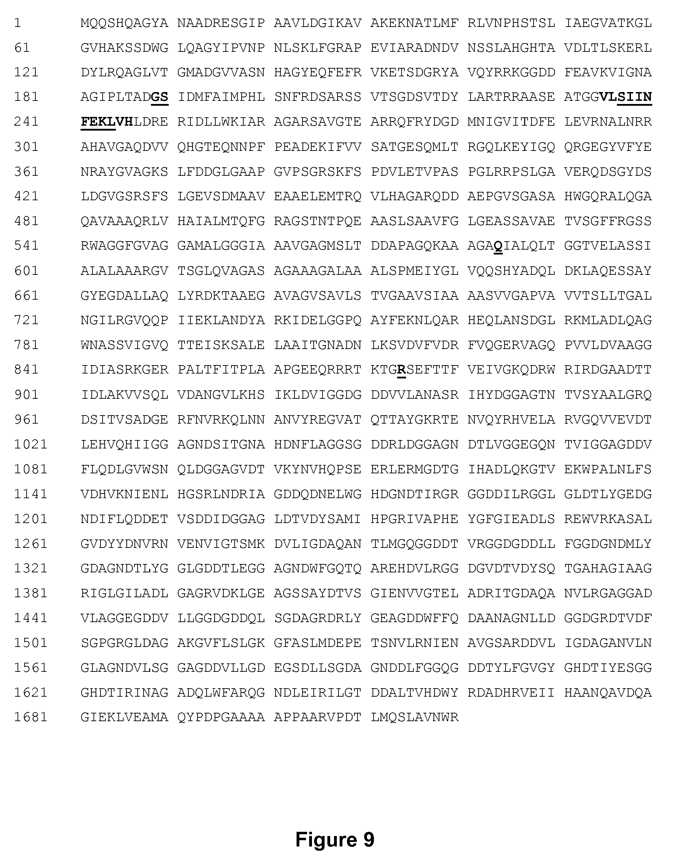

Fig. 9. Amino acid sequence of the *Bordetella pertussis* CyaA/233OVA/E570Q+K860R/AC⁻ mutant (SEQ ID N° 4)
Fig. 10. Plasmid encoding the CyaA/E570Q+K860R/AC⁻ mutant (QR-AC⁻).
Fig. 11. DNA sequence of the QR-AC- plasmid encoding the CyaA/E570Q+K860R/AC⁻ mutant (SEQ ID N° 5)
Fig. 12. Plasmid encoding the CyaA/233OVA/E570Q+K860R/AC⁻ mutant (OVA-QR-AC⁻).
Fig. 13. DNA sequence of OVA-QR-AC- plasmid encoding the CyaA/233OVA/E570Q+K860R/AC⁻ mutant (SEQ ID N° 6)
Fig. 14. Amino acid sequence of the *Bordetella parapertussis* CyaA toxin (accession number CAB76450, SEQ ID N°7)
Fig. 15. Amino acid sequence of the *Bordetella hinzii* CyaA toxin (accession number AAY57201, SEQ ID N°8)
Fig. 16. Amino acid sequence of the *Bordetella bronchiseptica* CyaA toxin (accession number CAA85481, SEQ ID N°9)

### Examples

### Adenylate Cyclase Toxin Translocates Across Target Cell Membrane Without Forming a Pore

### Materials and Methods

**Construction, Production and Purification of CyaA proteins.** The modifications yielding CyaA/AC⁻, CyaA/233OVA, CyaA/E570Q and CyaA/K860R constructs were previously described (13, 20, 21) and were introduced into CyaA/233OVA/AC⁻ individually or in combination. The CyaA-derived proteins were produced in *E. coli* XL-1 Blue and purified close to homogeneity as previously described (29). During the hydrophobic chromatography, the resin with bound toxin was repeatedly washed with 60% isopropanol (30) to reduce the endotoxin content of CyaA samples below 100 IU/mg of protein, as determined by the LAL assay QCL-1000 (Cambrex).

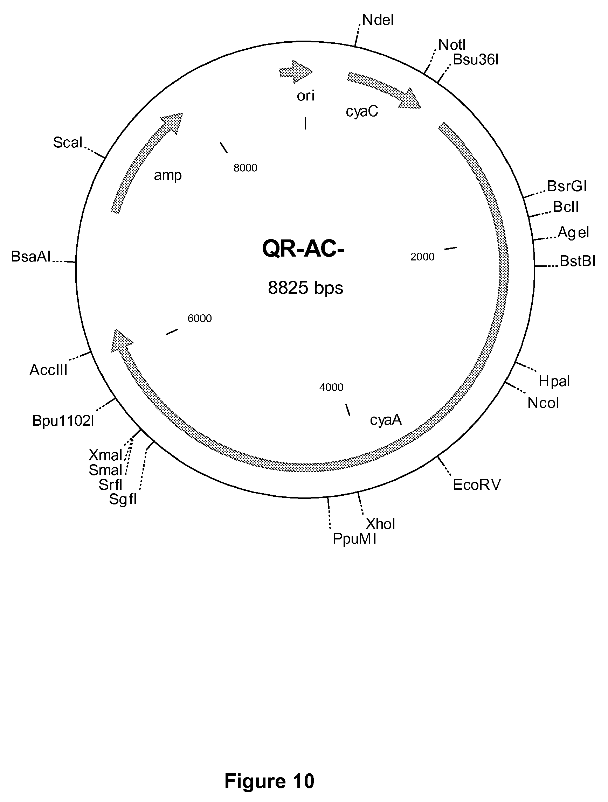

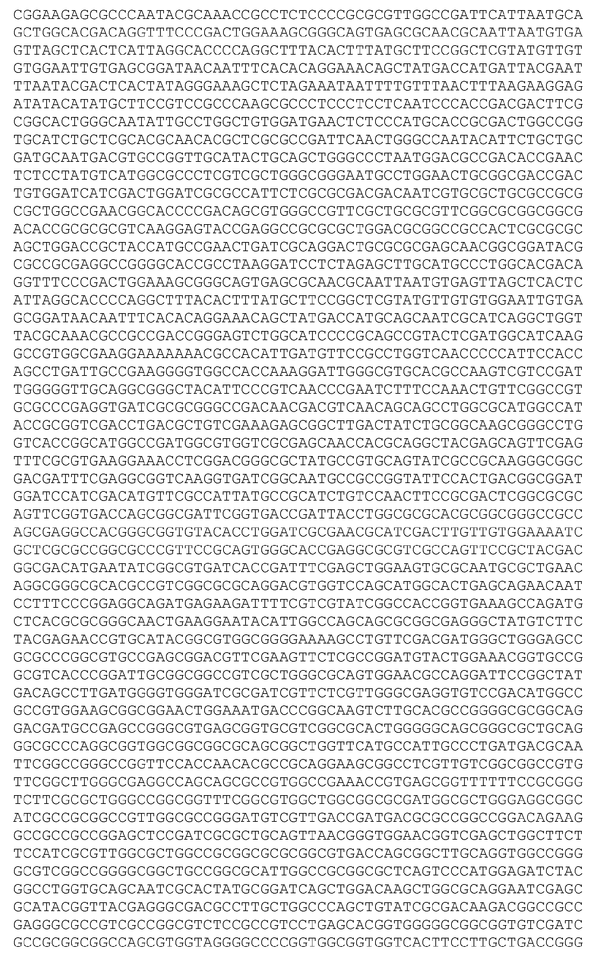

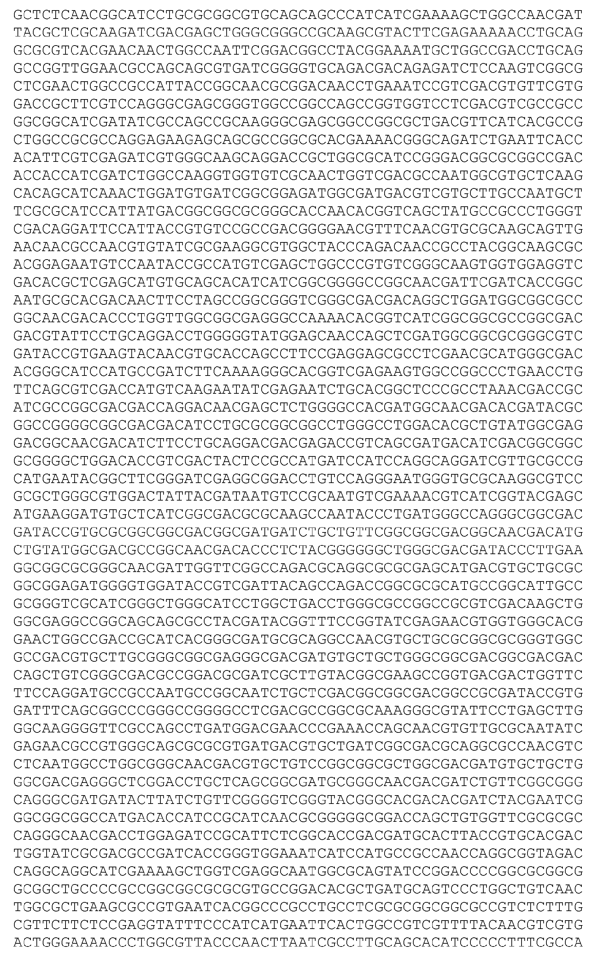

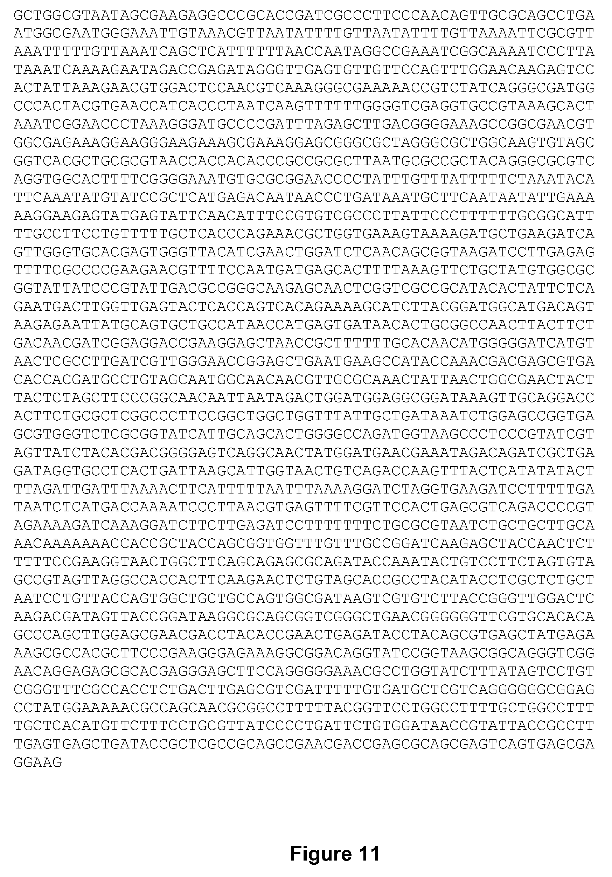

An *Escherichia coli* XL1-Blue strain (Stratagene) containing the QR-AC-plasmid (figure 10) which encodes the CyaA/E570Q+K860R/AC⁻ mutant was deposited on March 18, 2009 at the CNCM (Collection Nationale de Cultures de Microorganismes, France) under the accession number CNCM I-4136 (figure 10). The DNA sequence of the QR-AC- plasmid (SEQ ID N°5) is disclosed in figure 11.

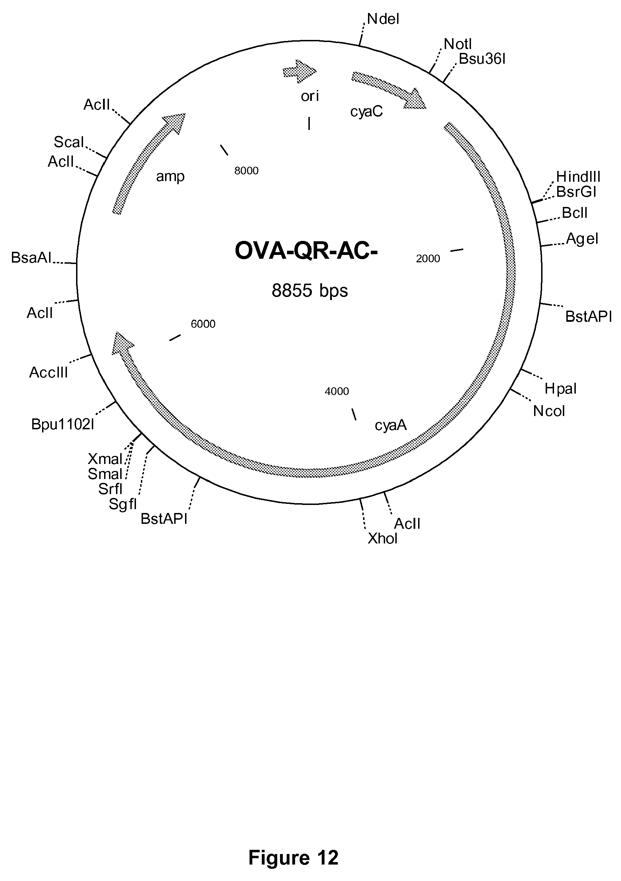

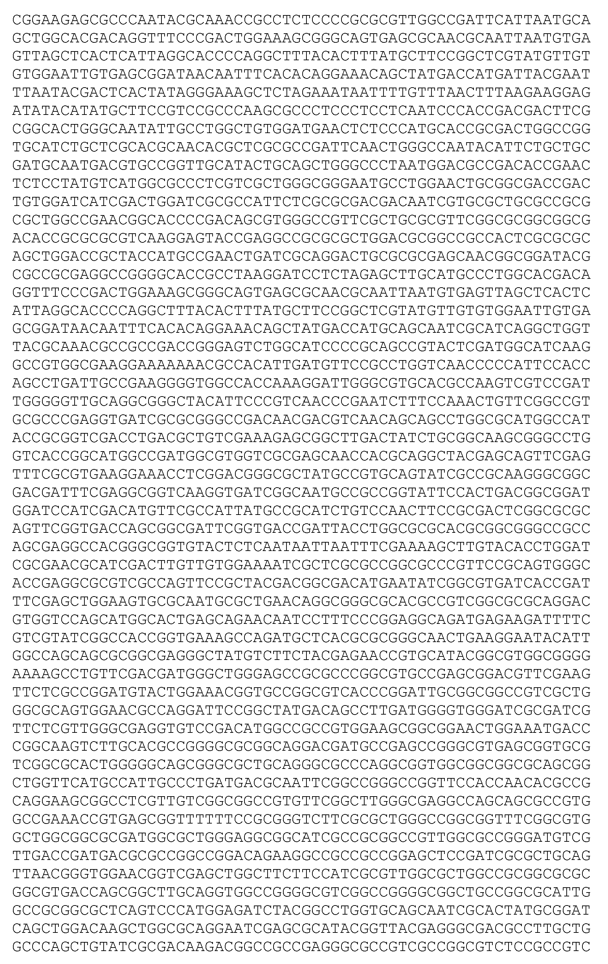

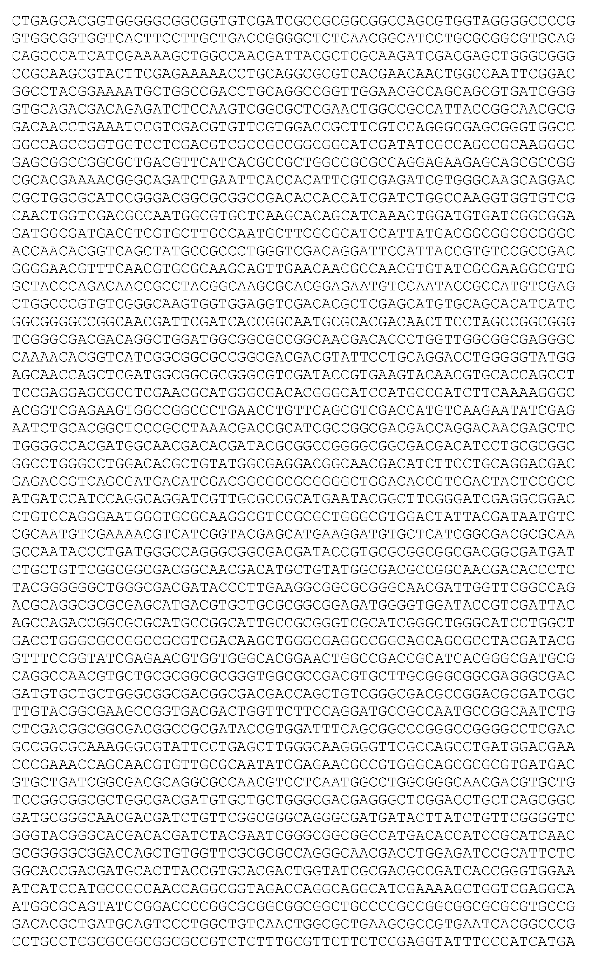

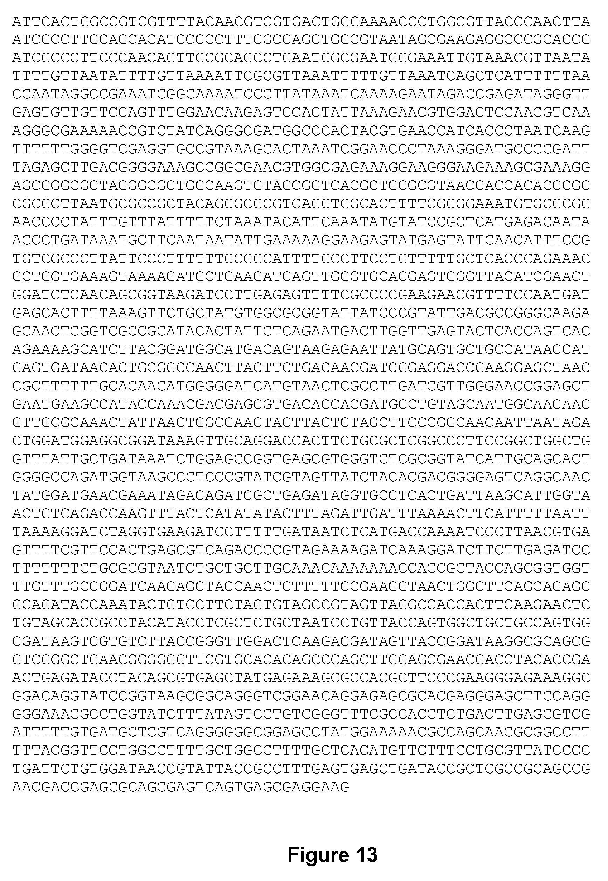

An *Escherichia coli* XL1-Blue strain (Stratagene) containing the OVA-QR-AC- plasmid (figure 12) which encodes the CyaA/233OVA/E570Q+K860R/AC⁻mutant was deposited on March 18, 2009 at the CNCM (Collection Nationale de Cultures de Microorganismes, France) under the accession number CNCM I-4137. The DNA sequence of the OVA-QR-AC- plasmid (SEQ ID N° 6) is disclosed in figure 13.

**Cell Binding and Hemolytic Activities on Sheep Erythrocytes.** 5x10⁸ washed sheep erythrocytes in 50 mM Tris pH 7.4, 150 mM NaCl and 2 mM CaCl₂ (TNC buffer) were incubated at 37°C with 5 µg/ml of CyaA proteins and cell binding, cell-invasive AC and hemolytic activities of CyaA were determined as described in detail previously (13). Significance of differences in activity values was analyzed using a one-way analysis of variance (ANOVA) with Bonferroni post-test (SigmaStat v. 3.11, Systat, San Jose, CA).

**Macrophage Binding, Elevation of cAMP and Cell Lysis Capacities of CyaA.** J774.Al murine monocytes/macrophages (ATCC, number TIB-67) were cultured at 37°C in a humidified air/CP₂ (19:1) atmosphere in RPMI medium supplemented with 10% (v/v) heat-inactivated fetal bovine serum, penicillin (100 IU ml⁻¹), streptomycin (100 µg ml⁻¹) and amphotericin B (250 ng ml⁻¹). Prior to assays, RPMI was replaced with Dulbecco's modified Eagle's medium (DMEM) (1.9 mM Ca2⁺) without FCS and the cells were allowed to rest in DMEM for 1 h at 37°C in a humidified 5% CO₂ atmosphere (8). J774A.1 macrophages (10⁶) were incubated in D-MEM with 2.5 µg/ml of CyaA variants for 30 min at 4°C, prior to removal of unbound toxin by three washes in D-MEM. Cells were lyzed with 0.1% Triton X-100 for determination of cell-bound AC activity. For intracellular cAMP assays, 10⁵ cells were incubated with CyaA for 30 minutes in D-MEM with 100 µM IBMX (3-isobutyl-1-methylxanthin), the reaction was stopped by addition of 0.2% Tween-20 in 100 mM HCl, samples were boiled for 15 min at 100°C, neutralized by addition of 150 mM unbuffered imidazol and cAMP was measured as described (29). To block the CD11b/CD18 receptor, cells were preincubated for 30 min on ice with the CD11b-specific blocking MAb M1/70 (Exbio, Czech Republic) at a final concentration of 10 µg/ml prior to addition of CyaA. Toxin-induced lysis of J774A.1 cells was determined using the CytoTox 96 kit assay (Promega) as the amount of lactate dehydrogenase released from 10⁵ cells in 3 hours of incubation with 10 µg/ml of the appropriate protein at 37°C in D-MEM as described (8). Significance of differences in activity values was analyzed as above.

**Patch Clamp Measurements.** Whole-cell patch-clamp measurements were performed on J774A.1 cells bathing in HBSS (140 mM NaCl, 5 mM KCI, 2 mM CaCl₂, 3 mM MgCl₂, 10 mM Hepes-Na, 50 mM glucose; pH 7.4). Fire-polished glass micropipettes with outer diameter of about 3 µm were filled with a solution of 125 mM potassium gluconate, 15 mM KCI, 0.5 mM CaCl₂, 1 mM MgCl₂, 5 mM EGTA, 10 mM HEPES-KOH pH 7.2. The resulting resistances of the microelectrodes were 3 to 5 MΩ. Cells were clamped at -40 mV, the data were filtered at 1 kHz and digitized at 2 kHz using Axopatch 200A amplifier, Digidata 1320A digitizer and PClamp-9 software package (Axon Instruments, Foster City, CA).

**Determination of decrease of cytosolic K⁺ concentration.** Cells grown on grass coverslips were washed in HBSS and loaded with 9.5 µM PFBI acetoxymethyl ester (PBFI/AM, Molecular Probes, Eugene, OR, USA) for 30 min at 25°C in the presence of 0.05% (w/w) Pluronic F-127 (Sigma, St.Louis, MO), in the dark. Ratiometric measurement was performed at 25°C using a FluoroMax-3 spectrofluorimeter (Jobin Yvon Horriba, France) equipped with DataMax software. Fluorescence intensity of PBFI (excitation wavelength 340, emission wavelengths 450 and 510 nm) was recorded every 15 s and the integration time for each wavelength was 3 s. The ratio of 450/510 nm wavelengths is shown. The observed area of coverslip mounted in the 1 cm cuvette was about 10 mm², corresponding to approximately 10⁴ cells. Calibration experiments were performed in 50 mM HEPES, pH 7.2, with varying concentrations of Potassium Acetate (10, 30, 60 or 140 mM) and Sodium Acetate (135, 115, 85 or 5 mM), respectively, on cells permeabilized for 30 min with 3 pM valinomycin or nigericin. Final intensity ratio (450/510 nm) is shown on right vertical axis of the plots.

**Mice and Cell Lines.** Female C57BL/6 obtained from Charles River Laboratories were kept under specific pathogen-free conditions and manipulated according to institutional guidelines. CTLL-2 cells were obtained from ATCC. B3Z, a CD8⁺ specific T cell hybridoma specific for the K^{b} restricted OVA (SIINFEKL) epitope, was provided by N. Shastri (University of California, Berkeley) and maintained in the presence of 1 mg/ml G418 and 400 µg/ml hygromycin B in complete RPMI 1640 medium (Invitrogen Life Technologies) with 10% heat-inactivated FCS, 100 U/ml penicillin, 100 µg/ml streptomycin, and 5x 10⁻⁵ M 2-ME.

**Antigen Presentation Studies.** Bone Marrow Dendritic Cells (BMDC, 3 x 10⁵ per well) used as APCs were incubated in the presence of various concentrations (0 to 60 nM) of the recombinant CyaA/OVA/AC⁻ carrying the OVA (SIINFEKL) epitope or mock CyaA/AC⁻ and cocultured for 24 hours with B3Z T cells (1 x 10⁵ per well), selectively recognizing the OVA SIINFEKL/H-2K^{b} MHC class I complexes. After 18 h of culture, supernatants were frozen for at least 2 h at -80°C. The amount of IL-2 produced by the stimulated B3Z cells was then determined by the CTLL proliferation method. Briefly, 10⁴ cells of the IL-2-dependent CTLL line per well were cultured with 100 µl of supernatant in 200 µl of final volume. Twenty-four hours later, [³H]-thymidine (50 µCi/well) was added and cells were harvested 6 h later with an automated cell harvester. Incorporated [³H]-thymidine was detected by scintillation counting. Each point was done in duplicate and the experiment was repeated five times. Results are expressed in Δcpm of incorporated [³H]-thymidine (cpm in the presence of toxoid - cpm in the absence of toxoid).

***In vivo* Killing Assay.** For CTL priming, mice were immunized by i.v. injection with 50 µg of recombinant CyaA/OVA/AC⁻ carrying the OVA (SIINFEKL) epitope or mock CyaA/AC⁻. Seven days after immunization, naive syngenic splenocytes were pulsed with OVA (SIINFEKL) peptide (10 µg/ml) (30 min, 37°C), washed extensively and labeled with a high concentration (1.25 µM) of carboxyfluoroscein succinimidyl ester (CFSE; Molecular Probes, Eugene, OR). The nonpulsed control population was labeled with a low concentration (0.125 µM) of CFSE. CFSE^{high}-and CFSE^{low}-labeled cells were mixed in a 1:1 ratio (5x 10⁶ cells of each population) and injected i.v. into mice. Spleen cells were collected 20 h after, washed and resuspended in FACS buffer (PBS supplemented with 1% BSA and 0,1% NaN₃). The number of CFSE-positive cells remaining in the spleen after 20 h was determined by FACS. The percentage of specific lysis was calculated as follows: percent specific lysis = 100 - [100 x (% CFSE^{high} immunized mice/% CFSE^{low} immunized mice)/(% CFSE^{high} naive mouse/% CFSE^{low} naive mouse)].

***Statistical Analysis:*** Significance of differences in values was analyzed using a one-way analysis of variance (ANOVA) with Bonferroni post-test (SigmaStat v. 3.11, Systat, San Jose, CA).

### Results

**Combined Elimination of Negatively Charged Glutamate 570 and of Acylated Lysine 860 Ablates Cell-permeabilizing Capacity of CyaA.** The working model of CyaA action predicts that CyaA can be modified to lose its pore-forming (hemolytic) activity while preserving the capacity to deliver the AC domain into cytosol of target cells. To test this hypothesis, the inventors sought to produce CyaA constructs exhibiting as low hemolytic and cytolytic activities as possible, building on previous observation that the capacity of CyaA/AC⁻ toxoids to lyze cells can be modulated both up or down by substitutions within the pore-forming domain (8, 12-14, 18). To enable assessment of target cell penetration also for the CyaA/AC⁻ toxoids, the inventors derived such mutants from a CyaA/233OVA toxin that was previously tagged by insertion of the SIINFEKL peptide from ovalbumin (OVA). This CyaA variant was chosen as the insertion of reporter K^{b}-restricted CD8⁺ T-cell epitope at residue 233 does not affect the AC activity and allows to quantify translocation of the OVA/AC enzyme into cells as elevation of cytosolic cAMP. More importantly, presence of the OVA epitope allows to assess also the capacity of enzymatically inactive CyaA/233OVA/AC⁻ toxoids to deliver their OVA/AC⁻ domain into cytosol of CD11b⁺ antigen presenting cells (APC), as this enables proteasome processing and cell surface presentation of the OVA epitope on MHC Class I glycoproteins that can be determined as stimulation of OVA-specific CD8⁺ T cells, both *in vitro* and *in vivo* (20).

To generate CyaA/AC⁻ toxoids possibly lacking the cytolytic activity, the inventors combined the E570Q and K860R substitutions previously shown to reduce the specific hemolytic activity of CyaA on sheep erythrocytes, with the E570Q substitution having been found to reduce also the cytolytic activity of the CyaA/AC⁻ on CD11b⁺ J774A.1 monocytes (8, 13). These substitutions were engineered into CyaA/2330VA/AC⁻ individually and in combination and the specific hemolytic and cytolytic activities of resulting toxoids were compared using sheep erythrocytes as model CD11b⁻ target and J774A.1 as model CD11b⁺ target in parallel (Table I). In agreement with results obtained previously with toxoids lacking the OVA epitope (4, 8, 13, 21), under the used conditions the OVA/AC⁻toxoids carrying individually the E570Q and K860R substitutions exhibited respectively a two-fold reduced (55 ± 8) and nil (1 ± 1) relative hemolytic activity on erythrocytes and the relative cytolytic activity of the E570Q toxoid towards CD11b-expressing J774A.1 cells was also reduced (37 ± 10), as compared to OVA/AC⁻. In turn, as expected from results obtained with an enzymatically active K860R construct, despite the low hemolytic activity on CD11b⁻ erythrocytes, the K860R toxoid exhibited only a slightly reduced relative cytolytic activity on CD11b⁺ J774A.1 cells (72 ± 22%), confirming that the structural defect caused by the K860R substitution was rescued by interaction with the CD11b/CD18 receptor (4). Nevertheless, when combined with E570Q, the K860R substitution exhibited a clear synergic effect in reducing the relative cytolytic activity of the E570Q+K860R construct towards J774A.1 cells down to 14 ± 7%.

**Table I : Cytolytic activities of OVA/AC⁻ and derivatives on sheep erythrocytes and J774A.1 macrophages.**

| Protein | Lysis of erythrocytes (% of AC⁻)*^{a}* | Lysis of J774A.1 cells (% of AC⁻)*^{b}* |
|---|---|---|
| AC⁻ | 100±5 | 100 ± 10 |
| OVA/AC⁻ | 93 ± 4 | 93 ± 12 |
| OVA/E570Q/AC⁻ | 55 ± 8** | 37 ± 10** |
| OVA/K860R/AC⁻ | 1 ± 1** | 72 ± 22** |
| OVA-L247Q-AC⁻ | 97 ± 3 | 41 ± 9 |
| OVA/E570Q+K860R/AC⁻ | 1 ± 1** | 14 ± 7** |
| OVA-E570Q-L247Q-AC⁻ | 50 ± 12 | 40±11 |
| OVA-K860R-L247Q-AC⁻ | 1 ± 1 | 45 ± 11 |
| OVA-E570Q-K860R-L247Q-AC⁻ | 0 ± 1 | 16 ± 10 |

### Table Legend

- *^{a}*Lysis of sheep erythrocytes was determined after 4.5 hours as the amount of hemoglobin released upon incubation of 5x10⁸ RBC at 37°C in the presence of 2 mM Ca²⁺ with 5 µg/ml of the given protein (31). The hemolytic activity of CyaA/AC⁻ was taken as 100% activity. The results represent the average of values obtained in four independent experiments performed in duplicates ± S.D with two different protein preparations.
- *^{b}*Lysis of J774A.1 cells was determined as the amount of released lactate dehydrogenase from 10⁵ cells upon 3 hours of cell incubation with 10 µg/ml of the appropriate protein at 37°C in D-MEM. J774A.1 cell lysis by CyaA/AC⁻ was taken as 100%. The results represent the average of values obtained in four separate experiments performed in duplicates ± S.D with two different protein preparations (*, *p* < 0.05; **, *p* < 0.001).

To enable quantification of capacity of the E570Q+K860R construct to deliver the AC domain into cytosol of cells, the E570Q and K860R substitutions were transferred into enzymatically active constructs derived from CyaA/233OVA (CyaA/OVA). These were produced and purified in the same way as the AC-toxoids (not shown) and characterized for cell binding, hemolytic and AC translocation capacities on sheep erythrocytes. As shown in Fig. 1A and expected from results with toxins lacking the OVA epitope (4, 13, 21), the E570Q substitution had no impact on erythrocyte binding or the capacity of CyaA/OVA to deliver the AC domain into erythrocyte cytosol and selectively reduced only its relative hemolytic activity. As further expected (4), the K860R substitution significantly reduced the capacity of CyaA/OVA to bind and penetrate erythrocytes, causing a sharp reduction of the relative hemolytic and cell-invasive AC activities of the E570Q and E570Q+K860R mutants on erythrocytes.

It has to be noted, that the hemolytic activity of CyaA is a highly cooperative function of the amount of cell-associated CyaA (Hill number >3), suggesting that CyaA oligomerization is a prerequisite for pore formation (22). Therefore, to assess the impact of combined E570Q+K860R substitutions on the hemolytic activity, the loss of erythrocyte-binding capacity of the K860R constructs had to be compensated by increasing their concentration in the assay to 25 µg/ml (5 µg/ml for intact toxin), in order to achieve binding of equal amounts of all proteins to erythrocytes, as shown in Fig. 1C. Under these conditions the combination of E570Q and K860R substitutions exhibited a clear synergy in further reducing by a factor of two the already impaired hemolytic activities of constructs carrying the E570Q (∼50%) and K860R substitutions (∼30%) individually, as shown in figure 2C. This suggests that combination of the two substitutions affected the specific cell-permeabilizing capacity of CyaA.

**Pore-forming Activity of CyaA is Dispensable for Membrane Translocation of the AC Domain.** In contrast to impact of the K860R substitution on toxin activity on erythrocytes, both the E570Q and K860R substitutions were previously found to have no effect on the capacity of CyaA to bind and penetrate J774A.1 monocytes expressing the CD11b/CD18 receptor (4, 8). Moreover, as documented in Fig. 2, when the two substitutions were combined in the same toxin molecule, the CyaA/OVA/E570Q+K860R construct exhibited an equal capacity to bind J774A.1 cells (Fig. 2A) and to deliver the AC domain into their cytosol to elevate cytosolic cAMP concentrations (Fig. 2B), as did intact CyaA. At the same time, however, the doubly mutated E570Q+K860R toxoid exhibited an about seven-fold reduced (14 ± 7%) relative cytolytic capacity on these cells (cf. Table I). As shown in Fig. 2C, when compared with intact CyaA, the singly mutated E570Q and the doubly mutated E570Q+K860R constructs were importantly impaired in the capacity to elicit decrease of intracellular concentration of potassium ions ([K⁺]*i*) in toxin-treated J774A.1 cells. While no cell lysis was detected over 20 min by the assay for release of lactate dehydrogenase, the [K⁺]*i* of J774A.1 cells exposed to 3 µg ml⁻¹ of intact CyaA decreased from 140 mM to well below 30 mM already in 10 min upon toxin addition. In turn, when the same amounts of the ER570QiK860R constructs were used (3 µg ml⁻¹), the [K⁺]*i* levels in cells did not decrease below 100 mM (Fig. 2C). Indeed, efflux of potassium from cells was previously shown to be the hallmark of insertion of the CyaA pore precursors into cell membrane (32). This suggested that the combination of E570Q and K860R substitutions selectively impaired only the capacity of the toxoid to permeabilize J774A.1 cells and not its capacity to translocate the AC domain across cell membrane.

This conclusion was further supported by an importantly reduced relative cytolytic activity of the corresponding E570Q/AC- and E570Q+K860R/AC- toxoids, as discussed above (Table I) and documented in detail in Fig. 3A. The doubly mutated E570Q+K860R toxoid at 3 µg ml⁻¹ was essentially unable to provoke any detectable release of lactate dehydrogenase from J774A.1 cells in 3 h of incubation, while 20% of cells lysed in the presence of equal amounts of intact toxoid.

To test this, the inventors analyzed the cell-permeabilizing capacity of the E570Q+K860R construct in single whole cell patch-clamp experiments. Here again the AC- toxoids had to be used, in order to avoid the massive ruffling of J774A.1 cells provoked by toxin-generated cAMP (23). As shown in Fig. 3A by a representative recording of ion currents across the membrane of patch-clamped single J774A.1 cells exposed to 1 µg/ ml of CyaA/OVA/AC⁻, upon an initial lag of about 3 minutes the J774A.1 cells were progressively and massively permeabilized by CyaA/OVA/AC⁻ and the currents across cell membrane reached - 3,000 pA within 10 minutes. In contrast, as shown in Fig. 3B, exposure to the CyaA/OVA/E570Q+K860R/AC⁻ reproducibly caused only a transient and minimal initial permeabilization of the cells, with currents across cell membrane not exceeding -200 pA and returning close to zero within 10 minutes after toxoid addition. Quite similar picture was observed when toxoid concentrations were elevated to 10 µg ml⁻¹, which was the concentration used for comparisons of relative cytolytic activity of toxoids summarized in Table I. The 10-fold increase of concentration from 1 to 10 µg ml⁻¹ resulted in about twofold increase of the currents produced across cell membrane by OVA/AC-, while essentially no enhancement of cell permeabilization was observed even at the increased concentration of OVA/E570Q+K860R/AC- (note the expanded scale of y-axis for measurements at 10 µg ml⁻¹). The shown recordings were representative of at least six determinations from 3 independent experiments and demonstrate that the combination of the E570Q and K860R substitutions had a major impact on the capacity of the toxoid to permeabilize the membrane of J774A.1 cells. Given that the enzymatically active version of the same construct was fully capable to translocate the AC domain into J774A.1 cells (cf. Fig. 2B), these results strongly suggest that the cell-permeabilizing (pore-forming) activity of CyaA was not required for AC domain translocation across cellular membrane.

**Membrane-permeabilizing Activity of CyaA is Dispensable for Delivery of Passenger Antigens to the Cytosolic MHC Class I Pathway.** Since the assay for cytosolic cAMP could not be used for assessment of cell penetration capacity of the AC- toxoids, the surrogate assay for their capacity to deliver the reporter OVA epitope to the cytosolic processing site of the MHC class I antigen presentation pathway was used (7, 24). Towards this end, the inventors determined the capacity of C57BL/6 mouse bone marrow-derived dendritic cells (BMDCs), loaded with the toxoids, to stimulate IL-2 release by B3Z T cells that selectively recognize the complex of K^{b} MHC class I molecules with the SIINFEKL (OVA) peptide on APCs. It has, indeed, been previously shown that the capacity of CyaA/AC- toxoids to translocate the AC domain across the cytoplasmic membrane into cytosol of BMDCs is essential for the capacity of the toxoids to promote presentation of the delivered OVA epitope in complex with the H-2Kb MHC class I molecules. This, in turn, is essential for specific *in vitro* stimulation of cytotoxic T cells to occur (29). Nevertheless, it was important to confirm here that delivery of the OVA epitope for proteasome processing and subsequent presentation was due to AC domain translocation into cytosol of BMDCs across their cytoplasmic membrane, and was not due to sampling of the added antigen by fluid phase uptake, or endocytosis. For this purpose, a doubly mutated non-translocating OVA/E570K+E581 P/AC- toxoid variant was used, which bears a combination of charge-reversing and a-helix-breaking substitutions of glutamates 570 and 581 in the transmembrane domain of CyaA (33). This construct was previously found to exhibit a full capacity to bind CD11b/CD18-expressing cells (cf. Fig. 2A), while its capacity to translocate the AC domain across target cell membrane was ablated by the combination of E570K and E581 P substitutions (cf. Fig. 2B).

As shown in Fig. 4A, the B3Z hybridoma cells were effectively stimulated upon co-incubation with BMDCs loaded with the OVAE570Q/AC- and OVA/E57OQ+K860R/AC- toxoids. In contrast, no B3Z stimulation was observed upon co-incubation with BMDCs loaded with the OVA/E570K+E581 P/AC- toxoid defective in AC domain translocation across cell membrane. Moreover, the OVA/E570Q/AC⁻ and OVA/E570Q+K860R/AC⁻ toxoids induced stimulation of the B3Z lymphocytes by APCs *in vitro* with as high efficiency as intact OVA/AC- toxoid. These results confirm that the E570Q+K860R double mutant was fully capable to translocate its AC domain into BMDC cytosol for processing and presentation of the OVA epitope by K^{b} MHC class I molecules, while being essentially unable to permeabilize the J774A.1 cells. These results suggest that the cell-permeabilizing (pore-forming) activity of CyaA was neither required for AC domain translocation across cellular membrane, nor did it play any role in the capacity of CyaA to deliver passenger epitopes into APC cytosol.

To corroborate the observed *in vitro* antigen delivery capacity of the non-cytolytic toxoids, the inventors assessed their *in vivo* capacity to prime OVA-specific cytotoxic CD8⁺ T lymphocytes (CTL). 50 µg of the various OVA-toxoids were injected intravenously into C57BL/6 mice and one week later the OVA-specific CTL responses were assessed in immunized mice by an *in vivo* killing assay. C57BL/6 mice received i.v. injection of a mixture (1:1) of OVA (SIINFEKL) peptide-loaded CFSE^{high} and unloaded CFSE^{low} splenocytes, followed one day later by FACS analysis of CFSE-labeled cells. As shown in Fig. 4B, immunization of mice with the mock toxoid did not induce any SIINFEKL-specific *in vivo* CTL activity. In turn, immunization with the E570Q+K860R toxoid induced the same OVA-specific *in vivo* CTL killing response as the unmutated toxoid used as positive control, with the slight difference in the values of mean response to the intact and doubly mutated toxoids not being statistically significant (p=0.065). These results show that the cell-permeabilizing activity of CyaA was dispensable for the *in vivo* capacity of the CyaA/2330VA/AC⁻ toxoids to deliver an AC-inserted passenger antigen into cytosol of APCs.

### Discussion

The inventors demonstrate here that translocation of the AC domain of CyaA across the membrane of CD11b/CD18 receptor-expressing myeloid target cells does not depend on the capacity of the toxin to form pores and permeabilize the cellular membrane.

As summarized in the model proposed in Fig. 5, the inventors have previously reported that balance between the two activities of CyaA can be shifted by mutations or alternative acylation of CyaA. Enhancement of the pore-forming (hemolytic) activity at the expense of the capacity to deliver AC into cells was, indeed, observed upon lysine substitutions of glutamates 509, 516 and 581 (13, 18), or upon blocking of AC translocation by the 3D1 monoclonal antibody (MAb) (25). In turn, a shift in the opposite direction was observed for the recombinant *r-Ec-CyaA,* acylated in *E. coli* by palmitoleyl (C16:1) residues, as compared to the native (C16:0) palmitylated *Bp*-CyaA produced by *B*. *pertussis.* The *r-Ec-*CyaA was found to exhibit about four-fold reduced hemolytic activity and about ten-fold lower pore-forming activity in planar lipid bilayers than *Bp*-CyaA (12), while both CyaA forms were equally active in penetrating cellular membrane and translocating the AC domain into erythrocytes (17, 26). Moreover, recently the CyaA/E570Q construct was found to exhibit a full capacity to deliver the AC domain into both erythrocytes and J774A.1 macrophages, while exhibiting reduced hemolytic activity and lower specific pore-forming capacity in planar lipid bilayers than intact CyaA, with the CyaA/E570Q/AC⁻ toxoid exhibiting a two-fold reduced cytolytic activity on J774A.1 cells (8, 13).

Despite the above mentioned and the many mutant CyaAs that the inventors characterized, the question remained whether formation of a membrane pore by CyaA is required for translocation of the AC domain across the membrane of CD11b-expressing cells. It is worth mentioning that, based on previous comparisons of haemolytic potency of the intact *r-Ec-*CyaA with that of the native *Bp*-CyaA purified from *B*. *pertussis,* the specific haemolytic activity of the here described *r*-*Ec*-CyaA/OVA/E570Q+K860R/AC- toxoid on sheep erythrocytes would be reduced by about three orders of magnitude. The residual specific cytolytic activity of *r*-*Ec*-CyaA/OVA/E570Q+K860R/AC- on CD11b-expressing cells would then be estimated to be about 50-fold lower than that of *Bp*-CyaA, while the specific capacity of both proteins to deliver the AC domain into these cells would be the same (using intact *r-Ec-*CyaA as 100% invasive AC activity standard for comparisons). The here described CyaA/233OVA/E570Q+K860R mutant is the first construct with an importantly reduced capacity to permeabilize cells that remains fully capable of translocating the AC domain across cellular membrane. This shows that on its way to cell cytosol the translocating AC domain can bypass the cation-selective pore formed by CyaA.

The mode and path of AC domain translocation across cellular membrane, however, remain to be defined in more detail. Given the differing effects of substitutions of glutamates 509, 516, 570 and 581 on the pore-forming and AC delivery activities of CyaA (8, 13, 18), where the balance between the two activities can be almost entirely shifted in either direction by specific substitutions, the amphipathic helices harboring these glutamate residues appear to be involved in both activities of CyaA in an alternative manner. This is supported by the effect of combined E509K+E516K substitution, which yields a hyper-hemolytic CyaA unable to deliver the AC domain into cells (8, 18), while the here described E570Q+K860R combination yields the opposite, an essentially non-cytolytic CyaA that is fully competent to translocate the AC domain into J774A.1 cells (CD11b⁺).

These observations further corroborate the proposed model that the two membrane activities of CyaA would depend on different conformers inserting into membrane, one yielding translocation of the AC domain by toxin monomers and the other leading to formation of oligomeric CyaA pores (13, 18). It is proposed that the transmembrane segments harbouring the critical glutamate residues 509, 516, 570 and 581 can participate in formation of an oligomeric and cation-selective cytolytic pore only if the membrane-inserted pore precursor conformer has the AC domain located outside the cell. In the AC translocating conformer, the same transmembrane segments would adopt a different conformation in the membrane, being potentially obtruded and prevented from entering CyaA oligomers by the polypeptide linking the C-terminal end of the AC domain to transmembrane segments. Support for this interpretation can be deduced from results obtained by Gray and co-workers (25). These authors showed that deletion of the AC domain together with the segment linking it to the pore-forming domain (up to residue 489), or binding of the 3D1 antibody that blocks membrane translocation of the terminal AC domain segment located between residues 373 and 399, importantly enhances the pore-forming (haemolytic) activity of the toxin. This is likely to be due to imposing a conformation on the transmembrane segments of CyaA that is favourable for formation of the otigomeric pores. It remains to be defined what CyaA segments outside of the pore-forming domain are involved in AC domain translocation across membrane. Given the requirement for its structural integrity (27), the large RTX repeat domain (residues 1006 to 1706) is likely to be taking part in AC translocation into cells. It would be sized enough (700 residues) to form a hydrophilic translocation interface within cellular membrane that might allow passage of an unfolded AC domain across the membrane without a concomitant formation of a real cell-permeabilizing pore. Alternatively, CyaA might promote formation of inverted nonlamellar (inverted hexagonal phase) lipid structures (28), which might potentially take part in a well sealed protein-lipid interface through which the AC domain could slide into cell cytosol.

CyaA was, indeed, previously shown to promote formation of inverted non-lamellar (inverted hexagonal phase) lipid structures (28). These might potentially take part in formation of a well-sealed protein-lipid interface, thus allowing translocation of the AC domain across membrane in the absence of cell permeabilization. Formation of non-lamellar lipid structures is favoured in cholesterol-enriched lipid rafts and CyaA was, indeed, recently found to mobilize into rafts in complex with its receptor CD11b/CD18. Moreover, the inventors have recently shown that AC domain translocation across membrane is accomplished only upon relocation of CyaA into rafts (L., Bumba, J., Masin, R., Fiser, and P., Sebo, submitted). Intriguingly, translocation of the catalytic subunit of diphtheria toxin (DT) across the cell cytoplasmic membrane was also previously found to occur without detectable cell permeabilization, when DT was pulsed into cells by low pH upon binding to a truncated GPI-anchored DT receptor (34). The authors did not examine whether the GPI-anchored DT receptor localized into lipid rafts, but this is highly likely. It is, hence, tempting to speculate that the specific lipid composition of the raft membrane may support translocation of different protein toxins into target cells without the need for formation of a true protein conducting pore permeabilizing the cell.

Last not least, a practical discovery reported herein is that the CyaA/E570Q+K860R/AC⁻ toxoid with the much reduced cell-permeabilizing (cytolytic) activity, remains fully active in antigen delivery into CD11b⁺ APCs. This is of importance in the light of its potential use as enhanced safety profile tool for delivery of tumor-specific antigens in second generation of CyaA/AC⁻-derived vaccines for immunotherapy of cancer.

### References

1. Vojtova J, Kamanova J, Sebo P (2006) Bordetella adenylate cyclase toxin: a swift saboteur of host defense. Curr Opin Microbiol 9: 69-75.
2. Glaser P, Sakamoto H, Bellalou J, Ullmann A, Danchin A (1988) Secretion of cyclolysin, the calmodulin-sensitive adenylate cyclase-haemolysin bifunctional protein of Bordetella pertussis. Embo J 7: 3997-4004.
3. Rose T, Sebo P, Bellalou J, Ladant D (1995) Interaction of calcium with Bordetella pertussis adenylate cyclase toxin. Characterization of multiple calcium-binding sites and calcium-induced conformational changes. J Biol Chem 270: 26370-26376.
4. Masin J, et al. (2005) Acylation of lysine 860 allows tight binding and cytotoxicity of Bordetella adenylate cyclase on CD11b-expressing cells. Biochemistry 44: 12759-12766.
5. Guermonprez P, et al. (2001) The adenylate cyclase toxin of Bordetella pertussis binds to target cells via the alpha(M)beta(2) integrin (CD11b/CD18). J Exp Med 193: 1035-1044.
6. Gordon VM, Leppla SH, Hewlett EL (1988) Inhibitors of receptor-mediated endocytosis block the entry of Bacillus anthracis adenylate cyclase toxin but not that of Bordetella pertussis adenylate cyclase toxin. Infect Immun 56: 1066-1069.
7. Schlecht G, Loucka J, Najar H, Sebo P, Leclerc C (2004) Antigen targeting to CD11b allows efficient presentation of CD4+ and CD8+ T cell epitopes and in vivo Th1-polarized T cell priming. J Immunol 173: 6089-6097.
8. Basler M, Masin J, Osicka R, Sebo P (2006) Pore-forming and enzymatic activities of Bordetella pertussis adenylate cyclase toxin synergize in promoting lysis of monocytes. Infect Immun 74: 2207-2214.
9. Khelef N, Zychlinsky A, Guiso N (1993) Bordetella pertussis induces apoptosis in macrophages: role of adenylate cyclase-hemolysin. Infect Immun 61: 4064-4071.
10. Morova J, Osicka R, Masin J, Sebo P (2008) RTX cytotoxins recognize {beta}2 integrin receptors through N-linked oligosaccharides. Proc Natl Acad Sci U S A.
11. Paccani SR, et al. (2008) Suppression of T-lymphocyte activation and chemotaxis by the adenylate cyclase toxin of Bordetella pertussis. Infect Immun 76: 2822-2832.
12. Benz R, Maier E, Ladant D, Ullmann A, Sebo P (1994) Adenylate cyclase toxin (CyaA) of Bordetella pertussis. Evidence for the formation of small ion-permeable channels and comparison with HlyA of Escherichia coli. J Biol Chem 269: 27231-27239.
13. Basler M, et al. (2007) Segments crucial for membrane translocation and pore-forming activity of Bordetella adenylate cyclase toxin. J Biol Chem 282: 12419-12429.
14. Hewlett EL, Donato GM, Gray MC (2006) Macrophage cytotoxicity produced by adenylate cyclase toxin from Bordetella pertussis: more than just making cyclic AMP! Mol Microbiol 59: 447-459.
15. Fayolle C, Sebo P, Ladant D, Ullmann A, Leclerc C (1996) In vivo induction of CTL responses by recombinant adenylate cyclase of Bordetella pertussis carrying viral CD8+ T cell epitopes. J Immunol 156: 4697-4706.
16. Rogel A, Hanski E (1992) Distinct steps in the penetration of adenylate cyclase toxin of Bordetella pertussis into sheep erythrocytes. Translocation of the toxin across the membrane. J Biol Chem 267: 22599-22605.
17. Havlicek V, et al. (2001) Mass spectrometric analysis of recombinant adenylate cyclase toxin from Bordetella pertussis strain 18323/pHSP9. J Mass Spectrom 36: 384-391.
18. Osickova A, Osicka R, Maier E, Benz R, Sebo P (1999) An amphipathic alpha-helix including glutamates 509 and 516 is crucial for membrane translocation of adenylate cyclase toxin and modulates formation and cation selectivity of its membrane channels. J Biol Chem 274: 37644-37650.
19. Fiser R, et al. (2007) Third activity of Bordetella adenylate cyclase (AC) toxin-hemolysin. Membrane translocation of AC domain polypeptide promotes calcium influx into CD11 b+ monocytes independently of the catalytic and hemolytic activities. J Biol Chem 282: 2808-2820.
20. Osicka R, et al. (2000) Delivery of CD8(+) T-cell epitopes into major histocompatibility complex class I antigen presentation pathway by Bordetella pertussis adenylate cyclase: delineation of cell invasive structures and permissive insertion sites. Infect Immun 68: 247-256.
21. Basar T, et al. (1999) The conserved lysine 860 in the additional fatty-acylation site of Bordetella pertussis adenylate cyclase is crucial for toxin function independently of its acylation status. J Biol Chem 274: 10777-10783.
22. Szabo G, Gray MC, Hewlett EL (1994) Adenylate cyclase toxin from Bordetella pertussis produces ion conductance across artificial lipid bilayers in a calcium- and polarity-dependent manner. J Biol Chem 269: 22496-22499.
23. Kamanova J, et al. (2008) Adenylate cyclase toxin subverts phagocyte function by RhoA inhibition and unproductive ruffling. J Immunol 181: 5587-5597.
24. Guermonprez P, Ladant D, Karimova G, Ullmann A, Leclerc C (1999) Direct delivery of the Bordetella pertussis adenylate cyclase toxin to the MHC class I antigen presentation pathway. J Immunol 162: 1910-1916.
25. Gray MC, et al. (2001) Translocation-specific conformation of adenylate cyclase toxin from Bordetella pertussis inhibits toxin-mediated hemolysis. J Bacteriol 183: 5904-5910.
26. Hackett M, et al. (1995) Hemolytic, but not cell-invasive activity, of adenylate cyclase toxin is selectively affected by differential fatty-acylation in Escherichia coli. J Biol Chem 270: 20250-20253.
27. Iwaki M, Ullmann A, Sebo P (1995) Identification by in vitro complementation of regions required for cell-invasive activity of Bordetella pertussis adenylate cyclase toxin. Mol Microbiol 17: 1015-1024.
28. Martin C, et al. (2004) Membrane restructuring by Bordetella pertussis adenylate cyclase toxin, a member of the RTX toxin family. J Bacteriol 186: 3760-3765.
29. Karimova G, Pidoux J, Ullmann A, Ladant D (1998) A bacterial two-hybrid system based on a reconstituted signal transduction pathway. Proc Natl Acad Sci U S A 95: 5752-5756.
30. Franken KL, et al. (2000) Purification of his-tagged proteins by immobilized chelate affinity chromatography: the benefits from the use of organic solvent. Protein Expr Purif 18: 95-99.
31. Bellalou J, Sakamoto H, Ladant D, Geoffroy C, Ullmann A (1990) Deletions affecting hemolytic and toxin activities of Bordetella pertussis adenylate cyclase. Infect Immun 58: 3242-3247.
32. Gray M.C., et al. (1998) Distinct mechanisms for K+ efflux, intoxication, and hemolysis by Bordetella pertussis AC toxin. J Biol CHem 273:18260-18267
33. Vojvova-Vodolanova J., et al. (2009) Oligomerization is involved in pore formation by Bordetella pertussis adenylate cyclase toxin. FASEB J 23:2331-2343
34. Lanzrein M., et al. (1996) GPI anchored diphteria toxin receptor allows membrane translocation of the toxin without detectable ion channel activity. EMBO J. 15:725-734

## Claims

1. A polypeptide which is a mutant of the adenylate cyclase protein (CyaA) of *Bordetella pertussis* having the amino acid sequence disclosed as SEQ ID NO: 1 and is capable of binding to cells and of translocating its N-terminal adenylate cyclase enzyme domain into said cells wherein said cells express the CD11b/CD18 receptor and wherein binding to said cells occurs through binding to said CD11b/CD18 receptor, and which polypeptide has a pore-forming activity which is reduced or suppressed as compared to that of the native CyaA toxin, and which has an amino acid sequence which differs from the amino acid sequence disclosed as SEQ ID NO: 1 by:
(i) the substitution of the glutamic acid residue corresponding to the position 570 in SEQ ID NO: 1 by a glutamine residue or a conservative residue selected from the group consisting of Asn, Met, Thr, Ser, Gly, Arg, Lys, Val, Leu, Cys, Ile and Asp; and
(ii) the substitution of the lysine residue corresponding to the position 860 in SEQ ID NO: 1 by an arginine residue or by a conservative residue selected from the group consisting of Asn, Gln, Met, Thr, Ser, Gly, Val, Leu, Cys and Ile; and
(iii) optionally from 1 to 10 amino acid residue substitutions, deletions, and/or insertions at locations other than the locations recited in (i) and (ii), said obtained polypeptide having an amino acid sequence which shares at least 99% identity with the sequence set forth in SEQ ID NO: 1.

2. The polypeptide according to claim 1, which is a mutant of an adenylate cyclase toxoid whose adenylate cyclase activity in cells is partly or totally suppressed as compared to that of the *Bordetella* CyaA toxin.

3. The polypeptide according to claim 2, wherein said partial or total suppression of adenylate cyclase activity is achieved by insertion of a dipeptide between the amino acid residues corresponding to the positions 188 and 189 of the adenylate cyclase as defined in SEQ ID N°1.

4. The polypeptide according to claim 1, the amino acid sequence of which is SEQ ID NO: 2.

5. The polypeptide according to claim 1, which is the amino acid sequence disclosed as SEQ ID NO: 2 into which a dipeptide has been inserted between positions 188 and 189, preferably which is the amino acid sequence disclosed as SEQ ID NO: 3.

6. Polypeptide derivative comprising or consisting of the polypeptide according to any one of claims 1 to 5 further combined with one or more molecules of interest selected in the group of peptides, glycopeptides, lipopeptides, polysaccharides, oligosaccharides, nucleic acids, lipids and chemicals.

7. The polypeptide derivative according to claim 6, wherein each of said one or more molecules of interest consists of a polypeptidic molecule or contains a polypeptidic molecule whose amino acid sequence is suitable for eliciting an immune response.

8. The polypeptide derivative according to claim 7, wherein the amino acid sequence of each of said molecule(s) suitable for eliciting an immune response consists of 5 to 800 especially 300 to 600, or 400 to 500 amino acid residues.

9. The polypeptide derivative according to claim 7 or 8, wherein the amino acid sequence of each of said molecule(s) suitable for eliciting an immune response comprises or consists of an amino acid sequence of a poliovirus antigen, an HIV virus antigen, an influenza virus antigen, a choriomeningitis virus sequence, a tumor antigen, or comprises or consists of a part of an amino acid sequence of any of these antigens which comprises at least one epitope.

10. The polypeptide derivative according to any one of claims 7 to 9, which is a recombinant polypeptide, wherein the amino acid sequence of each of said molecule(s) suitable for eliciting an immune response is inserted into a permissive site of said polypeptide, provided that the capacity of said polypeptide to translocate its N-terminal adenylate cyclase enzyme domain into target cells is preserved.

11. The polypeptide derivative according to claim 7 or 8, wherein each of said amino acid sequences suitable for eliciting an immune response is grafted, especially chemically grafted, onto an amino acid residue of said polypeptide.

12. The polypeptide according to any one of claims 1 to 5 or the polypeptide derivative according to any one of claims 6 to 11, for use in therapy.

13. The polypeptide according to any one of claims 1 to 5 or the polypeptide derivative according to any one of claims 6 to 11, for use in therapy in order to elicit a T-cell immune response and/or in order to elicit a B-cell immune response in a host in need thereof.

14. The polypeptide according to any one of claims 1 to 5 or polypeptide derivative according to any one of claims 6 to 11 for use in the prevention or the treatment of a disease selected from neoplasia, cancers and infectious diseases selected from viral-, retroviral-, bacterial-, parasite- or fungal-induced diseases.

15. Polypeptide or polypeptide derivative for use according to any one of claims 12 to 14, wherein said polypeptide or polypeptide derivative is to be administered in combination with an adjuvant and/or in combination with another therapeutically active molecule.

16. Polypeptide or polypeptide derivative for use according to any one of claims 12 to 14, wherein said polypeptide or polypeptide derivative is not to be administered in combination with an adjuvant.

17. Pharmaceutical composition comprising a polypeptide according to any one of claims 1 to 5 or a polypeptide derivative according to any one of claims 6 to 11, a pharmaceutically acceptable carrier, and optionally an adjuvant and/or a therapeutically active molecule.

18. A pharmaceutical composition according to claim 17 for use as a medicament.

19. Use of a polypeptide derivative according to any one of claims 6 to 11, for the preparation of a therapeutic composition for the treatment of a disease selected from neoplasia, cancers and infectious diseases selected from viral-, retroviral-, bacterial-, parasite- or fungal- induced diseases.

20. Method for the preparation of a proteinaceous vector suitable for the delivery of a molecule into a cell comprising binding said molecule to a polypeptide according to any one of claims 1 to 5.

## Patentansprüche

1. Ein Polypeptid, welches eine Mutante des Adenylatcyclaseproteins (CyaA) von *Bordetella pertussis* ist, das die als SEQ ID NO: 1 offenbarte Aminosäuresequenz aufweist und in der Lage ist, an Zellen zu binden und deren N-terminale Adenylatcyclase-Enzymdomäne in die Zellen zu translozieren, wobei die Zellen den CD11 b/CD18-Rezeptor exprimieren und wobei die Bindung an die Zellen durch die Bindung an den CD11b/CD18-Rezeptor erfolgt, und welches Polypeptid eine Poren-bildende Aktivität aufweist, welche im Vergleich zu der des nativen CyaA-Toxins reduziert oder supprimiert ist, und welches eine Aminosäuresequenz aufweist, die sich von der als SEQ ID NO: 1 offenbarten Aminosäuresequenz unterscheidet durch:
(i) die Substitution des Glutaminsäurerestes, entsprechend der Position 570 in SEQ ID NO: 1, durch einen Glutaminrest oder einen konservativen Rest, der ausgewählt ist aus der Gruppe, bestehend aus Asn, Met, Thr, Ser, Gly, Arg, Lys, Val, Leu, Cys, Ile und Asp; und
(ii) die Substitution des Lysinrestes, entsprechend der Position 860 in SEQ ID NO: 1, durch einen Argininrest oder durch einen konservativen Rest, der ausgewählt ist aus der Gruppe, bestehend aus Asn, Gln, Met, Thr, Ser, Gly, Val, Leu, Cys und Ile; und
(iii) gegebenenfalls von 1 bis 10 Substitutionen, Deletionen und/oder Insertionen von Aminosäureresten an Orten außer den unter (i) und (ii) genannten Orten, wobei das erhaltene Polypeptid eine Aminosäuresequenz aufweist, welche mindestens 99% Identität mit der in SEQ ID NO: 1 angegebenen Sequenz besitzt.

2. Das Polypeptid gemäß Anspruch 1, welches eine Mutante eines Adenylatcyclase-Toxoids ist, dessen Adenylatcyclaseaktivität in Zellen im Vergleich zu der des *Bordetella* CyaA-Toxins teilweise oder vollständig supprimiert ist.

3. Das Polypeptid gemäß Anspruch 2, wobei die vollständige oder teilweise Suppression der Adenylatcyclaseaktivität erreicht wird durch Insertion eines Dipeptids zwischen den Aminosäureresten, die den Positionen 188 und 189 der Adenylatcyclase, wie sie in SEQ ID NO: 1 definiert ist, entsprechen.

4. Das Polypeptid gemäß Anspruch 1, dessen Aminosäuresequenz SEQ ID NO: 2 ist.

5. Das Polypeptid gemäß Anspruch 1, welches die Aminosäuresequenz ist, die als SEQ ID NO: 2 offenbart wird, in welche ein Dipeptid zwischen den Positionen 188 und 189 eingefügt worden ist, welches vorzugsweise die als SEQ ID NO: 3 offenbarte Aminosäuresequenz ist.

6. Polypeptidderivat, umfassend oder bestehend aus dem Polypeptid gemäß einem beliebigen der Ansprüche 1 bis 5, des Weiteren kombiniert mit einem oder mehreren Molekülen von Interesse, die ausgewählt sind aus der Gruppe von Peptiden, Glycopeptiden, Lipopeptiden, Polysacchariden, Oligosacchariden, Nukleinsäuren, Lipiden und Chemikalien.

7. Das Polypeptidderivat gemäß Anspruch 6, wobei jedes des einen oder der mehreren Moleküle von Interesse aus einem polypeptidischen Molekül besteht oder ein polypeptidisches Molekül enthält, dessen Aminosäuresequenz zum Auslösen einer Immunantwort geeignet ist.

8. Das Polypeptidderivat gemäß Anspruch 7, wobei die Aminosäuresequenz von jedem des Moleküls/der Moleküle, das/die zum Auslösen einer Immunantwort geeignet ist/sind, aus 5 bis 800, insbesondere 300 bis 600 oder 400 bis 500 Aminosäureresten besteht.

9. Das Polypeptidderivat gemäß Anspruch 7 oder 8, wobei die Aminosäuresequenz von jedem des Moleküls/der Moleküle, das/die zum Auslösen einer Immunantwort geeignet ist/sind, eine Aminosäuresequenz eines Poliovirus-Antigens, eines HIV-Virus-Antigens, eines Influenzavirus-Antigens, einer Choriomeningitisvirus-Sequenz, eines Tumor-Antigens umfasst oder daraus besteht, oder einen Teil einer Aminosäuresequenz von einem beliebigen dieser Antigene, der mindestens ein Epitop umfasst, umfasst oder daraus besteht.

10. Das Polypeptidderivat gemäß einem beliebigen der Ansprüche 7 bis 9, welches ein rekombinantes Polypeptid ist, wobei die Aminosäuresequenz von jedem des Moleküls/der Moleküle, das/die zum Auslösen einer Immunantwort geeignet ist/sind, in eine permissive Stelle des Polypeptids eingefügt ist, mit der Maßgabe, dass die Fähigkeit des Polypeptids, dessen N-terminale Adenylatcyclase-Enzymdomäne in Zielzellen zu translozieren, bewahrt wird.

11. Das Polypeptidderivat gemäß Anspruch 7 oder 8, wobei jede der Aminosäuresequenzen, die zum Auslösen einer Immunantwort geeignet sind, auf einen Aminosäurerest des Polypeptids gegraftet, insbesondere chemisch gegraftet ist.

12. Das Polypeptid gemäß einem beliebigen der Ansprüche 1 bis 5 oder das Polypeptidderivat gemäß einem beliebigen der Ansprüche 6 bis 11 zur Verwendung in der Therapie.

13. Das Polypeptid gemäß einem beliebigen der Ansprüche 1 bis 5 oder das Polypeptidderivat gemäß einem beliebigen der Ansprüche 6 bis 11 zur Verwendung in der Therapie, um in einem Wirt, der dessen bedarf, eine T-Zell-Immunantwort auszulösen und/oder um eine B-Zell-Immunantwort auszulösen.

14. Das Polypeptid gemäß einem beliebigen der Ansprüche 1 bis 5 oder das Polypeptidderivat gemäß einem beliebigen der Ansprüche 6 bis 11 zur Verwendung in der Prävention oder der Behandlung einer Erkrankung, ausgewählt aus Neoplasie, Krebs und Infektionserkrankungen, ausgewählt aus viral-, retroviral-, bakteriell-, parasitär- oder Pilz-induzierten Erkrankungen.

15. Polypeptid oder Polypeptidderivat zur Verwendung gemäß einem beliebigen der Ansprüche 12 bis 14, wobei das Polypeptid oder Polypeptidderivat in Kombination mit einem Adjuvans und/oder in Kombination mit einem anderen therapeutisch aktiven Molekül zu verabreichen ist.

16. Polypeptid oder Polypeptidderivat zur Verwendung gemäß einem beliebigen der Ansprüche 12 bis 14, wobei das Polypeptid oder Polypeptidderivat nicht in Kombination mit einem Adjuvans zu verabreichen ist.

17. Pharmazeutische Zusammensetzung, umfassend ein Polypeptid gemäß einem beliebigen der Ansprüche 1 bis 5 oder ein Polypeptidderivat gemäß einem beliebigen der Ansprüche 6 bis 11, einen pharmazeutisch verträglichen Träger und gegebenenfalls ein Adjuvans und/oder ein therapeutisch aktives Molekül.

18. Eine pharmazeutische Zusammensetzung gemäß Anspruch 17 zur Verwendung als ein Medikament.

19. Verwendung eines Polypeptidderivats gemäß einem beliebigen der Ansprüche 6 bis 11 zur Herstellung einer therapeutischen Zusammensetzung für die Behandlung einer Erkrankung, ausgewählt aus Neoplasie, Krebs und Infektionserkrankungen, ausgewählt aus viral-, retroviral-, bakteriell-, parasitär- oder Pilz-induzierten Erkrankungen.

20. Verfahren zur Herstellung eines proteinösen Vektors, der zur Lieferung eines Moleküls in eine Zelle geeignet ist, umfassend das Binden des Moleküls an ein Polypeptid gemäß einem beliebigen der Ansprüche 1 bis 5.

## Revendications

1. Polypeptide qui est un mutant de la protéine adénylate cyclase (CyaA) de *Bordetella pertussis* ayant la séquence d'acides aminés décrite comme SEQ ID NO: 1 et est capable de se lier à des cellules et de transloquer son domaine d'enzyme adénylate cyclase N-terminal dans lesdites cellules, dans lequel lesdites cellules expriment le récepteur CD11b/CD18 et dans lequel la liaison auxdites cellules se produit à travers la liaison audit récepteur CD11b/CD18, et lequel polypeptide a une activité de formation de pores qui est réduite ou supprimée par rapport à celle de la toxine CyaA native, et qui a une séquence d'acides aminés qui diffère de la séquence d'acides aminés décrite comme SEQ ID NO: 1 par:
(i) la substitution du résidu acide glutamique correspondant à la position 570 dans SEQ ID NO: 1 par un résidu glutamine ou un résidu conservateur choisi dans le groupe constitué par Asn, Met, Thr, Ser, Gly, Arg, Lys, Val, Leu, Cys, Ile et Asp; et
(ii) la substitution du résidu lysine correspondant à la position 860 dans SEQ ID NO: 1 par un résidu arginine ou par un résidu conservateur choisi dans le groupe constitué par Asn, Gln, Met, Thr, Ser, Gly, Val, Leu, Cys et Ile; et
(iii) éventuellement de 1 à 10 substitutions, délétions, et/ou insertions de résidus d'acides aminés à des endroits autres que les endroits récités dans (i) et (ii), ledit polypeptide obtenu ayant une séquence d'acides aminés qui partage au moins 99% d'identité avec la séquence indiquée dans SEQ ID NO: 1.

2. Polypeptide selon la revendication 1, qui est un mutant d'une anatoxine adénylate cyclase dont l'activité adénylate cyclase dans les cellules est supprimée en partie ou totalement par rapport à celle de la toxine CyaA de *Bordetella.*

3. Polypeptide selon la revendication 2, dans lequel ladite suppression partielle ou totale de l'activité adénylate cyclase est obtenue par insertion d'un dipeptide entre les résidus d'acides aminés correspondant aux positions 188 et 189 de l'adénylate cyclase telle que définie dans SEQ ID NO: 1.

4. Polypeptide selon la revendication 1, dont la séquence d'acides aminés est SEQ ID NO: 2.

5. Polypeptide selon la revendication 1, qui est la séquence d'acides aminés décrite comme SEQ ID NO: 2 dans laquelle un dipeptide a été inséré entre les positions 188 et 189, qui est de préférence la séquence d'acides aminés décrite comme SEQ ID NO: 3.

6. Dérivé polypeptidique comprenant ou constitué par le polypeptide selon l'une quelconque des revendications 1 à 5, combiné en outre avec une ou plusieurs molécules d'intérêt choisie(s) dans le groupe des peptides, glycopeptides, lipopeptides, polysaccharides, oligosaccharides, acides nucléiques, lipides et produits chimiques.

7. Dérivé polypeptidique selon la revendication 6, dans lequel chacune desdites une ou plusieurs molécules d'intérêt est constituée d'une molécule polypeptidique ou contient une molécule polypeptidique dont la séquence d'acides aminés est appropriée pour susciter une réponse immunitaire.

8. Dérivé polypeptidique selon la revendication 7, dans lequel la séquence d'acides aminés de chacune de ladite(desdites) molécule(s) appropriée(s) pour susciter une réponse immunitaire est constituée de 5 à 800 en particulier 300 à 600, ou 400 à 500 résidus d'acides aminés.

9. Dérivé polypeptidique selon la revendication 7 ou 8, dans lequel la séquence d'acides aminés de chacune de ladite(desdites) molécule(s) appropriée(s) pour susciter une réponse immunitaire comprend ou est constituée d'une séquence d'acides aminés d'un antigène de poliovirus, d'un antigène de virus VIH, d'un antigène de virus de l'influenza, d'une séquence de virus de la chorioméningite, d'un antigène tumoral, ou comprend ou est constituée d'une partie d'une séquence d'acides aminés de n'importe lequel de ces antigènes qui comprend au moins un épitope.

10. Dérivé polypeptidique selon l'une quelconque des revendications 7 à 9, qui est un polypeptide recombinant dans lequel la séquence d'acides aminés de chacune de ladite(desdites) molécule(s) appropriée(s) pour susciter une réponse immunitaire est insérée dans un site permissif dudit polypeptide, à condition que la capacité dudit polypeptide de transloquer son domaine d'enzyme adénylate cyclase N-terminal dans les cellules cibles soit conservée.

11. Dérivé polypeptidique selon la revendication 7 ou 8, dans lequel chacune desdites séquences d'acides aminés appropriées pour susciter une réponse immunitaire est greffé, en particulier greffé chimiquement, sur un résidu d'acide aminé dudit polypeptide.

12. Polypeptide selon l'une quelconque des revendications 1 à 5 ou dérivé polypeptidique selon l'une quelconque des revendications 6 à 11, pour une utilisation en thérapie.

13. Polypeptide selon l'une quelconque des revendications 1 à 5 ou dérivé polypeptidique selon l'une quelconque des revendications 6 à 11, pour utilisation en thérapie afin de susciter une réponse immunitaire à cellules T et/ou afin de susciter une réponse immunitaire à cellules B chez un hôte en ayant besoin.

14. Polypeptide selon l'une quelconque des revendications 1 à 5 ou dérivé polypeptidique selon l'une quelconque des revendications 6 à 11, pour une utilisation dans la prévention ou le traitement d'une maladie choisie parmi une néoplasie, les cancers et les maladies infectieuses choisies parmi les maladies d'origine virale, rétrovirale, bactérienne, parasitaire ou fongique.

15. Polypeptide ou dérivé polypeptidique pour une utilisation selon l'une quelconque des revendications 12 à 14, dans lequel ledit polypeptide ou dérivé polypeptidique doit être administré en association avec un adjuvant et/ou en association avec une autre molécule thérapeutiquement active.

16. Polypeptide ou dérivé polypeptidique pour une utilisation selon l'une quelconque des revendications 12 à 14, dans lequel ledit polypeptide ou dérivé polypeptidique n'est pas destiné à être administré en association avec un adjuvant.

17. Composition pharmaceutique comprenant un polypeptide selon l'une quelconque des revendications 1 à 5 ou un dérivé polypeptidique selon l'une quelconque des revendications 6 à 11, un support pharmaceutiquement acceptable, et éventuellement un adjuvant et/ou une molécule thérapeutiquement active.

18. Composition pharmaceutique selon la revendication 17 pour une utilisation en tant que médicament.

19. Utilisation d'un dérivé polypeptidique selon l'une quelconque des revendications 6 à 11, pour la préparation d'une composition thérapeutique pour le traitement d'une maladie choisie parmi une néoplasie, les cancers et les maladies infectieuses choisies parmi les maladies d'origine virale, rétrovirale, bactérienne, parasitaire ou fongique.

20. Procédé de préparation d'un vecteur protéinique approprié pour la délivrance d'une molécule dans une cellule comprenant la liaison de ladite molécule à un polypeptide selon l'une quelconque des revendications 1 à 5.
